# EUROPEAN PATENT SPECIFICATION

(11) **EP 1 782 070 B1**
(45) Date of publication and mention of the grant of the patent: **08.09.2010**
(21) Application number: 05766030.0
(22) Date of filing: 17.06.2005
(51) Int. Cl.: G01N 33/574, A61K 39/00

(54) **TUMOR-ASSOCIATED ANTIGEN PROFILES IN CANCER DIAGNOSTICS AND IMMUNOTHERAPY**
TUMORASSOZIIERTE ANTIGENPROFILE IN DER KREBSDIAGNOSTIK UND IMMUNTHERAPIE
COMBINAISONS D'ANTIGENES ASSOCIES A UNE TUMEUR POUR LE DIAGNOSTIC DE DIFFERENTS TYPES DE CANCER

(30) Priority: 17.06.2004 US 580969 P
(43) Date of publication of application: 09.05.2007
(73) Proprietor: MannKind Corporation, Valencia, CA 91355 (US)
(72) Inventor: CHIANG, Chih-Sheng, Chatsworth, CA 91311 (US); SIMARD, John, J.L., West Vancouver, British Columbia V7V3C1 (CA)
(74) Representative: Neuefeind, Regina
(86) International application number: PCT/US2005/021836
(87) International publication number: WO 2006/002114

(56) References cited:
- US-A- 4 940 670
- MASHINO K ET AL: "Expression of multiple cancer-testis antigen genes in gastrointestinal and breast carcinomas" BRITISH JOURNAL OF CANCER, LONDON, GB, vol. 85, no. 5, 1 September 2001 (2001-09-01), pages 713-720, XP002355500 ISSN: 0007-0920
- NEUMANN E ET AL: "Heterogeneous expression of the tumor-associated antigens RAGE-1, PRAME, and glycoprotein 75 in human renal cell carcinoma: Candidates for T-cell-based immunotherapies?" CANCER RESEARCH, AMERICAN ASSOCIATION FOR CANCER RESEARCH, BALTIMORE, MD, US, vol. 58, no. 18, 15 September 1998 (1998-09-15), pages 4090-4095, XP002361281 ISSN: 0008-5472
- MAEURER M J ET AL: "New treatment options for patients with melanoma: review of melanoma derived T - cell epitope -based peptide vaccines" MELANOMA RESEARCH, vol. 6, no. 1, February 1996 (1996-02), pages 11-24, XP002111873
- KAWAKAMI YUTAKA ET AL: "Recognition of shared melanoma antigens in association with major HLA-A alleles by tumor infiltrating T lymphocytes from 123 patients with melanoma" JOURNAL OF IMMUNOTHERAPY, vol. 23, no. 1, January 2000 (2000-01), pages 17-27, XP008087079
- TAKEUCHI HIROYA ET AL: "Expression of differentiation melanoma-associated antigen genes is associated with favorable disease outcome in advanced-stage melanomas." CANCER RESEARCH, vol. 63, no. 2, 15 January 2003 (2003-01-15), pages 441-448, XP002463259 ISSN: 0008-5472
- DATABASE MEDLINE [Online] US NATIONAL LIBRARY OF MEDICINE (NLM), BETHESDA, MD, US; April 1999 (1999-04), SARDI I ET AL: "The role of the detection of hematogenous micrometastasis in prostate adenocarcinoma and malignant melanoma by RT-PCR." XP002463260 Database accession no. NLM10085416 & INTERNATIONAL JOURNAL OF MOLECULAR MEDICINE APR 1999, vol. 3, no. 4, April 1999 (1999-04), pages 417-419, ISSN: 1107-3756
- NEUMANN ELENA ET AL: "Heterogeneous expression of the tumor-associated antigens RAGE-1, PRAME, and glycoprotein 75 in human renal cell carcinoma: Candidates for T-cell-based immunotherapies?" CANCER RESEARCH, vol. 58, no. 18, 15 September 1998 (1998-09-15), pages 4090-4095, XP002361281 ISSN: 0008-5472
- VRIES DE T J ET AL: "HETEROGENOUS EXPRESSION OF IMMUNOTHERAPY CANDIDATE PROTEINS GP100, MART-1 AND TYROSINASE IN HUMAN MELANOMA CELL LINES AND IN HUMAN MELANOCYTIC LESIONS" CANCER RESEARCH, AMERICAN ASSOCIATION FOR CANCER RESEARCH, BALTIMORE, MD, US, vol. 57, 1 August 1997 (1997-08-01), pages 3223-3229, XP002952165 ISSN: 0008-5472
- GOLD D V ET AL: "DIFFERENTIAL EXPRESSION OF TUMOR ASSOCIATED ANTIGENS IN HUMAN COLON CARCINOMAS XENO GRAFTED INTO NUDE MICE" JOURNAL OF THE NATIONAL CANCER INSTITUTE, vol. 71, no. 1, 1983, pages 117-124, XP008057919
- LEWIS J J ET AL: "DEFINITION OF TUMOR ANTIGENS SUITABLE FOR VACCINE CONSTRUCTION" SEMINARS IN CANCER BIOLOGY, SAUNDERS SCIENTIFIC PUBLICATIONS, PHILADELPHIA, PA, US, vol. 6, December 1995 (1995-12), pages 321-327, XP002926806 ISSN: 1044-579X
- SCANLAN M J ET AL: "Challenges to the development of antigen-specific breast cancer vaccines." BREAST CANCER RESEARCH : BCR. 2001, vol. 3, no. 2, 2001, pages 95-98, XP002361282 ISSN: 1465-5411

### SEQUENCE LISTING

<110> Chiang, Chih-Sheng
   Simard, John J.L.
<120> COMBINATIONS OF TUMOR-ASSOCIATED
   ANTIGENS IN DIAGNOSTICS FOR VARIOUS TYPES OF CANCERS
<130> MANNK.050A
<150> 60/580,969
   <151> 2004-06-17
<160> 40
<170> FastSEQ for Windows Version 4.0
<210> 1
   <211> 529
   <212> PRT
   <213> Homo sapiens
<400> 1
<210> 2
   <211> 118
   <212> PRT
   <213> Homo sapiens
<400> 2
   Met Pro Arg Glu Asp Ala His Phe Ile Tyr Gly Tyr Pro Lys Lys Gly
    1               5                  10                  15
   His Gly His Ser Tyr Thr Thr Ala Glu Glu Ala Ala Gly Ile Gly Ile
               20                  25                  30
   Leu Thr Val Ile Leu Gly Val Leu Leu Leu Ile Gly Cys Trp Tyr Cys
           35                  40                  45
   Arg Arg Arg Asn Gly Tyr Arg Ala Leu Met Asp Lys Ser Leu His Val
       50                  55                  60
   Gly Thr Gln Cys Ala Leu Thr Arg Arg Cys Pro Gln Glu Gly Phe Asp
   65                  70                  75                  80
   His Arg Asp Ser Lys Val Ser Leu Gln Glu Lys Asn Cys Glu Pro Val
                   85                  90                  95
   Val Pro Asn Ala Pro Pro Ala Tyr Glu Lys Leu Ser Ala Glu Gln Ser
               100                 105                 110
   Pro Pro Pro Tyr Ser Pro
           115
<210> 3
   <211> 223
   <212> PRT
   <213> Homo sapiens
<400> 3
   Met Asn Gly Asp Asp Ala Phe Ala Arg Arg Pro Thr Val Gly Ala Gln
    1               5                  10                  15
   Ile Pro Glu Lys Ile Gln Lys Ala Phe Asp Asp Ile Ala Lys Tyr Phe
               20                  25                  30
   Ser Lys Glu Glu Trp Glu Lys Met Lys Ala Ser Glu Lys Ile Phe Tyr
           35                  40                  45
   Val Tyr Met Lys Arg Lys Tyr Glu Ala Met Thr Lys Leu Gly Phe Lys
       50                  55                  60
   Ala Thr Leu Pro Pro Phe Met Cys Asn Lys Arg Ala Glu Asp Phe Gln
   65                  70                  75                  80
   Gly Asn Asp Leu Asp Asn Asp Pro Asn Arg Gly Asn Gln Val Glu Arg
                   85                  90                  95
   Pro Gln Met Thr Phe Gly Arg Leu Gln Gly Ile Ser Pro Lys Ile Met
               100                 105                 110
   Pro Lys Lys Pro Ala Glu Glu Gly Asn Asp Ser Glu Glu Val Pro Glu
           115                 120                 125
   Ala Ser Gly Pro Gln Asn Asp Gly Lys Glu Leu Cys Pro Pro Gly Lys
       130                 135                 140
   Pro Thr Thr Ser Glu Lys Ile His Glu Arg Ser Gly Asn Arg Glu Ala
   145                 150                 155                 160
   Gln Glu Lys Glu Glu Arg Arg Gly Thr Ala His Arg Trp Ser Ser Gln
                   165                 170                 175
   Asn Thr His Asn Ile Gly Arg Phe Ser Leu Ser Thr Ser Met Gly Ala
               180                 185                 190
   Val His Gly Thr Pro Lys Thr Ile Thr His Asn Arg Asp Pro Lys Gly
           195                 200                 205
   Gly Asn Met Pro Gly Pro Thr Asp Cys Val Arg Glu Asn Ser Trp
       210                 215                 220
<210> 4
   <211> 750
   <212> PRT
   <213> Homo sapiens
<400> 4
   Met Trp Asn Leu Leu His Glu Thr Asp Ser Ala Val Ala Thr Ala Arg
    1               5                  10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
           35                  40                  45
   Ala Thr Asn Ile Thr Pro Lys His Asn Met Lys Ala Phe Leu Asp Glu
       50                  55                  60
   Leu Lys Ala Glu Asn Ile Lys Lys Phe Leu Tyr Asn Phe Thr Gln Ile
   65                  70                  75                  80
   Pro His Leu Ala Gly Thr Glu Gln Asn Phe Gln Leu Ala Lys Gln Ile
                   85                  90                  95
   Gln Ser Gln Trp Lys Glu Phe Gly Leu Asp Ser Val Glu Leu Ala His
               100                 105                 110
   Tyr Asp Val Leu Leu Ser Tyr Pro Asn Lys Thr His Pro Asn Tyr Ile
           115                 120                 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
   180 185 190
   Lys Ile Asn Cys Ser Gly Lys Ile Val Ile Ala Arg Tyr Gly Lys Val
   195 200 205
   Phe Arg Gly Asn Lys Val Lys Asn Ala Gln Leu Ala Gly Ala Lys Gly
   210 215 220
   Val Ile Leu Tyr Ser Asp Pro Ala Asp Tyr Phe Ala Pro Gly Val Lys
   225 230 235 240
   Ser Tyr Pro Asp Gly Trp Asn Leu Pro Gly Gly Gly Val Gln Arg Gly
   245 250 255
   Asn Ile Leu Asn Leu Asn Gly Ala Gly Asp Pro Leu Thr Pro Gly Tyr
   260 265 270
   Pro Ala Asn Glu Tyr Ala Tyr Arg Arg Gly Ile Ala Glu Ala Val Gly
   275 280 285
   Leu Pro Ser Ile Pro Val His Pro Ile Gly Tyr Tyr Asp Ala Gln Lys
   290 295 300
   Leu Leu Glu Lys Met Gly Gly Ser Ala Pro Pro Asp Ser Ser Trp Arg
   305 310 315 320
   Gly Ser Leu Lys Val Pro Tyr Asn Val Gly Pro Gly Phe Thr Gly Asn
   325 330 335
   Phe Ser Thr Gln Lys Val Lys Met His Ile His Ser Thr Asn Glu Val
   340 345 350
   Thr Arg Ile Tyr Asn Val Ile Gly Thr Leu Arg Gly Ala Val Glu Pro
   355 360 365
   Asp Arg Tyr Val Ile Leu Gly Gly His Arg Asp Ser Trp Val Phe Gly
   370 375 380
   Gly Ile Asp Pro Gln Ser Gly Ala Ala Val Val His Glu Ile Val Arg
   385 390 395 400
   Ser Phe Gly Thr Leu Lys Lys Glu Gly Trp Arg Pro Arg Arg Thr Ile
   405 410 415
   Leu Phe Ala Ser Trp Asp Ala Glu Glu Phe Gly Leu Leu Gly Ser Thr
   420 425 430
   Glu Trp Ala Glu Glu Asn Ser Arg Leu Leu Gln Glu Arg Gly Val Ala
   435 440 445
   Tyr Ile Asn Ala Asp Ser Ser Ile Glu Gly Asn Tyr Thr Leu Arg Val
   450 455 460
   Asp Cys Thr Pro Leu Met Tyr Ser Leu Val His Asn Leu Thr Lys Glu
   465 470 475 480
   Leu Lys Ser Pro Asp Glu Gly Phe Glu Gly Lys Ser Leu Tyr Glu Ser
   485 490 495
   Trp Thr Lys Lys Ser Pro Ser Pro Glu Phe Ser Gly Met Pro Arg Ile
   500 505 510
   Ser Lys Leu Gly Ser Gly Asn Asp Phe Glu Val Phe Phe Gln Arg Leu
   515 520 525
   Gly Ile Ala Ser Gly Arg Ala Arg Tyr Thr Lys Asn Trp Glu Thr Asn
   530 535 540
   Lys Phe Ser Gly Tyr Pro Leu Tyr His Ser Val Tyr Glu Thr Tyr Glu
   545 550 555 560
   Leu Val Glu Lys Phe Tyr Asp Pro Met Phe Lys Tyr His Leu Thr Val
   565 570 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
   645 650 655
   Asn Pro Ile Val Leu Arg Met Met Asn Asp Gln Leu Met Phe Leu Glu
   660 665 670
   Arg Ala Phe Ile Asp Pro Leu Gly Leu Pro Asp Arg Pro Phe Tyr Arg
               675                     680                     685
   His Val Ile Tyr Ala Pro Ser Ser His Asn Lys Tyr Ala Gly Glu Ser
           690                     695                     700
   Phe Pro Gly Ile Tyr Asp Ala Leu Phe Asp Ile Glu Ser Lys Val Asp
   705                     710                     715                     720
   Pro Ser Lys Ala Trp Gly Glu Val Lys Arg Gln Ile Tyr Val Ala Ala
                   725                     730                     735
   Phe Thr Val Gln Ala Ala Ala Glu Thr Leu Ser Glu Val Ala
               740                     745                     750
<210> 5
   <211> 309
   <212> PRT
   <213> Homo sapiens
<400> 5
   Met Ser Leu Glu Gln Arg Ser Leu His Cys Lys Pro Glu Glu Ala Leu
    1               5                  10                  15
   Glu Ala Gln Gln Glu Ala Leu Gly Leu Val Cys Val Gln Ala Ala Thr
               20                  25                  30
   Ser Ser Ser Ser Pro Leu Val Leu Gly Thr Leu Glu Glu Val Pro Thr
           35                  40                  45
   Ala Gly Ser Thr Asp Pro Pro Gln Ser Pro Gln Gly Ala Ser Ala Phe
       50                  55                  60
   Pro Thr Thr Ile Asn Phe Thr Arg Gln Arg Gln Pro Ser Glu Gly Ser
   65                  70                  75                  80
   Ser Ser Arg Glu Glu Glu Gly Pro Ser Thr Ser Cys Ile Leu Glu Ser
                   85                  90                  95
   Leu Phe Arg Ala Val Ile Thr Lys Lys Val Ala Asp Leu Val Gly Phe
               100                 105                 110
   Leu Leu Leu Lys Tyr Arg Ala Arg Glu Pro Val Thr Lys Ala Glu Met
           115                 120                 125
   Leu Glu Ser Val Ile Lys Asn Tyr Lys His Cys Phe Pro Glu Ile Phe
       130                 135                 140
   Gly Lys Ala Ser Glu Ser Leu Gln Leu Val Phe Gly Ile Asp Val Lys
   145                 150                 155                 160
   Glu Ala Asp Pro Thr Gly His Ser Tyr Val Leu Val Thr Cys Leu Gly
                   165                 170                 175
   Leu Ser Tyr Asp Gly Leu Leu Gly Asp Asn Gln Ile Met Pro Lys Thr
               180                 185                 190
   Gly Phe Leu Ile Ile Val Leu Val Met Ile Ala Met Glu Gly Gly His
           195                 200                 205
   Ala Pro Glu Glu Glu Ile Trp Glu Glu Leu Ser Val Met Glu Val Tyr
       210                 215                 220
   Asp Gly Arg Glu His Ser Ala Tyr Gly Glu Pro Arg Lys Leu Leu Thr
   225                 230                 235                 240
   Gln Asp Leu Val Gln Glu Lys Tyr Leu Glu Tyr Arg Gln Val Pro Asp
                   245                 250                 255
   Ser Asp Pro Ala Arg Tyr Glu Phe Leu Trp Gly Pro Arg Ala Leu Ala
               260                 265                 270
   Glu Thr Ser Tyr Val Lys Val Leu Glu Tyr Val Ile Lys Val Ser Ala
           275                 280                 285
   Arg Val Arg Phe Phe Phe Pro Ser Leu Arg Glu Ala Ala Leu Arg Glu
       290                 295                 300
   Glu Glu Glu Gly Val
   305
<210> 6
   <211> 314
   <212> PRT
   <213> Homo sapiens
<400> 6
   Met Pro Leu Glu Gln Arg Ser Gln His Cys Lys Pro Glu Glu Gly Leu
    1               5                  10                  15
   Glu Ala Arg Gly Glu Ala Leu Gly Leu Val Gly Ala Gln Ala Pro Ala
               20                  25                  30
   Thr Glu Glu Gln Glu Ala Ala Ser Ser Ser Ser Thr Leu Val Glu Val
           35                  40                  45
   Thr Leu Gly Glu Val Pro Ala Ala Glu Ser Pro Asp Pro Pro Gln Ser
       50                  55                  60
   Pro Gln Gly Ala Ser Ser Leu Pro Thr Thr Met Asn Tyr Pro Leu Trp
   65                  70                  75                  80
   Ser Gln Ser Tyr Glu Asp Ser Ser Asn Gln Glu Glu Glu Gly Pro Ser
                   85                  90                  95
   Thr Phe Pro Asp Leu Glu Ser Glu Phe Gln Ala Ala Leu Ser Arg Lys
               100                 105                 110
   Val Ala Glu Leu Val His Phe Leu Leu Leu Lys Tyr Arg Ala Arg Glu
           115                 120                 125
   Pro Val Thr Lys Ala Glu Met Leu Gly Ser Val Val Gly Asn Trp Gln
       130                 135                 140
   Tyr Phe Phe Pro Val Ile Phe Ser Lys Ala Ser Ser Ser Leu Gln Leu
   145                 150                 155                 160
   Val Phe Gly Ile Glu Leu Met Glu Val Asp Pro Ile Gly His Leu Tyr
                   165                 170                 175
   Ile Phe Ala Thr Cys Leu Gly Leu Ser Tyr Asp Gly Leu Leu Gly Asp
               180                 185                 190
   Asn Gln Ile Met Pro Lys Ala Gly Leu Leu Ile Ile Val Leu Ala Ile
           195                 200                 205
   Ile Ala Arg Glu Gly Asp Cys Ala Pro Glu Glu Lys Ile Trp Glu Glu
       210                 215                 220
   Leu Ser Val Leu Glu Val Phe Glu Gly Arg Glu Asp Ser Ile Leu Gly
   225                 230                 235                 240
   Asp Pro Lys Lys Leu Leu Thr Gln His Phe Val Gln Glu Asn Tyr Leu
                   245                 250                 255
   Glu Tyr Arg Gln Val Pro Gly Ser Asp Pro Ala Cys Tyr Glu Phe Leu
               260                 265                 270
   Trp Gly Pro Arg Ala Leu Val Glu Thr Ser Tyr Val Lys Val Leu His
           275                 280                 285
   His Met Val Lys Ile Ser Gly Gly Pro His Ile Ser Tyr Pro Pro Leu
       290                 295                 300
   His Glu Trp Val Leu Arg Glu Gly Glu Glu
   305                 310
<210> 7
   <211> 180
   <212> PRT
   <213> Homo sapiens
<400> 7
   Met Gln Ala Glu Gly Arg Gly Thr Gly Gly Ser Thr Gly Asp Ala Asp
    1               5                  10                  15
   Gly Pro Gly Gly Pro Gly Ile Pro Asp Gly Pro Gly Gly Asn Ala Gly
               20                  25                  30
   Gly Pro Gly Glu Ala Gly Ala Thr Gly Gly Arg Gly Pro Arg Gly Ala
           35                  40                  45
   Gly Ala Ala Arg Ala Ser Gly Pro Gly Gly Gly Ala Pro Arg Gly Pro
       50                  55                  60
   His Gly Gly Ala Ala Ser Gly Leu Asn Gly Cys Cys Arg Cys Gly Ala
   65                  70                  75                  80
   Arg Gly Pro Glu Ser Arg Leu Leu Glu Phe Tyr Leu Ala Met Pro Phe
                   85                  90                  95
   Ala Thr Pro Met Glu Ala Glu Leu Ala Arg Arg Ser Leu Ala Gln Asp
               100                 105                 110
   Ala Pro Pro Leu Pro Val Pro Gly Val Leu Leu Lys Glu Phe Thr Val
           115                 120                 125
   Ser Gly Asn Ile Leu Thr Ile Arg Leu Thr Ala Ala Asp His Arg Gln
       130                 135                 140
   Leu Gln Leu Ser Ile Ser Ser Cys Leu Gln Gln Leu Ser Leu Leu Met
   145                 150                 155                 160
   Trp Ile Thr Gln Cys Phe Leu Pro Val Phe Leu Ala Gln Pro Pro Ser
                   165                 170                 175
   Gly Gln Arg Arg
               180
<210> 8
   <211> 509
   <212> PRT
   <213> Homo sapiens
<400> 8
   Met Glu Arg Arg Arg Leu Trp Gly Ser Ile Gln Ser Arg Tyr Ile Ser
   1 5 10 15
   Met Ser Val Trp Thr Ser Pro Arg Arg Leu Val Glu Leu Ala Gly Gln
   20 25 30
   Ser Leu Leu Lys Asp Glu Ala Leu Ala Ile Ala Ala Leu Glu Leu Leu
   35 40 45
   Pro Arg Glu Leu Phe Pro Pro Leu Phe Met Ala Ala Phe Asp Gly Arg
   50 55 60
   His Ser Gln Thr Leu Lys Ala Met Val Gln Ala Trp Pro Phe Thr Cys
   65 70 75 80
   Leu Pro Leu Gly Val Leu Met Lys Gly Gln His Leu His Leu Glu Thr
   85 90 95
   Phe Lys Ala Val Leu Asp Gly Leu Asp Val Leu Leu Ala Gln Glu Val
   100 105 110
   Arg Pro Arg Arg Trp Lys Leu Gln Val Leu Asp Leu Arg Lys Asn Ser
   115 120 125
   His Gln Asp Phe Trp Thr Val Trp Ser Gly Asn Arg Ala Ser Leu Tyr
   130 135 140
   Ser Phe Pro Glu Pro Glu Ala Ala Gln Pro Met Thr Lys Lys Arg Lys
   145 150 155 160
   Val Asp Gly Leu Ser Thr Glu Ala Glu Gln Pro Phe Ile Pro Val Glu
   165 170 175
   Val Leu Val Asp Leu Phe Leu Lys Glu Gly Ala Cys Asp Glu Leu Phe
   180 185 190
   Ser Tyr Leu Ile Glu Lys Val Lys Arg Lys Lys Asn Val Leu Arg Leu
   195 200 205
   Cys Cys Lys Lys Leu Lys Ile Phe Ala Met Pro Met Gln Asp Ile Lys
   210 215 220
   Met Ile Leu Lys Met Val Gln Leu Asp Ser Ile Glu Asp Leu Glu Val
   225 230 235 240
   Thr Cys Thr Trp Lys Leu Pro Thr Leu Ala Lys Phe Ser Pro Tyr Leu
   245 250 255
   Gly Gln Met Ile Asn Leu Arg Arg Leu Leu Leu Ser His Ile His Ala
   260 265 270
   Ser Ser Tyr Ile Ser Pro Glu Lys Glu Glu Gln Tyr Ile Ala Gln Phe
   275 280 285
   Thr Ser Gln Phe Leu Ser Leu Gln Cys Leu Gln Ala Leu Tyr Val Asp
   290 295 300
   Ser Leu Phe Phe Leu Arg Gly Arg Leu Asp Gln Leu Leu Arg His Val
   305 310 315 320
   Met Asn Pro Leu Glu Thr Leu Ser Ile Thr Asn Cys Arg Leu Ser Glu
                   325                     330                     335
   Gly Asp Val Met His Leu Ser Gln Ser Pro Ser Val Ser Gln Leu Ser
               340                     345                     350
   Val Leu Ser Leu Ser Gly Val Met Leu Thr Asp Val Ser Pro Glu Pro
           355                     360                     365
   Leu Gln Ala Leu Leu Glu Arg Ala Ser Ala Thr Leu Gln Asp Leu Val
       370                     375                     380
   Phe Asp Glu Cys Gly Ile Thr Asp Asp Gln Leu Leu Ala Leu Leu Pro
   385                     390                     395                     400
   Ser Leu Ser His Cys Ser Gln Leu Thr Thr Leu Ser Phe Tyr Gly Asn
                   405                     410                     415
   Ser Ile Ser Ile Ser Ala Leu Gln Ser Leu Leu Gln His Leu Ile Gly
               420                     425                     430
   Leu Ser Asn Leu Thr His Val Leu Tyr Pro Val Pro Leu Glu Ser Tyr
           435                     440                     445
   Glu Asp Ile His Gly Thr Leu His Leu Glu Arg Leu Ala Tyr Leu His
       450                     455                     460
   Ala Arg Leu Arg Glu Leu Leu Cys Glu Leu Gly Arg Pro Ser Met Val
   465                     470                     475                     480
   Trp Leu Ser Ala Asn Pro Cys Pro His Cys Gly Asp Arg Thr Phe Tyr
                   485                     490                     495
   Asp Pro Glu Pro Ile Leu Cys Pro Cys Phe Met Pro Asn
               500                     505
<210> 9
   <211> 1255
   <212> PRT
   <213> Homo sapiens
<400> 9
<210> 10
   <211> 261
   <212> PRT
   <213> Homo sapiens
<400> 10
   Met Trp Val Pro Val Val Phe Leu Thr Leu Ser Val Thr Trp Ile Gly
    1               5                  10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Asn Lys Ser Val Ile Leu Leu Gly Arg His Ser Leu
   65                  70                  75                  80
   Phe His Pro Glu Asp Thr Gly Gln Val Phe Gln Val Ser His Ser Phe
                   85                  90                  95
   Pro His Pro Leu Tyr Asp Met Ser Leu Leu Lys Asn Arg Phe Leu Arg
               100                 105                 110
   Pro Gly Asp Asp Ser Ser His Asp Leu Met Leu Leu Arg Leu Ser Glu
           115                 120                 125
   Pro Ala Glu Leu Thr Asp Ala Val Lys Val Met Asp Leu Pro Thr Gln
       130                 135                 140
   Glu Pro Ala Leu Gly Thr Thr Cys Tyr Ala Ser Gly Trp Gly Ser Ile
   145                 150                 155                 160
   Glu Pro Glu Glu Phe Leu Thr Pro Lys Lys Leu Gln Cys Val Asp Leu
                   165                 170                 175
   His Val Ile Ser Asn Asp Val Cys Ala Gln Val His Pro Gln Lys Val
               180                 185                 190
   Thr Lys Phe Met Leu Cys Ala Gly Arg Trp Thr Gly Gly Lys Ser Thr
           195                 200                 205
   Cys Ser Gly Asp Ser Gly Gly Pro Leu Val Cys Asn Gly Val Leu Gln
       210                 215                 220
   Gly Ile Thr Ser Trp Gly Ser Glu Pro Cys Ala Leu Pro Glu Arg Pro
   225                 230                 235                 240
   Ser Leu Tyr Thr Lys Val Val His Tyr Arg Lys Trp Ile Lys Asp Thr
                   245                 250                 255
   Ile Val Ala Asn Pro
               260
<210> 11
   <211> 115
   <212> PRT
   <213> Homo sapiens
<400> 11
   Met Ser Gly Glu Pro Gly Gln Thr Ser Val Ala Pro Pro Pro Glu Glu
   1 5 10 15
   Val Glu Pro Gly Ser Gly Val Arg Ile Val Val Glu Tyr Cys Glu Pro
   20 25 30
   Cys Gly Phe Glu Ala Thr Tyr Leu Glu Leu Ala Ser Ala Val Lys Glu
   35 40 45
   Gln Tyr Pro Gly Ile Glu Ile Glu Ser Arg Leu Gly Gly Thr Gly Ala
   50 55 60
   Phe Glu Ile Glu Ile Asn Gly Gln Leu Val Phe Ser Lys Leu Glu Asn
   65 70 75 80
   Gly Gly Phe Pro Tyr Glu Lys Asp Leu Ile Glu Ala Ile Arg Arg Ala
   85 90 95
   Ser Asn Gly Glu Thr Leu Glu Lys Ile Thr Asn Ser Arg Pro Pro Cys
   100 105 110
   Val Ile Leu
   115
<210> 12
   <211> 153
   <212> PRT
   <213> Homo sapiens
<400> 12
   Met Asn Gly Asp Asp Ala Phe Ala Arg Arg Pro Arg Asp Asp Ala Gln
   1 5 10 15
   Ile Ser Glu Lys Leu Arg Lys Ala Phe Asp Asp Ile Ala Lys Tyr Phe
   20 25 30
   Ser Lys Lys Glu Trp Glu Lys Met Lys Ser Ser Glu Lys Ile Val Tyr
   35 40 45
   Val Tyr Met Lys Leu Asn Tyr Glu Val Met Thr Lys Leu Gly Phe Lys
   50 55 60
   Val Thr Leu Pro Pro Phe Met Arg Ser Lys Arg Ala Ala Asp Phe His
   65 70 75 80
   Gly Asn Asp Phe Gly Asn Asp Arg Asn His Arg Asn Gln Val Glu Arg
   85 90 95
   Pro Gln Met Thr Phe Gly Ser Leu Gln Arg Ile Phe Pro Lys Asp Pro
   100 105 110
   Lys Gly Gly Asn Met Pro Gly Pro Thr Asp Cys Val Arg Glu Ser Ser
   115 120 125
   Trp Trp Phe Met Lys Arg Ser Ala Thr Leu Arg Lys Met Thr Ser Asn
   130 135 140
   Ser Pro Arg Gly Tyr Asp Thr Cys Pro
   145 150
<210> 13
   <211> 661
   <212> PRT
   <213> Homo sapiens
<400> 13
   Met Asp Leu Val Leu Lys Arg Cys Leu Leu His Leu Ala Val Ile Gly
    1               5                  10                  15
   Ala Leu Leu Ala Val Gly Ala Thr Lys Val Pro Arg Asn Gln Asp Trp
               20                  25                  30
   Leu Gly Val Ser Arg Gln Leu Arg Thr Lys Ala Trp Asn Arg Gln Leu
           35                  40                  45
   Tyr Pro Glu Trp Thr Glu Ala Gln Arg Leu Asp Cys Trp Arg Gly Gly
       50                  55                  60
   Gln Val Ser Leu Lys Val Ser Asn Asp Gly Pro Thr Leu Ile Gly Ala
   65                  70                  75                  80
   Asn Ala Ser Phe Ser Ile Ala Leu Asn Phe Pro Gly Ser Gln Lys Val
                   85                  90                  95
   Leu Pro Asp Gly Gln Val Ile Trp Val Asn Asn Thr Ile Ile Asn Gly
               100                 105                 110
   Ser Gln Val Trp Gly Gly Gln Pro Val Tyr Pro Gln Glu Thr Asp Asp
           115                 120                 125
   Ala Cys Ile Phe Pro Asp Gly Gly Pro Cys Pro Ser Gly Ser Trp Ser
       130                 135                 140
   Gln Lys Arg Ser Phe Val Tyr Val Trp Lys Thr Trp Gly Gln Tyr Trp
   145                 150                 155                 160
   Gln Val Leu Gly Gly Pro Val Ser Gly Leu Ser Ile Gly Thr Gly Arg
                   165                 170                 175
   Ala Met Leu Gly Thr His Thr Met Glu Val Thr Val Tyr His Arg Arg
               180                 185                 190
   Gly Ser Arg Ser Tyr Val Pro Leu Ala His Ser Ser Ser Ala Phe Thr
           195                 200                 205
   Ile Thr Asp Gln Val Pro Phe Ser Val Ser Val Ser Gln Leu Arg Ala
       210                 215                 220
   Leu Asp Gly Gly Asn Lys His Phe Leu Arg Asn Gln Pro Leu Thr Phe
   225                 230                 235                 240
   Ala Leu Gln Leu His Asp Pro Ser Gly Tyr Leu Ala Glu Ala Asp Leu
                   245                 250                 255
   Ser Tyr Thr Trp Asp Phe Gly Asp Ser Ser Gly Thr Leu Ile Ser Arg
               260                 265                 270
   Ala Leu Val Val Thr His Thr Tyr Leu Glu Pro Gly Pro Val Thr Ala
           275                 280                 285
   Gln Val Val Leu Gln Ala Ala Ile Pro Leu Thr Ser Cys Gly Ser Ser
       290                 295                 300
   Pro Val Pro Gly Thr Thr Asp Gly His Arg Pro Thr Ala Glu Ala Pro
   305                 310                 315                 320
   Asn Thr Thr Ala Gly Gln Val Pro Thr Thr Glu Val Val Gly Thr Thr
                   325                 330                 335
   Pro Gly Gln Ala Pro Thr Ala Glu Pro Ser Gly Thr Thr Ser Val Gln
               340                 345                 350
   Val Pro Thr Thr Glu Val Ile Ser Thr Ala Pro Val Gln Met Pro Thr
           355                 360                 365
   Ala Glu Ser Thr Gly Met Thr Pro Glu Lys Val Pro Val Ser Glu Val
       370                 375                 380
   Met Gly Thr Thr Leu Ala Glu Met Ser Thr Pro Glu Ala Thr Gly Met
   385                 390                 395                 400
   Thr Pro Ala Glu Val Ser Ile Val Val Leu Ser Gly Thr Thr Ala Ala
                   405                 410                 415
   Gln Val Thr Thr Thr Glu Trp Val Glu Thr Thr Ala Arg Glu Leu Pro
               420                 425                 430
   Ile Pro Glu Pro Glu Gly Pro Asp Ala Ser Ser Ile Met Ser Thr Glu
           435                 440                 445
   Ser Ile Thr Gly Ser Leu Gly Pro Leu Leu Asp Gly Thr Ala Thr Leu
       450                 455                 460
   Arg Leu Val Lys Arg Gln Val Pro Leu Asp Cys Val Leu Tyr Arg Tyr
   465                 470                 475                 480
   Gly Ser Phe Ser Val Thr Leu Asp Ile Val Gln Gly Ile Glu Ser Ala
                   485                 490                 495
   Glu Ile Leu Gln Ala Val Pro Ser Gly Glu Gly Asp Ala Phe Glu Leu
               500                 505                 510
   Thr Val Ser Cys Gln Gly Gly Leu Pro Lys Glu Ala Cys Met Glu Ile
           515                 520                 525
   Ser Ser Pro Gly Cys Gln Pro Pro Ala Gln Arg Leu Cys Gln Pro Val
       530                 535                 540
   Leu Pro Ser Pro Ala Cys Gln Leu Val Leu His Gln Ile Leu Lys Gly
   545                 550                 555                 560
   Gly Ser Gly Thr Tyr Cys Leu Asn Val Ser Leu Ala Asp Thr Asn Ser
                   565                 570                 575
   Leu Ala Val Val Ser Thr Gln Leu Ile Met Pro Gly Gln Glu Ala Gly
               580                 585                 590
   Leu Gly Gln Val Pro Leu Ile Val Gly Ile Leu Leu Val Leu Met Ala
           595                 600                 605
   Val Val Leu Ala Ser Leu Ile Tyr Arg Arg Arg Leu Met Lys Gln Asp
       610                 615                 620
   Phe Ser Val Pro Gln Leu Pro His Ser Ser Ser His Trp Leu Arg Leu
   625                 630                 635                 640
   Pro Arg Ile Phe Cys Ser Cys Pro Ile Gly Glu Asn Ser Pro Leu Leu
   645 650 655
   Ser Gly Gln Gln Val
   660
<210> 14
   <211> 371
   <212> PRT
   <213> Homo sapiens
<400> 14
   Met Ser Met Ser Pro Lys His Thr Thr Pro Phe Ser Val Ser Asp Ile
   1               5                   10                  15
   Leu Ser Pro Leu Glu Glu Ser Tyr Lys Lys Val Gly Met Glu Gly Gly
               20                  25                  30
   Gly Leu Gly Ala Pro Leu Ala Ala Tyr Arg Gln Gly Gln Ala Ala Pro
           35                  40                  45
   Pro Thr Ala Ala Met Gln Gln His Ala Val Gly His His Gly Ala Val
       50                  55                  60
   Thr Ala Ala Tyr His Met Thr Ala Ala Gly Val Pro Gln Leu Ser His
   65                  70                  75                  80
   Ser Ala Val Gly Gly Tyr Cys Asn Gly Asn Leu Gly Asn Met Ser Glu
                   85                  90                  95
   Leu Pro Pro Tyr Gln Asp Thr Met Arg Asn Ser Ala Ser Gly Pro Gly
               100                 105                 110
   Trp Tyr Gly Ala Asn Pro Asp Pro Arg Phe Pro Ala Ile Ser Arg Phe
           115                 120                 125
   Met Gly Pro Ala Ser Gly Met Asn Met Ser Gly Met Gly Gly Leu Gly
       130                 135                 140
   Ser Leu Gly Asp Val Ser Lys Asn Met Ala Pro Leu Pro Ser Ala Pro
   145                 150                 155                 160
   Arg Arg Lys Arg Arg Val Leu Phe Ser Gln Ala Gln Val Tyr Glu Leu
                   165                 170                 175
   Glu Arg Arg Phe Lys Gln Gln Lys Tyr Leu Ser Ala Pro Glu Arg Glu
               180                 185                 190
   His Leu Ala Ser Met Ile His Leu Thr Pro Thr Gln Val Lys Ile Trp
           195                 200                 205
   Phe Gln Asn His Arg Tyr Lys Met Lys Arg Gln Ala Lys Asp Lys Ala
       210                 215                 220
   Ala Gln Gln Gln Leu Gln Gln Asp Ser Gly Gly Gly Gly Gly Gly Gly
   225                 230                 235                 240
   Gly Thr Gly Cys Pro Gln Gln Gln Gln Ala Gln Gln Gln Ser Pro Arg
                   245                 250                 255
   Arg Val Ala Val Pro Val Leu Val Lys Asp Gly Lys Pro Cys Gln Ala
               260                 265                 270
   Gly Ala Pro Ala Pro Gly Ala Ala Ser Leu Gln Gly His Ala Gln Gln
           275                 280                 285
   Gln Ala Gln His Gln Ala Gln Ala Ala Gln Ala Ala Ala Ala Ala Ile
       290                 295                 300
   Ser Val Gly Ser Gly Gly Ala Gly Leu Gly Ala His Pro Gly His Gln
   305                 310                 315                 320
   Pro Gly Ser Ala Gly Gln Ser Pro Asp Leu Ala His His Ala Ala Ser
                   325                 330                 335
   Pro Ala Ala Leu Gln Gly Gln Val Ser Ser Leu Ser His Leu Asn Ser
               340                 345                 350
   Ser Gly Ser Asp Tyr Gly Thr Met Ser Cys Ser Thr Leu Leu Tyr Gly
           355                 360                 365
   Arg Thr Trp
       370
<210> 15
   <211> 93
   <212> PRT
   <213> Homo sapiens
<400> 15
   Met Lys Leu Leu Met Val Leu Met Leu Ala Ala Leu Ser Gln His Cys
    1               5                  10                  15
   Tyr Ala Gly Ser Gly Cys Pro Leu Leu Glu Asn Val Ile Ser Lys Thr
               20                  25                  30
   Ile Asn Pro Gln Val Ser Lys Thr Glu Tyr Lys Glu Leu Leu Gln Glu
           35                  40                  45
   Phe Ile Asp Asp Asn Ala Thr Thr Asn Ala Ile Asp Glu Leu Lys Glu
       50                  55                  60
   Cys Phe Leu Asn Gln Thr Asp Glu Thr Leu Ser Asn Val Glu Val Phe
   65                  70                  75                  80
   Met Gln Leu Ile Tyr Asp Ser Ser Leu Cys Asp Leu Phe
                   85                  90
<210> 16
   <211> 146
   <212> PRT
   <213> Homo sapiens
<400> 16
   Met Arg Leu Leu Gln Leu Leu Phe Arg Ala Ser Pro Ala Thr Leu Leu
    1               5                  10                  15
   Leu Val Leu Cys Leu Gln Leu Gly Ala Asn Lys Ala Gln Asp Asn Thr
               20                  25                  30
   Arg Lys Ile Ile Ile Lys Asn Phe Asp Ile Pro Lys Ser Val Arg Pro
           35                  40                  45
   Asn Asp Glu Val Thr Ala Val Leu Ala Val Gln Thr Glu Leu Lys Glu
       50                  55                  60
   Cys Met Val Val Lys Thr Tyr Leu Ile Ser Ser Ile Pro Leu Gln Gly
   65                  70                  75                  80
   Ala Phe Asn Tyr Lys Tyr Thr Ala Cys Leu Cys Asp Asp Asn Pro Lys
                   85                  90                  95
   Thr Phe Tyr Trp Asp Phe Tyr Thr Asn Arg Thr Val Gln Ile Ala Ala
               100                 105                 110
   Val Val Asp Val Ile Arg Glu Leu Gly Ile Cys Pro Asp Asp Ala Ala
           115                 120                 125
   Val Ile Pro Ile Lys Asn Asn Arg Phe Tyr Thr Ile Glu Ile Leu Lys
       130                 135                 140
   Val Glu
   145
<210> 17
   <211> 622
   <212> PRT
   <213> Homo sapiens
<400> 17
   Met Ala Leu Pro Thr Ala Arg Pro Leu Leu Gly Ser Cys Gly Thr Pro
    1               5                  10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385                 390                 395                 400
   Val Asn Lys Gly His Glu Met Ser Pro Gln Val Ala Thr Leu Ile Asp
                   405                 410                 415
   Arg Phe Val Lys Gly Arg Gly Gln Leu Asp Lys Asp Thr Leu Asp Thr
               420                 425                 430
   Leu Thr Ala Phe Tyr Pro Gly Tyr Leu Cys Ser Leu Ser Pro Glu Glu
           435                 440                 445
   Leu Ser Ser Val Pro Pro Ser Ser Ile Trp Ala Val Arg Pro Gln Asp
       450                 455                 460
   Leu Asp Thr Cys Asp Pro Arg Gln Leu Asp Val Leu Tyr Pro Lys Ala
   465                 470                 475                 480
   Arg Leu Ala Phe Gln Asn Met Asn Gly Ser Glu Tyr Phe Val Lys Ile
                   485                 490                 495
   Gln Ser Phe Leu Gly Gly Ala Pro Thr Glu Asp Leu Lys Ala Leu Ser
               500                 505                 510
   Gln Gln Asn Val Ser Met Asp Leu Ala Thr Phe Met Lys Leu Arg Thr
           515                 520                 525
   Asp Ala Val Leu Pro Leu Thr Val Ala Glu Val Gln Lys Leu Leu Gly
       530                 535                 540
   Pro His Val Glu Gly Leu Lys Ala Glu Glu Arg His Arg Pro Val Arg
   545                 550                 555                 560
   Asp Trp Ile Leu Arg Gln Arg Gln Asp Asp Leu Asp Thr Leu Gly Leu
                           565                 570                 575
   Gly Leu Gln Gly Gly Ile Pro Asn Gly Tyr Leu Val Leu Asp Leu Ser
                   580                 585                 590
   Met Gln Glu Ala Leu Ser Gly Thr Pro Cys Leu Leu Gly Pro Gly Pro
               595                 600                 605
   Val Leu Thr Val Leu Ala Leu Leu Leu Ala Ser Thr Leu Ala
       610                 615                 620
<210> 18
   <211> 630
   <212> PRT
   <213> Homo sapiens
<400> 18
   Met Ala Leu Pro Thr Ala Arg Pro Leu Leu Gly Ser Cys Gly Thr Pro
   1 5 10 15
   Ala Leu Gly Ser Leu Leu Phe Leu Leu Phe Ser Leu Gly Trp Val Gln
   20 25 30
   Pro Ser Arg Thr Leu Ala Gly Glu Thr Gly Gln Glu Ala Ala Pro Leu
   35 40 45
   Asp Gly Val Leu Ala Asn Pro Pro Asn Ile Ser Ser Leu Ser Pro Arg
   50 55 60
   Gln Leu Leu Gly Phe Pro Cys Ala Glu Val Ser Gly Leu Ser Thr Glu
   65 70 75 80
   Arg Val Arg Glu Leu Ala Val Ala Leu Ala Gln Lys Asn Val Lys Leu
   85 90 95
   Ser Thr Glu Gln Leu Arg Cys Leu Ala His Arg Leu Ser Glu Pro Pro
   100 105 110
   Glu Asp Leu Asp Ala Leu Pro Leu Asp Leu Leu Leu Phe Leu Asn Pro
   115 120 125
   Asp Ala Phe Ser Gly Pro Gln Ala Cys Thr Arg Phe Phe Ser Arg Ile
   130 135 140
   Thr Lys Ala Asn Val Asp Leu Leu Pro Arg Gly Ala Pro Glu Arg Gln
   145 150 155 160
   Arg Leu Leu Pro Ala Ala Leu Ala Cys Trp Gly Val Arg Gly Ser Leu
   165 170 175
   Leu Ser Glu Ala Asp Val Arg Ala Leu Gly Gly Leu Ala Cys Asp Leu
   180 185 190
   Pro Gly Arg Phe Val Ala Glu Ser Ala Glu Val Leu Leu Pro Arg Leu
   195 200 205
   Val Ser Cys Pro Gly Pro Leu Asp Gln Asp Gln Gln Glu Ala Ala Arg
   210 215 220
   Ala Ala Leu Gln Gly Gly Gly Pro Pro Tyr Gly Pro Pro Ser Thr Trp
   225 230 235 240
   Ser Val Ser Thr Met Asp Ala Leu Arg Gly Leu Leu Pro Val Leu Gly
   245 250 255
   Gln Pro Ile Ile Arg Ser Ile Pro Gln Gly Ile Val Ala Ala Trp Arg
   260 265 270
   Gln Arg Ser Ser Arg Asp Pro Ser Trp Arg Gln Pro Glu Arg Thr Ile
   275 280 285
   Leu Arg Pro Arg Phe Arg Arg Glu Val Glu Lys Thr Ala Cys Pro Ser
   290 295 300
   Gly Lys Lys Ala Arg Glu Ile Asp Glu Ser Leu Ile Phe Tyr Lys Lys
   305 310 315 320
   Trp Glu Leu Glu Ala Cys Val Asp Ala Ala Leu Leu Ala Thr Gln Met
   325 330 335
   Asp Arg Val Asn Ala Ile Pro Phe Thr Tyr Glu Gln Leu Asp Val Leu
   340 345 350
   Lys His Lys Leu Asp Glu Leu Tyr Pro Gln Gly Tyr Pro Glu Ser Val
   355 360 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385                 390                 395                 400
   Val Asn Lys Gly His Glu Met Ser Pro Gln Ala Pro Arg Arg Pro Leu
                   405                 410                 415
   Pro Gln Val Ala Thr Leu Ile Asp Arg Phe Val Lys Gly Arg Gly Gln
               420                 425                 430
   Leu Asp Lys Asp Thr Leu Asp Thr Leu Thr Ala Phe Tyr Pro Gly Tyr
           435                 440                 445
   Leu Cys Ser Leu Ser Pro Glu Glu Leu Ser Ser Val Pro Pro Ser Ser
       450                 455                 460
   Ile Trp Ala Val Arg Pro Gln Asp Leu Asp Thr Cys Asp Pro Arg Gln
   465                 470                 475                 480
   Leu Asp Val Leu Tyr Pro Lys Ala Arg Leu Ala Phe Gln Asn Met Asn
                   485                 490                 495
   Gly Ser Glu Tyr Phe Val Lys Ile Gln Ser Phe Leu Gly Gly Ala Pro
               500                 505                 510
   Thr Glu Asp Leu Lys Ala Leu Ser Gln Gln Asn Val Ser Met Asp Leu
           515                 520                 525
   Ala Thr Phe Met Lys Leu Arg Thr Asp Ala Val Leu Pro Leu Thr Val
       530                 535                 540
   Ala Glu Val Gln Lys Leu Leu Gly Pro His Val Glu Gly Leu Lys Ala
   545                 550                 555                 560
   Glu Glu Arg His Arg Pro Val Arg Asp Trp Ile Leu Arg Gln Arg Gln
                   565                 570                 575
   Asp Asp Leu Asp Thr Leu Gly Leu Gly Leu Gln Gly Gly Ile Pro Asn
               580                 585                 590
   Gly Tyr Leu Val Leu Asp Leu Ser Met Gln Glu Ala Leu Ser Gly Thr
           595                 600                 605
   Pro Cys Leu Leu Gly Pro Gly Pro Val Leu Thr Val Leu Ala Leu Leu
       610                 615                 620
   Leu Ala Ser Thr Leu Ala
   625                 630
<210> 19
   <211> 123
   <212> PRT
   <213> Homo sapiens
<400> 19
   Met Lys Ala Val Leu Leu Ala Leu Leu Met Ala Gly Leu Ala Leu Gln
   1               5                   10                  15
   Pro Gly Thr Ala Leu Leu Cys Tyr Ser Cys Lys Ala Gln Val Ser Asn
               20                  25                  30
   Glu Asp Cys Leu Gln Val Glu Asn Cys Thr Gln Leu Gly Glu Gln Cys
           35                  40                  45
   Trp Thr Ala Arg Ile Arg Ala Val Gly Leu Leu Thr Val Ile Ser Lys
       50                  55                  60
   Gly Cys Ser Leu Asn Cys Val Asp Asp Ser Gln Asp Tyr Tyr Val Gly
   65                  70                  75                  80
   Lys Lys Asn Ile Thr Cys Cys Asp Thr Asp Leu Cys Asn Ala Ser Gly
                   85                  90                  95
   Ala His Ala Leu Gln Pro Ala Ala Ala Ile Leu Ala Leu Leu Pro Ala
               100                 105                 110
   Leu Gly Leu Leu Leu Trp Gly Pro Gly Gln Leu
           115                 120
<210> 20
   <211> 976
   <212> PRT
   <213> Homo sapiens
<400> 20
<210> 21
   <211> 2384
   <212> DNA
   <213> Homo sapiens
<400> 21
<210> 22
   <211> 1524
   <212> DNA
   <213> Homo sapiens
<400> 22
   agcagacaga ggactctcat taaggaaggt gtcctgtgcc ctgaccctac aagatgccaa 60
   gagaagatgc tcacttcatc tatggttacc ccaagaaggg gcacggccac tcttacacca 120
   cggctgaaga ggccgctggg atcggcatcc tgacagtgat cctgggagtc ttactgctca 180
   tcggctgttg gtattgtaga agacgaaatg gatacagagc cttgatggat aaaagtcttc 240
   atgttggcac tcaatgtgcc ttaacaagaa gatgcccaca agaagggttt gatcatcggg 300
   acagcaaagt gtctcttcaa gagaaaaact gtgaacctgt ggttcccaat gctccacctg 360
   cttatgagaa actctctgca gaacagtcac caccacctta ttcaccttaa gagccagcga 420
   gacacctgag acatgctgaa attatttctc tcacactttt gcttgaattt aatacagaca 480
   tctaatgttc tcctttggaa tggtgtagga aaaatgcaag ccatctctaa taataagtca 540
   gtgttaaaat tttagtaggt ccgctagcag tactaatcat gtgaggaaat gatgagaaat 600
   attaaattgg gaaaactcca tcaataaatg ttgcaatgca tgatactatc tgtgccagag 660
   gtaatgttag taaatccatg gtgttatttt ctgagagaca gaattcaagt gggtattctg 720
   gggccatcca atttctcttt acttgaaatt tggctaataa caaactagtc aggttttcga 780
   accttgaccg acatgaactg tacacagaat tgttccagta ctatggagtg ctcacaaagg 840
   atacttttac aggttaagac aaagggttga ctggcctatt tatctgatca agaacatgtc 900
   agcaatgtct ctttgtgctc taaaattcta ttatactaca ataatatatt gtaaagatcc 960
   tatagctctt tttttttgag atggagtttc gcttttgttg cccaggctgg agtgcaatgg 1020
   cgcgatcttg gctcaccata acctccgcct cccaggttca agcaattctc ctgccttagc 1080
   ctcctgagta gctgggatta caggcgtgcg ccactatgcc tgactaattt tgtagtttta 1140
   gtagagacgg ggtttctcca tgttggtcag gctggtctca aactcctgac ctcaggtgat 1200
   ctgcccgcct cagcctccca aagtgctgga attacaggcg tgagccacca cgcctggctg 1260
   gatcctatat cttaggtaag acatataacg cagtctaatt acatttcact tcaaggctca 1320
   atgctattct aactaatgac aagtattttc tactaaacca gaaattggta gaaggattta 1380
   aataagtaaa agctactatg tactgcctta gtgctgatgc ctgtgtactg ccttaaatgt 1440
   acctatggca atttagctct cttgggttcc caaatccctc tcacaagaat gtgcagaaga 1500
   aatcataaag gatcagagat tctg                                        1524
<210> 23
   <211> 1466
   <212> DNA
   <213> Homo sapiens
<400> 23
   gcatgctctg actttctctc tctttcgatt cttccatact cagagtacgc acggtctgat 60
   tttctctttg gattcttcca aaatcagagt cagactgctc ccggtgccat gaacggagac 120
   gacgcctttg caaggagacc cacggttggt gctcaaatac cagagaagat ccaaaaggcc 180
   ttcgatgata ttgccaaata cttctctaag gaagagtggg aaaagatgaa agcctcggag 240
   aaaatcttct atgtgtatat gaagagaaag tatgaggcta tgactaaact aggtttcaag 300
   gccaccctcc cacctttcat gtgtaataaa cgggccgaag acttccaggg gaatgatttg 360
   gataatgacc ctaaccgtgg gaatcaggtt gaacgtcctc agatgacttt cggcaggctc 420
   cagggaatct ccccgaagat catgcccaag aagccagcag aggaaggaaa tgattcggag 480
   gaagtgccag aagcatctgg cccacaaaat gatgggaaag agctgtgccc cccgggaaaa 540
   ccaactacct ctgagaagat tcacgagaga tctggaaata gggaggccca agaaaaggaa 600
   gagagacgcg gaacagctca tcggtggagc agtcagaaca cacacaacat tggtcgattc 660
   agtttgtcaa cttctatggg tgcagttcat ggtaccccca aaacaattac acacaacagg 720
   gacccaaaag gggggaacat gcctggaccc acagactgcg tgagagaaaa cagctggtga 780
   tttatgaaga gatcagcgac cctgaggaag atgacgagta actcccctca gggatacgac 840
   acatgcccat gatgagaagc agaacgtggt gacctttcac gaacatgggc atggctgcgg 900
   acccctcgtc atcaggtgca tagcaagtga aagcaagtgt tcacaacagt gaaaagttga 960
   gcgtcatttt tcttagtgtg ccaagagttc gatgttagcg tttacgttgt attttcttac 1020
   actgtgtcat tctgttagat actaacattt tcattgatga gcaagacata cttaatgcat 1080
   attttggttt gtgtatccat gcacctacct tagaaaacaa gtattgtcgg ttacctctgc 1140
   atggaacagc attaccctcc tctctcccca gatgtgacta ctgagggcag ttctgagtgt 1200
   ttaatttcag attttttcct ctgcatttac acacacacgc acacaaacca caccacacac 1260
   acacacacac acacacacac acacacacac acacaccaag taccagtata agcatctgcc 1320
   atctgctttt cccattgcca tgcgtcctgg tcaagctccc ctcactctgt ttcctggtca 1380
   gcatgtactc ccctcatccg attcccctgt agcagtcact gacagttaat aaacctttgc 1440
   aaacgttcaa aaaaaaaaaa aaaaaa 1466
<210> 24
   <211> 2653
   <212> DNA
   <213> Homo sapiens
<400> 24
   ctcaaaaggg gccggatttc cttctcctgg aggcagatgt tgcctctctc tctcgctcgg 60
   attggttcag tgcactctag aaacactgct gtggtggaga aactggaccc caggtctgga 120
   gcgaattcca gcctgcaggg ctgataagcg aggcattagt gagattgaga gagactttac 180
   cccgccgtgg tggttggagg gcgcgcagta gagcagcagc acaggcgcgg gtcccgggag 240
   gccggctctg ctcgcgccga gatgtggaat ctccttcacg aaaccgactc ggctgtggcc 300
   accgcgcgcc gcccgcgctg gctgtgcgct ggggcgctgg tgctggcggg tggcttcttt 360
   ctcctcggct tcctcttcgg gtggtttata aaatcctcca atgaagctac taacattact 420
   ccaaagcata atatgaaagc atttttggat gaattgaaag ctgagaacat caagaagttc 480
   ttatataatt ttacacagat accacattta gcaggaacag aacaaaactt tcagcttgca 540
   aagcaaattc aatcccagtg gaaagaattt ggcctggatt ctgttgagct agcacattat 600
   gatgtcctgt tgtcctaccc aaataagact catcccaact acatctcaat aattaatgaa 660
   gatggaaatg agattttcaa cacatcatta tttgaaccac ctcctccagg atatgaaaat 720
   gtttcggata ttgtaccacc tttcagtgct ttctctcctc aaggaatgcc agagggcgat 780
   ctagtgtatg ttaactatgc acgaactgaa gacttcttta aattggaacg ggacatgaaa 840
   atcaattgct ctgggaaaat tgtaattgcc agatatggga aagttttcag aggaaataag 900
   gttaaaaatg cccagctggc aggggccaaa ggagtcattc tctactccga ccctgctgac 960
   tactttgctc ctggggtgaa gtcctatcca gatggttgga atcttcctgg aggtggtgtc 1020
   cagcgtggaa atatcctaaa tctgaatggt gcaggagacc ctctcacacc aggttaccca 1080
   gcaaatgaat atgcttatag gcgtggaatt gcagaggctg ttggtcttcc aagtattcct 1140
   gttcatccaa ttggatacta tgatgcacag aagctcctag aaaaaatggg tggctcagca 1200
   ccaccagata gcagctggag aggaagtctc aaagtgccct acaatgttgg acctggcttt 1260
   actggaaact tttctacaca aaaagtcaag atgcacatcc actctaccaa tgaagtgaca 1320
   agaatttaca atgtgatagg tactctcaga ggagcagtgg aaccagacag atatgtcatt 1380
   ctgggaggtc accgggactc atgggtgttt ggtggtattg accctcagag tggagcagct 1440
   gttgttcatg aaattgtgag gagctttgga acactgaaaa aggaagggtg gagacctaga 1500
   agaacaattt tgtttgcaag ctgggatgca gaagaatttg gtcttcttgg ttctactgag 1560
   tgggcagagg agaattcaag actccttcaa gagcgtggcg tggcttatat taatgctgac 1620
   tcatctatag aaggaaacta cactctgaga gttgattgta caccgctgat gtacagcttg 1680
   gtacacaacc taacaaaaga gctgaaaagc cctgatgaag gctttgaagg caaatctctt 1740
   tatgaaagtt ggactaaaaa aagtccttcc ccagagttca gtggcatgcc caggataagc 1800
   aaattgggat ctggaaatga ttttgaggtg ttcttccaac gacttggaat tgcttcaggc 1860
   agagcacggt atactaaaaa ttgggaaaca aacaaattca gcggctatcc actgtatcac 1920
   agtgtctatg aaacatatga gttggtggaa aagttttatg atccaatgtt taaatatcac 1980
   ctcactgtgg cccaggttcg aggagggatg gtgtttgagc tagccaattc catagtgctc 2040
   ccttttgatt gtcgagatta tgctgtagtt ttaagaaagt atgctgacaa aatctacagt 2100
   atttctatga aacatccaca ggaaatgaag acatacagtg tatcatttga ttcacttttt 2160
   tctgcagtaa agaattttac agaaattgct tccaagttca gtgagagact ccaggacttt 2220
   gacaaaagca acccaatagt attaagaatg atgaatgatc aactcatgtt tctggaaaga 2280
   gcatttattg atccattagg gttaccagac aggccttttt ataggcatgt catctatgct 2340
   ccaagcagcc acaacaagta tgcaggggag tcattcccag gaatttatga tgctctgttt 2400
   gatattgaaa gcaaagtgga cccttccaag gcctggggag aagtgaagag acagatttat 2460
   gttgcagcct tcacagtgca ggcagctgca gagactttga gtgaagtagc ctaagaggat 2520
   tctttagaga atccgtattg aatttgtgtg gtatgtcact cagaaagaat cgtaatgggt 2580
   atattgataa attttaaaat tggtatattt gaaataaagt tgaatattat atataaaaaa 2640
   aaaaaaaaaa aaa                                                     2653
<210> 25
   <211> 2420
   <212> DNA
   <213> Homo sapiens
<400> 25
   ggatccaggc cctgccagga aaaatataag ggccctgcgt gagaacagag ggggtcatcc 60
   actgcatgag agtggggatg tcacagagtc cagcccaccc tcctggtagc actgagaagc 120
   cagggctgtg cttgcggtct gcaccctgag ggcccgtgga ttcctcttcc tggagctcca 180
   ggaaccaggc agtgaggcct tggtctgaga cagtatcctc aggtcacaga gcagaggatg 240
   cacagggtgt gccagcagtg aatgtttgcc ctgaatgcac accaagggcc ccacctgcca 300
   caggacacat aggactccac agagtctggc ctcacctccc tactgtcagt cctgtagaat 360
   cgacctctgc tggccggctg taccctgagt accctctcac ttcctccttc aggttttcag 420
   gggacaggcc aacccagagg acaggattcc ctggaggcca cagaggagca ccaaggagaa 480
   gatctgtaag taggcctttg ttagagtctc caaggttcag ttctcagctg aggcctctca 540
   cacactccct ctctccccag gcctgtgggt cttcattgcc cagctcctgc ccacactcct 600
   gcctgctgcc ctgacgagag tcatcatgtc tcttgagcag aggagtctgc actgcaagcc 660
   tgaggaagcc cttgaggccc aacaagaggc cctgggcctg gtgtgtgtgc aggctgccac 720
   ctcctcctcc tctcctctgg tcctgggcac cctggaggag gtgcccactg ctgggtcaac 780
   agatcctccc cagagtcctc agggagcctc cgcctttccc actaccatca acttcactcg 840
   acagaggcaa cccagtgagg gttccagcag ccgtgaagag gaggggccaa gcacctcttg 900
   tatcctggag tccttgttcc gagcagtaat cactaagaag gtggctgatt tggttggttt 960
   tctgctcctc aaatatcgag ccagggagcc agtcacaaag gcagaaatgc tggagagtgt 1020
   catcaaaaat tacaagcact gttttcctga gatcttcggc aaagcctctg agtccttgca 1080
   gctggtcttt ggcattgacg tgaaggaagc agaccccacc ggccactcct atgtccttgt 1140
   cacctgccta ggtctctcct atgatggcct gctgggtgat aatcagatca tgcccaagac 1200
   aggcttcctg ataattgtcc tggtcatgat tgcaatggag ggcggccatg ctcctgagga 1260
   ggaaatctgg gaggagctga gtgtgatgga ggtgtatgat gggagggagc acagtgccta 1320
   tggggagccc aggaagctgc tcacccaaga tttggtgcag gaaaagtacc tggagtaccg 1380
   gcaggtgccg gacagtgatc ccgcacgcta tgagttcctg tggggtccaa gggccctcgc 1440
   tgaaaccagc tatgtgaaag tccttgagta tgtgatcaag gtcagtgcaa gagttcgctt 1500
   tttcttccca tccctgcgtg aagcagcttt gagagaggag gaagagggag tctgagcatg 1560
   agttgcagcc aaggccagtg ggagggggac tgggccagtg caccttccag ggccgcgtcc 1620
   agcagcttcc cctgcctcgt gtgacatgag gcccattctt cactctgaag agagcggtca 1680
   gtgttctcag tagtaggttt ctgttctatt gggtgacttg gagatttatc tttgttctct 1740
   tttggaattg ttcaaatgtt tttttttaag ggatggttga atgaacttca gcatccaagt 1800
   ttatgaatga cagcagtcac acagttctgt gtatatagtt taagggtaag agtcttgtgt 1860
   tttattcaga ttgggaaatc cattctattt tgtgaattgg gataataaca gcagtggaat 1920
   aagtacttag aaatgtgaaa aatgagcagt aaaatagatg agataaagaa ctaaagaaat 1980
   taagagatag tcaattcttg ccttatacct cagtctattc tgtaaaattt ttaaagatat 2040
   atgcatacct ggatttcctt ggcttctttg agaatgtaag agaaattaaa tctgaataaa 2100
   gaattcttcc tgttcactgg ctcttttctt ctccatgcac tgagcatctg ctttttggaa 2160
   ggccctgggt tagtagtgga gatgctaagg taagccagac tcatacccac ccatagggtc 2220
   gtagagtcta ggagctgcag tcacgtaatc gaggtggcaa gatgtcctct aaagatgtag 2280
   ggaaaagtga gagaggggtg agggtgtggg gctccgggtg agagtggtgg agtgtcaatg 2340
   ccctgagctg ggcatttttg ggctttggga aactgcagtt ccttctgggg gagctgattg 2400
   taatgatctt gggtggatcc 2420
<210> 26
   <211> 4204
   <212> DNA
   <213> Homo sapiens
<400> 26
   acgcaggcag tgatgtcacc cagaccacac cccttccccc aatgccactt cagggggtac 60
   tcagagtcag agacttggtc tgaggggagc agaagcaatc tgcagaggat ggcggtccag 120
   gctcagccag gcatcaactt caggaccctg agggatgacc gaaggccccg cccacccacc 180
   cccaactccc ccgaccccac caggatctac agcctcagga cccccgtccc aatccttacc 240
   ccttgcccca tcaccatctt catgcttacc tccaccccca tccgatcccc atccaggcag 300
   aatccagttc cacccctgcc cggaacccag ggtagtaccg ttgccaggat gtgacgccac 360
   tgacttgcgc attggaggtc agaagaccgc gagattctcg ccctgagcaa cgagcgacgg 420
   cctgacgtcg gcggagggaa gccggcccag gctcggtgag gaggcaaggt aagacgctga 480
   gggaggactg aggcgggcct cacctcagac agagggcctc aaataatcca gtgctgcctc 540
   tgctgccggg cctgggccac cccgcagggg aagacttcca ggctgggtcg ccactacctc 600
   accccgccga cccccgccgc tttagccacg gggaactctg gggacagagc ttaatgtggc 660
   cagggcaggg ctggttagaa gaggtcaggg cccacgctgt ggcaggaatc aaggtcagga 720
   ccccgagagg gaactgaggg cagcctaacc accaccctca ccaccattcc cgtcccccaa 780
   cacccaaccc cacccccatc ccccattccc atccccaccc ccacccctat cctggcagaa 840
   tccgggcttt gcccctggta tcaagtcacg gaagctccgg gaatggcggc caggcacgtg 900
   agtcctgagg ttcacatcta cggctaaggg agggaagggg ttcggtatcg cgagtatggc 960
   cgttgggagg cagcgaaagg gcccaggcct cctggaagac agtggagtcc tgaggggacc 1020
   cagcatgcca ggacaggggg cccactgtac ccctgtctca aaccgaggca ccttttcatt 1080
   cggctacggg aatcctaggg atgcagaccc acttcagcag ggggttgggg cccagccctg 1140
   cgaggagtca tggggaggaa gaagagggag gactgagggg accttggagt ccagatcagt 1200
   ggcaaccttg ggctggggga tgctgggcac agtggccaaa tgtgctctgt gctcattgcg 1260
   ccttcagggt gaccagagag ttgagggctg tggtctgaag agtgggactt caggtcagca 1320
   gagggaggaa tcccaggatc tgcagggccc aaggtgtacc cccaaggggc ccctatgtgg 1380
   tggacagatg cagtggtcct aggatctgcc aagcatccag gtgaagagac tgagggagga 1440
   ttgagggtac ccctgggaca gaatgcggac tgggggcccc ataaaaatct gccctgctcc 1500
   tgctgttacc tcagagagcc tgggcagggc tgtcagctga ggtccctcca ttatcctagg 1560
   atcactgatg tcagggaagg ggaagccttg gtctgagggg gctgcactca gggcagtaga 1620
   gggaggctct cagaccctac taggagtgga ggtgaggacc aagcagtctc ctcacccagg 1680
   gtacatggac ttcaataaat ttggacatct ctcgttgtcc tttccgggag gacctgggaa 1740
   tgtatggcca gatgtgggtc ccctcatgtt tttctgtacc atatcaggta tgtgagttct 1800
   tgacatgaga gattctcagg ccagcagaag ggagggatta ggccctataa ggagaaaggt 1860
   gagggccctg agtgagcaca gaggggatcc tccaccccag tagagtgggg acctcacaga 1920
   gtctggccaa ccctcctgac agttctggga atccgtggct gcgtttgctg tctgcacatt 1980
   gggggcccgt ggattcctct cccaggaatc aggagctcca ggaacaaggc agtgaggact 2040
   tggtctgagg cagtgtcctc aggtcacaga gtagaggggg ctcagatagt gccaacggtg 2100
   aaggtttgcc ttggattcaa accaagggcc ccacctgccc cagaacacat ggactccaga 2160
   gcgcctggcc tcaccctcaa tactttcagt cctgcagcct cagcatgcgc tggccggatg 2220
   taccctgagg tgccctctca cttcctcctt caggttctga ggggacaggc tgacctggag 2280
   gaccagaggc ccccggagga gcactgaagg agaagatctg taagtaagcc tttgttagag 2340
   cctccaaggt tccattcagt actcagctga ggtctctcac atgctccctc tctccccagg 2400
   ccagtgggtc tccattgccc agctcctgcc cacactcccg cctgttgccc tgaccagagt 2460
   catcatgcct cttgagcaga ggagtcagca ctgcaagcct gaagaaggcc ttgaggcccg 2520
   aggagaggcc ctgggcctgg tgggtgcgca ggctcctgct actgaggagc aggaggctgc 2580
   ctcctcctct tctactctag ttgaagtcac cctgggggag gtgcctgctg ccgagtcacc 2640
   agatcctccc cagagtcctc agggagcctc cagcctcccc actaccatga actaccctct 2700
   ctggagccaa tcctatgagg actccagcaa ccaagaagag gaggggccaa gcaccttccc 2760
   tgacctggag tccgagttcc aagcagcact cagtaggaag gtggccgagt tggttcattt 2820
   tctgctcctc aagtatcgag ccagggagcc ggtcacaaag gcagaaatgc tggggagtgt 2880
   cgtcggaaat tggcagtatt tctttcctgt gatcttcagc aaagcttcca gttccttgca 2940
   gctggtcttt ggcatcgagc tgatggaagt ggaccccatc ggccacttgt acatctttgc 3000
   cacctgcctg ggcctctcct acgatggcct gctgggtgac aatcagatca tgcccaaggc 3060
   aggcctcctg ataatcgtcc tggccataat cgcaagagag ggcgactgtg cccctgagga 3120
   gaaaatctgg gaggagctga gtgtgttaga ggtgtttgag gggagggaag acagtatctt 3180
   gggggatccc aagaagctgc tcacccaaca tttcgtgcag gaaaactacc tggagtaccg 3240
   gcaggtcccc ggcagtgatc ctgcatgtta tgaattcctg tggggtccaa gggccctcgt 3300
   tgaaaccagc tatgtgaaag tcctgcacca tatggtaaag atcagtggag gacctcacat 3360
   ttcctaccca cccctgcatg agtgggtttt gagagagggg gaagagtgag tctgagcacg 3420
   agttgcagcc agggccagtg ggagggggtc tgggccagtg caccttccgg ggccgcatcc 3480
   cttagtttcc actgcctcct gtgacgtgag gcccattctt cactctttga agcgagcagt 3540
   cagcattctt agtagtgggt ttctgttctg ttggatgact ttgagattat tctttgtttc 3600
   ctgttggagt tgttcaaatg ttccttttaa cggatggttg aatgagcgtc agcatccagg 3660
   tttatgaatg acagtagtca cacatagtgc tgtttatata gtttaggagt aagagtcttg 3720
   ttttttactc aaattgggaa atccattcca ttttgtgaat tgtgacataa taatagcagt 3780
   ggtaaaagta tttgcttaaa attgtgagcg aattagcaat aacatacatg agataactca 3840
   agaaatcaaa agatagttga ttcttgcctt gtacctcaat ctattctgta aaattaaaca 3900
   aatatgcaaa ccaggatttc cttgacttct ttgagaatgc aagcgaaatt aaatctgaat 3960
   aaataattct tcctcttcac tggctcgttt cttttccgtt cactcagcat ctgctctgtg 4020
   ggaggccctg ggttagtagt ggggatgcta aggtaagcca gactcacgcc tacccatagg 4080
   gctgtagagc ctaggacctg cagtcatata attaaggtgg tgagaagtcc tgtaagatgt 4140
   agaggaaatg taagagaggg gtgagggtgt ggcgctccgg gtgagagtag tggagtgtca 4200
   gtgc                                                              4204
<210> 27
   <211> 752
   <212> DNA
   <213> Homo sapiens
<400> 27
   atcctcgtgg gccctgacct tctctctgag agccgggcag aggctccgga gccatgcagg 60
   ccgaaggccg gggcacaggg ggttcgacgg gcgatgctga tggcccagga ggccctggca 120
   ttcctgatgg cccagggggc aatgctggcg gcccaggaga ggcgggtgcc acgggcggca 180
   gaggtccccg gggcgcaggg gcagcaaggg cctcggggcc gggaggaggc gccccgcggg 240
   gtccgcatgg cggcgcggct tcagggctga atggatgctg cagatgcggg gccagggggc 300
   cggagagccg cctgcttgag ttctacctcg ccatgccttt cgcgacaccc atggaagcag 360
   agctggcccg caggagcctg gcccaggatg ccccaccgct tcccgtgcca ggggtgcttc 420
   tgaaggagtt cactgtgtcc ggcaacatac tgactatccg actgactgct gcagaccacc 480
   gccaactgca gctctccatc agctcctgtc tccagcagct ttccctgttg atgtggatca 540
   cgcagtgctt tctgcccgtg tttttggctc agcctccctc agggcagagg cgctaagccc 600
   agcctggcgc cccttcctag gtcatgcctc ctcccctagg gaatggtccc agcacgagtg 660
   gccagttcat tgtgggggcc tgattgtttg tcgctggagg aggacggctt acatgtttgt 720
   ttctgtagaa aataaaactg agctacgaaa aa                               752
<210> 28
   <211> 2148
   <212> DNA
   <213> Homo sapiens
<400> 28
   gcttcagggt acagctcccc cgcagccaga agccgggcct gcagcgcctc agcaccgctc 60
   cgggacaccc cacccgcttc ccaggcgtga cctgtcaaca gcaacttcgc ggtgtggtga 120
   actctctgag gaaaaaccat tttgattatt actctcagac gtgcgtggca acaagtgact 180
   gagacctaga aatccaagcg ttggaggtcc tgaggccagc ctaagtcgct tcaaaatgga 240
   acgaaggcgt ttgtggggtt ccattcagag ccgatacatc agcatgagtg tgtggacaag 300
   cccacggaga cttgtggagc tggcagggca gagcctgctg aaggatgagg ccctggccat 360
   tgccgccctg gagttgctgc ccagggagct cttcccgcca ctcttcatgg cagcctttga 420
   cgggagacac agccagaccc tgaaggcaat ggtgcaggcc tggcccttca cctgcctccc 480
   tctgggagtg ctgatgaagg gacaacatct tcacctggag accttcaaag ctgtgcttga 540
   tggacttgat gtgctccttg cccaggaggt tcgccccagg aggtggaaac ttcaagtgct 600
   ggatttacgg aagaactctc atcaggactt ctggactgta tggtctggaa acagggccag 660
   tctgtactca tttccagagc cagaagcagc tcagcccatg acaaagaagc gaaaagtaga 720
   tggtttgagc acagaggcag agcagccctt cattccagta gaggtgctcg tagacctgtt 780
   cctcaaggaa ggtgcctgtg atgaattgtt ctcctacctc attgagaaag tgaagcgaaa 840
   gaaaaatgta ctacgcctgt gctgtaagaa gctgaagatt tttgcaatgc ccatgcagga 900
   tatcaagatg atcctgaaaa tggtgcagct ggactctatt gaagatttgg aagtgacttg 960
   tacctggaag ctacccacct tggcgaaatt ttctccttac ctgggccaga tgattaatct 1020
   gcgtagactc ctcctctccc acatccatgc atcttcctac atttccccgg agaaggaaga 1080
   gcagtatatc gcccagttca cctctcagtt cctcagtctg cagtgcctgc aggctctcta 1140
   tgtggactct ttatttttcc ttagaggccg cctggatcag ttgctcaggc acgtgatgaa 1200
   ccccttggaa accctctcaa taactaactg ccggctttcg gaaggggatg tgatgcatct 1260
   gtcccagagt cccagcgtca gtcagctaag tgtcctgagt ctaagtgggg tcatgctgac 1320
   cgatgtaagt cccgagcccc tccaagctct gctggagaga gcctctgcca ccctccagga 1380
   cctggtcttt gatgagtgtg ggatcacgga tgatcagctc cttgccctcc tgccttccct 1440
   gagccactgc tcccagctta caaccttaag cttctacggg aattccatct ccatatctgc 1500
   cttgcagagt ctcctgcagc acctcatcgg gctgagcaat ctgacccacg tgctgtatcc 1560
   tgtccccctg gagagttatg aggacatcca tggtaccctc cacctggaga ggcttgccta 1620
   tctgcatgcc aggctcaggg agttgctgtg tgagttgggg cggcccagca tggtctggct 1680
   tagtgccaac ccctgtcctc actgtgggga cagaaccttc tatgacccgg agcccatcct 1740
   gtgcccctgt ttcatgccta actagctggg tgcacatatc aaatgcttca ttctgcatac 1800
   ttggacacta aagccaggat gtgcatgcat cttgaagcaa caaagcagcc acagtttcag 1860
   acaaatgttc agtgtgagtg aggaaaacat gttcagtgag gaaaaaacat tcagacaaat 1920
   gttcagtgag gaaaaaaagg ggaagttggg gataggcaga tgttgacttg aggagttaat 1980
   gtgatctttg gggagataca tcttatagag ttagaaatag aatctgaatt tctaaaggga 2040
   gattctggct tgggaagtac atgtaggagt taatccctgt gtagactgtt gtaaagaaac 2100
   tgttgaaaat aaagagaagc aatgtgaagc aaaaaaaaaa aaaaaaaa 2148
<210> 29
   <211> 4530
   <212> DNA
   <213> Homo sapiens
<400> 29
   aattctcgag ctcgtcgacc ggtcgacgag ctcgagggtc gacgagctcg agggcgcgcg 60
   cccggccccc acccctcgca gcaccccgcg ccccgcgccc tcccagccgg gtccagccgg 120
   agccatgggg ccggagccgc agtgagcacc atggagctgg cggccttgtg ccgctggggg 180
   ctcctcctcg ccctcttgcc ccccggagcc gcgagcaccc aagtgtgcac cggcacagac 240
   atgaagctgc ggctccctgc cagtcccgag acccacctgg acatgctccg ccacctctac 300
   cagggctgcc aggtggtgca gggaaacctg gaactcacct acctgcccac caatgccagc 360
   ctgtccttcc tgcaggatat ccaggaggtg cagggctacg tgctcatcgc tcacaaccaa 420
   gtgaggcagg tcccactgca gaggctgcgg attgtgcgag gcacccagct ctttgaggac 480
   aactatgccc tggccgtgct agacaatgga gacccgctga acaataccac ccctgtcaca 540
   ggggcctccc caggaggcct gcgggagctg cagcttcgaa gcctcacaga gatcttgaaa 600
   ggaggggtct tgatccagcg gaacccccag ctctgctacc aggacacgat tttgtggaag 660
   gacatcttcc acaagaacaa ccagctggct ctcacactga tagacaccaa ccgctctcgg 720
   gcctgccacc cctgttctcc gatgtgtaag ggctcccgct gctggggaga gagttctgag 780
   gattgtcaga gcctgacgcg cactgtctgt gccggtggct gtgcccgctg caaggggcca 840
   ctgcccactg actgctgcca tgagcagtgt gctgccggct gcacgggccc caagcactct 900
   gactgcctgg cctgcctcca cttcaaccac agtggcatct gtgagctgca ctgcccagcc 960
   ctggtcacct acaacacaga cacgtttgag tccatgccca atcccgaggg ccggtataca 1020
   ttcggcgcca gctgtgtgac tgcctgtccc tacaactacc tttctacgga cgtgggatcc 1080
   tgcaccctcg tctgccccct gcacaaccaa gaggtgacag cagaggatgg aacacagcgg 1140
   tgtgagaagt gcagcaagcc ctgtgcccga gtgtgctatg gtctgggcat ggagcacttg 1200
   cgagaggtga gggcagttac cagtgccaat atccaggagt ttgctggctg caagaagatc 1260
   tttgggagcc tggcatttct gccggagagc tttgatgggg acccagcctc caacactgcc 1320
   ccgctccagc cagagcagct ccaagtgttt gagactctgg aagagatcac aggttaccta 1380
   tacatctcag catggccgga cagcctgcct gacctcagcg tcttccagaa cctgcaagta 1440
   atccggggac gaattctgca caatggcgcc tactcgctga ccctgcaagg gctgggcatc 1500
   agctggctgg ggctgcgctc actgagggaa ctgggcagtg gactggccct catccaccat 1560
   aacacccacc tctgcttcgt gcacacggtg ccctgggacc agctctttcg gaacccgcac 1620
   caagctctgc tccacactgc caaccggcca gaggacgagt gtgtgggcga gggcctggcc 1680
   tgccaccagc tgtgcgcccg agggcactgc tggggtccag ggcccaccca gtgtgtcaac 1740
   tgcagccagt tccttcgggg ccaggagtgc gtggaggaat gccgagtact gcaggggctc 1800
   cccagggagt atgtgaatgc caggcactgt ttgccgtgcc accctgagtg tcagccccag 1860
   aatggctcag tgacctgttt tggaccggag gctgaccagt gtgtggcctg tgcccactat 1920
   aaggaccctc ccttctgcgt ggcccgctgc cccagcggtg tgaaacctga cctctcctac 1980
   atgcccatct ggaagtttcc agatgaggag ggcgcatgcc agccttgccc catcaactgc 2040
   acccactcct gtgtggacct ggatgacaag ggctgccccg ccgagcagag agccagccct 2100
   ctgacgtcca tcgtctctgc ggtggttggc attctgctgg tcgtggtctt gggggtggtc 2160
   tttgggatcc tcatcaagcg acggcagcag aagatccgga agtacacgat gcggagactg 2220
   ctgcaggaaa cggagctggt ggagccgctg acacctagcg gagcgatgcc caaccaggcg 2280
   cagatgcgga tcctgaaaga gacggagctg aggaaggtga aggtgcttgg atctggcgct 2340
   tttggcacag tctacaaggg catctggatc cctgatgggg agaatgtgaa aattccagtg 2400
   gccatcaaag tgttgaggga aaacacatcc cccaaagcca acaaagaaat cttagacgaa 2460
   gcatacgtga tggctggtgt gggctcccca tatgtctccc gccttctggg catctgcctg 2520
   acatccacgg tgcagctggt gacacagctt atgccctatg gctgcctctt agaccatgtc 2580
   cgggaaaacc gcggacgcct gggctcccag gacctgctga actggtgtat gcagattgcc 2640
   aaggggatga gctacctgga ggatgtgcgg ctcgtacaca gggacttggc cgctcggaac 2700
   gtgctggtca agagtcccaa ccatgtcaaa attacagact tcgggctggc tcggctgctg 2760
   gacattgacg agacagagta ccatgcagat gggggcaagg tgcccatcaa gtggatggcg 2820
   ctggagtcca ttctccgccg gcggttcacc caccagagtg atgtgtggag ttatggtgtg 2880
   actgtgtggg agctgatgac ttttggggcc aaaccttacg atgggatccc agcccgggag 2940
   atccctgacc tgctggaaaa gggggagcgg ctgccccagc cccccatctg caccattgat 3000
   gtctacatga tcatggtcaa atgttggatg attgactctg aatgtcggcc aagattccgg 3060
   gagttggtgt ctgaattctc ccgcatggcc agggaccccc agcgctttgt ggtcatccag 3120
   aatgaggact tgggcccagc cagtcccttg gacagcacct tctaccgctc actgctggag 3180
   gacgatgaca tgggggacct ggtggatgct gaggagtatc tggtacccca gcagggcttc 3240
   ttctgtccag accctgcccc gggcgctggg ggcatggtcc accacaggca ccgcagctca 3300
   tctaccagga gtggcggtgg ggacctgaca ctagggctgg agccctctga agaggaggcc 3360
   cccaggtctc cactggcacc ctccgaaggg gctggctccg atgtatttga tggtgacctg 3420
   ggaatggggg cagccaaggg gctgcaaagc ctccccacac atgaccccag ccctctacag 3480
   cggtacagtg aggaccccac agtacccctg ccctctgaga ctgatggcta cgttgccccc 3540
   ctgacctgca gcccccagcc tgaatatgtg aaccagccag atgttcggcc ccagccccct 3600
   tcgccccgag agggccctct gcctgctgcc cgacctgctg gtgccactct ggaaagggcc 3660
   aagactctct ccccagggaa gaatggggtc gtcaaagacg tttttgcctt tgggggtgcc 3720
   gtggagaacc ccgagtactt gacaccccag ggaggagctg cccctcagcc ccaccctcct 3780
   cctgccttca gcccagcctt cgacaacctc tattactggg accaggaccc accagagcgg 3840
   ggggctccac ccagcacctt caaagggaca cctacggcag agaacccaga gtacctgggt 3900
   ctggacgtgc cagtgtgaac cagaaggcca agtccgcaga agccctgatg tgtcctcagg 3960
   gagcagggaa ggcctgactt ctgctggcat caagaggtgg gagggccctc cgaccacttc 4020
   caggggaacc tgccatgcca ggaacctgtc ctaaggaacc ttccttcctg cttgagttcc 4080
   cagatggctg gaaggggtcc agcctcgttg gaagaggaac agcactgggg agtctttgtg 4140
   gattctgagg ccctgcccaa tgagactcta gggtccagtg gatgccacag cccagcttgg 4200
   ccctttcctt ccagatcctg ggtactgaaa gccttaggga agctggcctg agaggggaag 4260
   cggccctaag ggagtgtcta agaacaaaag cgacccattc agagactgtc cctgaaacct 4320
   agtactgccc cccatgagga aggaacagca atggtgtcag tatccaggct ttgtacagag 4380
   tgcttttctg tttagttttt actttttttg ttttgttttt ttaaagacga aataaagacc 4440
   caggggagaa tgggtgttgt atggggaggc aagtgtgggg ggtccttctc cacacccact 4500
   ttgtccattt gcaaatatat tttggaaaac 4530
<210> 30
   <211> 1464
   <212> DNA
   <213> Homo sapiens
<400> 30
   agccccaagc ttaccacctg cacccggaga gctgtgtcac catgtgggtc ccggttgtct 60
   tcctcaccct gtccgtgacg tggattggtg ctgcacccct catcctgtct cggattgtgg 120
   gaggctggga gtgcgagaag cattcccaac cctggcaggt gcttgtggcc tctcgtggca 180
   gggcagtctg cggcggtgtt ctggtgcacc cccagtgggt cctcacagct gcccactgca 240
   tcaggaacaa aagcgtgatc ttgctgggtc ggcacagcct gtttcatcct gaagacacag 300
   gccaggtatt tcaggtcagc cacagcttcc cacacccgct ctacgatatg agcctcctga 360
   agaatcgatt cctcaggcca ggtgatgact ccagccacga cctcatgctg ctccgcctgt 420
   cagagcctgc cgagctcacg gatgctgtga aggtcatgga cctgcccacc caggagccag 480
   cactggggac cacctgctac gcctcaggct ggggcagcat tgaaccagag gagttcttga 540
   ccccaaagaa acttcagtgt gtggacctcc atgttatttc caatgacgtg tgtgcgcaag 600
   ttcaccctca gaaggtgacc aagttcatgc tgtgtgctgg acgctggaca gggggcaaaa 660
   gcacctgctc gggtgattct gggggcccac ttgtctgtaa tggtgtgctt caaggtatca 720
   cgtcatgggg cagtgaacca tgtgccctgc ccgaaaggcc ttccctgtac accaaggtgg 780
   tgcattaccg gaagtggatc aaggacacca tcgtggccaa cccctgagca cccctatcaa 840
   ccccctattg tagtaaactt ggaaccttgg aaatgaccag gccaagactc aagcctcccc 900
   agttctactg acctttgtcc ttaggtgtga ggtccagggt tgctaggaaa agaaatcagc 960
   agacacaggt gtagaccaga gtgtttctta aatggtgtaa ttttgtcctc tctgtgtcct 1020
   ggggaatact ggccatgcct ggagacatat cactcaattt ctctgaggac acagatagga 1080
   tggggtgtct gtgttatttg tggggtacag agatgaaaga ggggtgggat ccacactgag 1140
   agagtggaga gtgacatgtg ctggacactg tccatgaagc actgagcaga agctggaggc 1200
   acaacgcacc agacactcac agcaaggatg gagctgaaaa cataacccac tctgtcctgg 1260
   aggcactggg aagcctagag aaggctgtga gccaaggagg gagggtcttc ctttggcatg 1320
   ggatggggat gaagtaagga gagggactgg accccctgga agctgattca ctatgggggg 1380
   aggtgtattg aagtcctcca gacaaccctc agatttgatg atttcctagt agaactcaca 1440
   gaaataaaga gctgttatac tgtg 1464
<210> 31
   <211> 781
   <212> DNA
   <213> Homo sapiens
<400> 31
   gtcacacccg gaagcagggg cccgagcgga gccggccgcg atgagcgggg agccggggca 60
   gacgtccgta gcgccccctc ccgaggaggt cgagccgggc agtggggtcc gcatcgtggt 120
   ggagtactgt gaaccctgcg gcttcgaggc gacctacctg gagctggcca gtgctgtgaa 180
   ggagcagtat ccgggcatcg agatcgagtc gcgcctcggg ggcacaggtg cctttgagat 240
   agagataaat ggacagctgg tgttctccaa gctggagaat gggggctttc cctatgagaa 300
   agatctcatt gaggccatcc gaagagccag taatggagaa accctagaaa agatcaccaa 360
   cagccgtcct ccctgcgtca tcctgtgact gcacaggact ctgggttcct gctctgttct 420
   ggggtccaaa ccttggtctc cctttggtcc tgctgggagc tccccctgcc tctttcccct 480
   acttagctcc ttagcaaaga gaccctggcc tccactttgc cctttgggta caaagaagga 540
   atagaagatt ccgtggcctt gggggcagga gagagacact ctccatgaac acttctccag 600
   ccacctcata cccccttccc agggtaagtg cccacgaaag cccagtccac tcttcgcctc 660
   ggtaatacct gtctgatgcc acagatttta tttattctcc cctaacccag ggcaatgtca 720
   gctattggca gtaaagtggc gctacaaaca ctaaaaaaaa aaaaaaaaaa aaaaaaaaaa 780
   a                                                                 781
<210> 32
   <211> 1114
   <212> DNA
   <213> Homo sapiens
<400> 32
   acacgccgat ttgccctttt gattcttcca caatcagggt gagactgctc ccagtgccat 60
   gaacggagac gacgcctttg caaggagacc cagggatgat gctcaaatat cagagaagtt 120
   acgaaaggcc ttcgatgata ttgccaaata cttctctaag aaagagtggg aaaagatgaa 180
   atcctcggag aaaatcgtct atgtgtatat gaagctaaac tatgaggtca tgactaaact 240
   aggtttcaag gtcaccctcc cacctttcat gcgtagtaaa cgggctgcag acttccacgg 300
   gaatgatttt ggtaacgatc gaaaccacag gaatcaggtt gaacgtcctc agatgacttt 360
   cggcagcctc cagagaatct tcccgaagga cccaaaaggg ggaaacatgc ctggacccac 420
   agactgcgtg agagaaagca gctggtggtt tatgaagaga tcagcgaccc tgaggaagat 480
   gacgagtaac tcccctcggg gatatgacac atgcccatga tgagaagcag aacgtggtga 540
   cctttcacga acatgggcat ggctgcggac ccctcgtcat caggtgcata gcaagtgaaa 600
   gcaagtgttc acaacagtga aaagttgagc gtcatttttc ttagtgtgcc aagagttcga 660
   tgttggcgtt tccgctgtat tttcttgcag tgtgccattc tgttagacat tagcgttttc 720
   gttgatgagc aagacatgct taatgcatat ttcggcttgt gtatccatgc acctacctca 780
   gaaaacaagt attgtcaggt attctctcca tagaacagca ctaccctcct ctctccccag 840
   atgtgactac tgaggggagg tctgagtgtt taatttccga ttttttcctc tgcatttaca 900
   cacacaccac acacgcacac acacacacca agtaccagta taagcatctc ccatctgctt 960
   ttctccattg ccatgcgacc tggtcaagcc cccctcactc tgtttcctgt tcagcatgta 1020
   ctcccctcat ccgattccgt tgtatcagtc actgacagtt aataaacctt tgcaaacgtt 1080
   caaaaaaaaa aaaaaaaaaa aaaaaaaaaa aaaa                             1114
<210> 33
   <211> 2143
   <212> DNA
   <213> Homo sapiens
<400> 33
   agtgcctttg gttgctggag ggaagaacac aatggatctg gtgctaaaaa gatgccttct 60
   tcatttggct gtgataggtg ctttgctggc tgtgggggct acaaaagtac ccagaaacca 120
   ggactggctt ggtgtctcaa ggcaactcag aaccaaagcc tggaacaggc agctgtatcc 180
   agagtggaca gaagcccaga gacttgactg ctggagaggt ggtcaagtgt ccctcaaggt 240
   cagtaatgat gggcctacac tgattggtgc aaatgcctcc ttctctattg ccttgaactt 300
   ccctggaagc caaaaggtat tgccagatgg gcaggttatc tgggtcaaca ataccatcat 360
   caatgggagc caggtgtggg gaggacagcc agtgtatccc caggaaactg acgatgcctg 420
   catcttccct gatggtggac cttgcccatc tggctcttgg tctcagaaga gaagctttgt 480
   ttatgtctgg aagacctggg gccaatactg gcaagttcta gggggcccag tgtctgggct 540
   gagcattggg acaggcaggg caatgctggg cacacacacc atggaagtga ctgtctacca 600
   tcgccgggga tcccggagct atgtgcctct tgctcattcc agctcagcct tcaccattac 660
   tgaccaggtg cctttctccg tgagcgtgtc ccagttgcgg gccttggatg gagggaacaa 720
   gcacttcctg agaaatcagc ctctgacctt tgccctccag ctccatgacc ccagtggcta 780
   tctggctgaa gctgacctct cctacacctg ggactttgga gacagtagtg gaaccctgat 840
   ctctcgggca cttgtggtca ctcatactta cctggagcct ggcccagtca ctgcccaggt 900
   ggtcctgcag gctgccattc ctctcacctc ctgtggctcc tccccagttc caggcaccac 960
   agatgggcac aggccaactg cagaggcccc taacaccaca gctggccaag tgcctactac 1020
   agaagttgtg ggtactacac ctggtcaggc gccaactgca gagccctctg gaaccacatc 1080
   tgtgcaggtg ccaaccactg aagtcataag cactgcacct gtgcagatgc caactgcaga 1140
   gagcacaggt atgacacctg agaaggtgcc agtttcagag gtcatgggta ccacactggc 1200
   agagatgtca actccagagg ctacaggtat gacacctgca gaggtatcaa ttgtggtgct 1260
   ttctggaacc acagctgcac aggtaacaac tacagagtgg gtggagacca cagctagaga 1320
   gctacctatc cctgagcctg aaggtccaga tgccagctca atcatgtcta cggaaagtat 1380
   tacaggttcc ctgggccccc tgctggatgg tacagccacc ttaaggctgg tgaagagaca 1440
   agtccccctg gattgtgttc tgtatcgata tggttccttt tccgtcaccc tggacattgt 1500
   ccagggtatt gaaagtgccg agatcctgca ggctgtgccg tccggtgagg gggatgcatt 1560
   tgagctgact gtgtcctgcc aaggcgggct gcccaaggaa gcctgcatgg agatctcatc 1620
   gccagggtgc cagccccctg cccagcggct gtgccagcct gtgctaccca gcccagcctg 1680
   ccagctggtt ctgcaccaga tactgaaggg tggctcgggg acatactgcc tcaatgtgtc 1740
   tctggctgat accaacagcc tggcagtggt cagcacccag cttatcatgc ctggtcaaga 1800
   agcaggcctt gggcaggttc cgctgatcgt gggcatcttg ctggtgttga tggctgtggt 1860
   ccttgcatct ctgatatata ggcgcagact tatgaagcaa gacttctccg taccccagtt 1920
   gccacatagc agcagtcact ggctgcgtct accccgcatc ttctgctctt gtcccattgg 1980
   tgagaatagc cccctcctca gtgggcagca ggtctgagta ctctcatatg atgctgtgat 2040
   tttcctggag ttgacagaaa cacctatatt tcccccagtc ttccctggga gactactatt 2100
   aactgaaata aatactcaga gcctgaaaaa aaaaaaaaaa aaa 2143
<210> 34
   <211> 2352
   <212> DNA
   <213> Homo sapiens
<400> 34
<210> 35
   <211> 503
   <212> DNA
   <213> Homo sapiens
<400> 35
   gacagcggct tccttgatcc ttgccacccg cgactgaaca ccgacagcag cagcctcacc 60
   atgaagttgc tgatggtcct catgctggcg gccctctccc agcactgcta cgcaggctct 120
   ggctgcccct tattggagaa tgtgatttcc aagacaatca atccacaagt gtctaagact 180
   gaatacaaag aacttcttca agagttcata gacgacaatg ccactacaaa tgccatagat 240
   gaattgaagg aatgttttct taaccaaacg gatgaaactc tgagcaatgt tgaggtgttt 300
   atgcaattaa tatatgacag cagtctttgt gatttatttt aactttctgc aagacctttg 360
   gctcacagaa ctgcagggta tggtgagaaa ccaactacgg attgctgcaa accacacctt 420
   ctctttctta tgtcttttta ctacaaacta caagacaatt gttgaaacct gctatacatg 480
   tttattttaa taaattgatg gca 503
<210> 36
   <211> 591
   <212> DNA
   <213> Homo sapiens
<400> 36
   cttctctggg acacattgcc ttctgttttc tccagcatgc gcttgctcca gctcctgttc 60
   agggccagcc ctgccaccct gctcctggtt ctctgcctgc agttgggggc caacaaagct 120
   caggacaaca ctcggaagat cataataaag aattttgaca ttcccaagtc agtacgtcca 180
   aatgacgaag tcactgcagt gcttgcagtt caaacagaat tgaaagaatg catggtggtt 240
   aaaacttacc tcattagcag catccctcta caaggtgcat ttaactataa gtatactgcc 300
   tgcctatgtg acgacaatcc aaaaaccttc tactgggact tttacaccaa cagaactgtg 360
   caaattgcag ccgtcgttga tgttattcgg gaattaggca tctgccctga tgatgctgct 420
   gtaatcccca tcaaaaacaa ccggttttat actattgaaa tcctaaaggt agaataatgg 480
   aagccctgtc tgtttgccac acccaggtga tttcctctaa agaaacttgg ctggaatttc 540
   tgctgtggtc tataaaataa acttcttaac atgcttaaaa aaaaaaaaaa a 591
<210> 37
   <211> 2388
   <212> DNA
   <213> Homo sapiens
<400> 37
   ggaaaccgag gcagaggagg ctcaggtgtg gccaatcacc ctgcacatca gagttaccct 60
   gggcagggcc cactgagacc tgggaggggc cactcgggac ctggagggct gggggctgcc 120
   cgggcgttag gggtaaagct ccctacccaa ctgcgcagaa ggcctcagag gcctgggggc 180
   tgggcttccc ctttcacatc gccctttaga ggcccacgtg tgggcattgg cccgcgatct 240
   gaaaggggct gtcctgttcc tcatgggcgc tgccagcgcc acgcactcct ctttctgcct 300
   ggccggccac tcccgtctgc tgtgacgcgc ggacagagag ctaccggtgg acccacggtg 360
   cctccctccc tgggatctac acagaccatg gccttgccaa cggctcgacc cctgttgggg 420
   tcctgtggga cccccgccct cggcagcctc ctgttcctgc tcttcagcct cggatgggtg 480
   cagccctcga ggaccctggc tggagagaca gggcaggagg ctgcgcccct ggacggagtc 540
   ctggccaacc cacctaacat ttccagcctc tcccctcgcc aactccttgg cttcccgtgt 600
   gcggaggtgt ccggcctgag cacggagcgt gtccgggagc tggctgtggc cttggcacag 660
   aagaatgtca agctctcaac agagcagctg cgctgtctgg ctcaccggct ctctgagccc 720
   cccgaggacc tggacgccct cccattggac ctgctgctat tcctcaaccc agatgcgttc 780
   tcggggcccc aggcctgcac ccgtttcttc tcccgcatca cgaaggccaa tgtggacctg 840
   ctcccgaggg gggctcccga gcgacagcgg ctgctgcctg cggctctggc ctgctggggt 900
   gtgcgggggt ctctgctgag cgaggctgat gtgcgggctc tgggaggcct ggcttgcgac 960
   ctgcctgggc gctttgtggc cgagtcggcc gaagtgctgc taccccggct ggtgagctgc 1020
   ccgggacccc tggaccagga ccagcaggag gcagccaggg cggctctgca gggcggggga 1080
   cccccctacg gccccccgtc gacatggtct gtctccacga tggacgctct gcggggcctg 1140
   ctgcccgtgc tgggccagcc catcatccgc agcatcccgc agggcatcgt ggccgcgtgg 1200
   cggcaacgct cctctcggga cccatcctgg cggcagcctg aacggaccat cctccggccg 1260
   cggttccggc gggaagtgga gaagacagcc tgtccttcag gcaagaaggc ccgcgagata 1320
   gacgagagcc tcatcttcta caagaagtgg gagctggaag cctgcgtgga tgcggccctg 1380
   ctggccaccc agatggaccg cgtgaacgcc atccccttca cctacgagca gctggacgtc 1440
   ctaaagcata aactggatga gctctaccca caaggttacc ccgagtctgt gatccagcac 1500
   ctgggctacc tcttcctcaa gatgagccct gaggacattc gcaagtggaa tgtgacgtcc 1560
   ctggagaccc tgaaggcttt gcttgaagtc aacaaagggc acgaaatgag tcctcaggtg 1620
   gccaccctga tcgaccgctt tgtgaaggga aggggccagc tagacaaaga caccctagac 1680
   accctgaccg ccttctaccc tgggtacctg tgctccctca gccccgagga gctgagctcc 1740
   gtgcccccca gcagcatctg ggcggtcagg ccccaggacc tggacacgtg tgacccaagg 1800
   cagctggacg tcctctatcc caaggcccgc cttgctttcc agaacatgaa cgggtccgaa 1860
   tacttcgtga agatccagtc cttcctgggt ggggccccca cggaggattt gaaggcgctc 1920
   agtcagcaga atgtgagcat ggacttggcc acgttcatga agctgcggac ggatgcggtg 1980
   ctgccgttga ctgtggctga ggtgcagaaa cttctgggac cccacgtgga gggcctgaag 2040
   gcggaggagc ggcaccgccc ggtgcgggac tggatcctac ggcagcggca ggacgacctg 2100
   gacacgctgg ggctggggct acagggcggc atccccaacg gctacctggt cctagacctc 2160
   agcatgcaag aggccctctc ggggacgccc tgcctcctag gacctggacc tgttctcacc 2220
   gtcctggcac tgctcctagc ctccaccctg gcctgagggc cccactccct tgctggcccc 2280
   agccctgctg gggatccccg cctggccagg agcaggcacg ggtggtcccc gttccacccc 2340
   aagagaactc gcgctcagta aacgggaaca tgccccctgc agacacgt 2388
<210> 38
   <211> 2412
   <212> DNA
   <213> Homo sapiens
<400> 38
   ggaaaccgag gcagaggagg ctcaggtgtg gccaatcacc ctgcacatca gagttaccct 60
   gggcagggcc cactgagacc tgggaggggc cactcgggac ctggagggct gggggctgcc 120
   cgggcgttag gggtaaagct ccctacccaa ctgcgcagaa ggcctcagag gcctgggggc 180
   tgggcttccc ctttcacatc gccctttaga ggcccacgtg tgggcattgg cccgcgatct 240
   gaaaggggct gtcctgttcc tcatgggcgc tgccagcgcc acgcactcct ctttctgcct 300
   ggccggccac tcccgtctgc tgtgacgcgc ggacagagag ctaccggtgg acccacggtg 360
   cctccctccc tgggatctac acagaccatg gccttgccaa cggctcgacc cctgttgggg 420
   tcctgtggga cccccgccct cggcagcctc ctgttcctgc tcttcagcct cggatgggtg 480
   cagccctcga ggaccctggc tggagagaca gggcaggagg ctgcgcccct ggacggagtc 540
   ctggccaacc cacctaacat ttccagcctc tcccctcgcc aactccttgg cttcccgtgt 600
   gcggaggtgt ccggcctgag cacggagcgt gtccgggagc tggctgtggc cttggcacag 660
   aagaatgtca agctctcaac agagcagctg cgctgtctgg ctcaccggct ctctgagccc 720
   cccgaggacc tggacgccct cccattggac ctgctgctat tcctcaaccc agatgcgttc 780
   tcggggcccc aggcctgcac ccgtttcttc tcccgcatca cgaaggccaa tgtggacctg 840
   ctcccgaggg gggctcccga gcgacagcgg ctgctgcctg cggctctggc ctgctggggt 900
   gtgcgggggt ctctgctgag cgaggctgat gtgcgggctc tgggaggcct ggcttgcgac 960
   ctgcctgggc gctttgtggc cgagtcggcc gaagtgctgc taccccggct ggtgagctgc 1020
   ccgggacccc tggaccagga ccagcaggag gcagccaggg cggctctgca gggcggggga 1080
   cccccctacg gccccccgtc gacatggtct gtctccacga tggacgctct gcggggcctg 1140
   ctgcccgtgc tgggccagcc catcatccgc agcatcccgc agggcatcgt ggccgcgtgg 1200
   cggcaacgct cctctcggga cccatcctgg cggcagcctg aacggaccat cctccggccg 1260
   cggttccggc gggaagtgga gaagacagcc tgtccttcag gcaagaaggc ccgcgagata 1320
   gacgagagcc tcatcttcta caagaagtgg gagctggaag cctgcgtgga tgcggccctg 1380
   ctggccaccc agatggaccg cgtgaacgcc atccccttca cctacgagca gctggacgtc 1440
   ctaaagcata aactggatga gctctaccca caaggttacc ccgagtctgt gatccagcac 1500
   ctgggctacc tcttcctcaa gatgagccct gaggacattc gcaagtggaa tgtgacgtcc 1560
   ctggagaccc tgaaggcttt gcttgaagtc aacaaagggc acgaaatgag tcctcaggct 1620
   cctcggcggc ccctcccaca ggtggccacc ctgatcgacc gctttgtgaa gggaaggggc 1680
   cagctagaca aagacaccct agacaccctg accgccttct accctgggta cctgtgctcc 1740
   ctcagccccg aggagctgag ctccgtgccc cccagcagca tctgggcggt caggccccag 1800
   gacctggaca cgtgtgaccc aaggcagctg gacgtcctct atcccaaggc ccgccttgct 1860
   ttccagaaca tgaacgggtc cgaatacttc gtgaagatcc agtccttcct gggtggggcc 1920
   cccacggagg atttgaaggc gctcagtcag cagaatgtga gcatggactt ggccacgttc 1980
   atgaagctgc ggacggatgc ggtgctgccg ttgactgtgg ctgaggtgca gaaacttctg 2040
   ggaccccacg tggagggcct gaaggcggag gagcggcacc gcccggtgcg ggactggatc 2100
   ctacggcagc ggcaggacga cctggacacg ctggggctgg ggctacaggg cggcatcccc 2160
   aacggctacc tggtcctaga cctcagcatg caagaggccc tctcggggac gccctgcctc 2220
   ctaggacctg gacctgttct caccgtcctg gcactgctcc tagcctccac cctggcctga 2280
   gggccccact cccttgctgg ccccagccct gctggggatc cccgcctggc caggagcagg 2340
   cacgggtggt ccccgttcca ccccaagaga actcgcgctc agtaaacggg aacatgcccc 2400
   ctgcagacac gt                                                     2412
<210> 39
   <211> 946
   <212> DNA
   <213> Homo sapiens
<400> 39
   tgacagccca ccagtgacca tgaaggctgt gctgcttgcc ctgttgatgg caggcttggc 60
   cctgcagcca ggcactgccc tgctgtgcta ctcctgcaaa gcccaggtga gcaacgagga 120
   ctgcctgcag gtggagaact gcacccagct gggggagcag tgctggaccg cgcgcatccg 180
   cgcagttggc ctcctgaccg tcatcagcaa aggctgcagc ttgaactgcg tggatgactc 240
   acaggactac tacgtgggca agaagaacat cacgtgctgt gacaccgact tgtgcaacgc 300
   cagcggggcc catgccctgc agccggctgc cgccatcctt gcgctgctcc ctgcactcgg 360
   cctgctgctc tggggacccg gccagctata ggctctgggg ggccccgctg cagcccacac 420
   tgggtgtggt gccccaggcc tctgtgccac tcctcacaga cctggcccag tgggagcctg 480
   tcctggttcc tgaggcacat cctaacgcaa gtctgaccat gtatgtttgc acccctgtcc 540
   cccaccctga ccctcccatg gccctctcca ggactcccac ccggcagatc agctctagtg 600
   acacagatcc gcctgcagat ggcccctcca accctctctg ctgctgtttc catggcccag 660
   cattctccac ccttaaccct gtgctcaggc acctcttccc ccaggaagcc ttccctgccc 720
   accccatcta tgacttgagc caggtctggt ccgtggtgtc ccccgcaccc agcaggggac 780
   aggcactcag gagggcccag taaaggctga gatgaagtgg actgagtaga actggaggac 840
   aagagtcgac gtgagttcct gggagtctcc agagatgggg cctggaggcc tggaggaagg 900
   ggccaggcct cacattcgtg gggctccctg aatggcagcc tgagca                946
<210> 40
   <211> 3503
   <212> DNA
   <213> Homo sapiens
<400> 40
   ttatttagcc gttgaactgc cagccgcccg gggcccacgc gctccgggcc gctcaggctg 60
   agcgatttcc cgccttttct gaggttctga ggcgggagcc attggttctt tctgttgccc 120
   tcatagaccg tatgtagcag ttcgcgtggg cacagaaccc acggtttccc gctagttctt 180
   caaagatatt tacaaccgta acagagaaaa tggaaaagca aaagcccttt gcattgttcg 240
   taccaccgag atcaagcagc agtcaggtgt ctgcggtgaa acctcagacc ctgggaggcg 300
   attccacttt cttcaagagt ttcaacaaat gtactgaaga tgattttgag tttccatttg 360
   caaagactaa tctctccaaa aatggggaaa acattgattc agatcctgct ttacaaaaag 420
   ttaatttctt gcccgtgctt gagcaggttg gtaattctga ctgtcactat caggaaggac 480
   taaaagactc tgatttggag aattcagagg gattgagcag agtgtattca aaactgtata 540
   aggaggctga aaagataaaa aaatggaaag taagtacaga agctgaactg agacagaaag 600
   aaagtaagtt gcaagaaaac agaaagataa ttgaagcaca gcgaaaagcc attcaggaac 660
   tgcaatttgg aaatgaaaaa gtaagtttga aattagaaga aggaatacaa gaaaataaag 720
   atttaataaa agagaataat gccacaaggc atttatgtaa tctactcaaa gaaacctgtg 780
   ctagatctgc agaaaagaca aagaaatatg aatatgaacg ggaagaaacc aggcaagttt 840
   atatggatct aaataataac attgagaaaa tgataacagc ttttgaggaa cttcgtgtgc 900
   aagctgagaa ttccagactg gaaatgcatt ttaagttaaa ggaagattat gaaaaaatcc 960
   aacaccttga acaagaatac aagaaggaaa taaatgacaa ggaaaagcag gtatcactac 1020
   tattgatcca aatcactgag aaagaaaata aaatgaaaga tttaacattt ctgctagagg 1080
   aatccagaga taaagttaat caattagagg aaaagacaaa attacagagt gaaaacttaa 1140
   aacaatcaat tgagaaacag catcatttga ctaaagaact agaagatatt aaagtgtcat 1200
   tacaaagaag tgtgagtact caaaaggctt tagaggaaga tttacagata gcaacaaaaa 1260
   caatttgtca gctaactgaa gaaaaagaaa ctcaaatgga agaatctaat aaagctagag 1320
   ctgctcattc gtttgtggtt actgaatttg aaactactgt ctgcagcttg gaagaattat 1380
   tgagaacaga acagcaaaga ttggaaaaaa atgaagatca attgaaaata cttaccatgg 1440
   agcttcaaaa gaaatcaagt gagctggaag agatgactaa gcttacaaat aacaaagaag 1500
   tagaacttga agaattgaaa aaagtcttgg gagaaaagga aacactttta tatgaaaata 1560
   aacaatttga gaagattgct gaagaattaa aaggaacaga acaagaacta attggtcttc 1620
   tccaagccag agagaaagaa gtacatgatt tggaaataca gttaactgcc attaccacaa 1680
   gtgaacagta ttattcaaaa gaggttaaag atctaaaaac tgagcttgaa aacgagaagc 1740
   ttaagaatac tgaattaact tcacactgca acaagctttc actagaaaac aaagagctca 1800
   cacaggaaac aagtgatatg accctagaac tcaagaatca gcaagaagat attaataata 1860
   acaaaaagca agaagaaagg atgttgaaac aaatagaaaa tcttcaagaa acagaaaccc 1920
   aattaagaaa tgaactagaa tatgtgagag aagagctaaa acagaaaaga gatgaagtta 1980
   aatgtaaatt ggacaagagt gaagaaaatt gtaacaattt aaggaaacaa gttgaaaata 2040
   aaaacaagta tattgaagaa cttcagcagg agaataaggc cttgaaaaaa aaaggtacag 2100
   cagaaagcaa gcaactgaat gtttatgaga taaaggtcaa taaattagag ttagaactag 2160
   aaagtgccaa acagaaattt ggagaaatca cagacaccta tcagaaagaa attgaggaca 2220
   aaaagatatc agaagaaaat cttttggaag aggttgagaa agcaaaagta atagctgatg 2280
   aagcagtaaa attacagaaa gaaattgata agcgatgtca acataaaata gctgaaatgg 2340
   tagcacttat ggaaaaacat aagcaccaat atgataagat cattgaagaa agagactcag 2400
   aattaggact ttataagagc aaagaacaag aacagtcatc actgagagca tctttggaga 2460
   ttgaactatc caatctcaaa gctgaacttt tgtctgttaa gaagcaactt gaaatagaaa 2520
   gagaagagaa ggaaaaactc aaaagagagg caaaagaaaa cacagctact cttaaagaaa 2580
   aaaaagacaa gaaaacacaa acatttttat tggaaacacc tgaaatttat tggaaattgg 2640
   attctaaagc agttccttca caaactgtat ctcgaaattt cacatcagtt gatcatggca 2700
   tatccaaaga taaaagagac tatctgtgga catctgccaa aaatacttta tctacaccat 2760
   tgccaaaggc atatacagtg aagacaccaa caaaaccaaa actacagcaa agagaaaact 2820
   tgaatatacc cattgaagaa agtaaaaaaa agagaaaaat ggcctttgaa tttgatatta 2880
   attcagatag ttcagaaact actgatcttt tgagcatggt ttcagaagaa gagacattga 2940
   aaacactgta taggaacaat aatccaccag cttctcatct ttgtgtcaaa acaccaaaaa 3000
   aggccccttc atctctaaca acccctggat ctacactgaa gtttggagct ataagaaaaa 3060
   tgcgggagga ccgttgggct gtaattgcta aaatggatag aaaaaaaaaa ctaaaagaag 3120
   ctgaaaagtt atttgtttaa tttcagagaa tcagtgtagt taaggagcct aataacgtga 3180
   aacttatagt taatattttg ttcttatttg ccagagccaa attttatctg gaagttgaga 3240
   cttaaaaaat acttgcatga atgatttgtg tttctttata tttttagcct aaatgttaac 3300
   tacatattgt ctggaaacct gtcattgtat tcagataatt agatgattat atattgttgt 3360
   tactttttct tgtattcatg aaaactgttt ttactaagtt ttcaaatttg taaagttagc 3420
   ctttgaatgc taagaatgca ttattgaggg tcattcttta ttctttacta ttaaaatatt 3480
   ttggatgcaa aaaaaaaaaa aaa                                          3503

## Description

### Background of the Invention

### Field of the Invention

Disclosed herein are methods for matching a cancer condition with an appropriate immunotherapeutic agent for use in an immunotherapeutic regimen. Also disclosed are methods for confirming diagnosis of a particular type of cancer.

### Description of the Related Art

The American Cancer Society has estimated that over one million people get cancer each year, and that approximately one out of every two American men and one out of every three American women will have some type of cancer at some point during their lifetime.

Cancer generally develops when cells in a part of the body begin to grow out of control. Although there are many kinds of cancer, they usually start because of out-of-control growth of abnormal cells.

Normal body cells grow, divide, and die in an orderly fashion. Cancer cells are different in that they continue to grow and divide. Instead of dying, they outlive normal cells and continue to form new abnormal cells.

Usual treatment options for cancer include surgery, radiation therapy, and chemotherapy. A fourth branch of treatment is developing, which is referred to as immunotherapy. Immunotherapies attempt to help the immune system recognize cancer cells, and/or to strengthen a response against cancer cells in order to destroy the cancer. Immunotherapies include active and passive immunotherapies. Active immunotherapies attempt to stimulate the body's own immune system to fight the disease. Passive immunotherapies generally do not rely on the body to attack the disease; instead, they use immune system components (such as antibodies) created outside of the body.

Despite the various types of treatments, a continuing need exists for additional treatment options that are more closely matched to a patient's cancer condition or type. In addition, there is a need for more accurate diagnostic tools for cancer.

### Summary of the Invention

Embodiments of the invention disclosed herein are directed to the use of a preselected panel of tumor-associated antigens (TuAAs) to match a patient's cancer condition or type with an appropriate immunotherapeutic agent for use in an immunotherapeutic regimen. In preferred embodiments, the TuAAs are antigens expressed by the cancer cell itself. In alternate embodiments, the TuAAs are antigens associated with non-cancerous components of the tumor, such as tumor-associated neovasculature or other stroma. Methods to determine, diagnose, or confirm a diagnosis of a type of cancer using a preselected panel of antigens is also disclosed. Methods for predicting disease progression in a cancer patient are also disclosed.

The disclosure also relates to methods for matching a patient's cancer condition or type with an immunotherapeutic agent including the steps of assaying the patient's tumor tissue for expression of a preselected panel of antigens and based on the assay results, selecting an immunotherapeutic agent targeting one, or two, or three or more of the antigens expressed by the patient's tumor tissue. The method can further include the step of developing an antigen profile for the tumor and selecting the immunotherapeutic agent based on the profile. In some embodiments the selected agent is an active immunotherapeutic. In some embodiments, the agent comprises an immunogen that includes or encodes at least a portion of at least one of the expressed antigens. In other embodiments the selected agent is a passive immunotherapeutic. In some embodiments the agent comprises a monoclonal antibody.

The disclosure also relates to methods for preparing a cancer immunotherapeutic composition wherein an immunotherapeutic is selected on the basis of the expression profile of tumor tissue for at least two tumor-associated antigens (TuAAs) in a preselected panel so that the immunotherapeutic agent comprises or encodes at least a segment of at least one of the expressed TuAAs and wherein the immunotherapeutic agent is optionally combined with pharmaceutically acceptable excipients.

The invention relates to a method of matching a cancer condition in a patient with one or more immunotherapeutic agents for use in an immunotherapeutic regimen, comprising the steps of classifying the patient's tumor tissue on the basis of assaying the tumor tissue for two or more expressed tumor-associated antigens (TuAAs) in a preselected panel, wherein the preselected panel comprises two or more antigens selected from the group consisting of an SSX protein, SSX-2, SSX-4, a MAGE protein, MAGE-1, MAGE-3, PRAME, NY-ESO-1, LAGE, PSMA, PSCA, SCP-1, melan-A/MART-1 and tyrosinase, to develop an antigen profile for the tumor; and selecting one or more immunotherapeutic agents for delineating an immunotherapeutic regimen based on the profile, the regimen comprising administration of one or more immunotherapeutic agents targeting two or more antigens in the profile, wherein the tumor tissue is classified by the TuAAs that it expresses and not by the tissue of origin. In some embodiments, the regimen comprises administering at least one immunotherapeutic agents targeting two, three, four, or more of the expressed antigens. The agents can be in forms such as, for example, nucleic acid, or polypeptide, or cellular, or humoral, or active, or passive, etc. For embodiments in which the regimen comprises administering two or more immunotherapeutic agents, the agents can be similar in form or different in form. Thus, in some embodiments, the regimen can include both an active immunotherapeutic agent and a passive immunotherapeutic agent.

The disclosure also relates to methods for matching a cancer condition in a patient with an immunotherapeutic agent are disclosed. The methods can include the steps of: determining the patient's class I MHC type; assaying the patient's tumor tissue for two or more expressed tumor-associated antigens (TuAAs) in a preselected panel; assaying the patient's tumor tissue for the expression of MHC class I or β2-microglobulin; selecting an immunotherapeutic agent for administration to the patient based on the assays, wherein the immunotherapeutic agent comprises or encodes an epitope restricted by the patient's class I MHC type, for each of two or more antigens expressed by the tumor. In some embodiments, antigen expression is detected on neoplastic cells, or tumor-associated stromal cells, or both. In some embodiments, the two or more antigens expressed by the tumor include an antigen expressed by a neoplastic cell and an antigen expressed by a tumor-associated stromal cell.

Methods of determining or confirming the occurrence of cancer comprising the step of determining the expression profile of tumor tissue for at least one polypeptide wherein the polypeptide is part of a preselected panel comprising at least two TuAAs and at least one lineage marker are also disclosed.

The preselected panel of TuAAs can include, for example, but not limited to, cancer testis antigens, tissue specific antigens, oncofetal antigens, differentiation antigens, growth factors, growth factor receptors, adhesion factors, signal transduction proteins, transcription factors, oncogene products, tumor suppressor gene products, microbial agents, and the like. In some embodiments, the preselected panel comprises two, or three, or more antigens selected from the group consisting of an SSX protein, SSX-2, SSX-4, a MAGE protein, MAGE-1, MAGE-3, PRAME, NY-ESO-1, LAGE, PSMA, PSCA, SCP-1, melan-A/MART-1 and tyrosinase. In some embodiments, the cancer is carcinoma. The carcinoma can be, for example, breast, colorectal, prostate, pancreatic, lung, ovarian, renal cell, or melanocyte.

The tumor tissue assayed can include primary tumor tissue or metastatic tumor tissue. Antigen expression can be detected on neoplastic cells, or tumor-associated stromal cells, or both. In some embodiments, the preselected panel includes an antigen expressed by a neoplastic cell and an antigen expressed by a tumor-associated stromal cell. The stromal cells can be neovasculature. The neovasculature associated antigen can be PSMA and the neoplastic cell antigen can be NY-ESO-1, SSX-2, LAGE, or PRAME.

Antigen expression can be detected, directly or indirectly. For example, the assay can detect the absence, presence and/or abundance of mRNA, polypeptide, mature protein, peptide, or MHC-peptide complex. In some embodiments, the assay detects the condition of the TuAAs, such as processing state, differential splicing, mutation from germline, variation from consensus sequence in human population, cellular localization, subcellular localization, co-expression with other markers, and the like. Examples of useful assays include RT-PCR, transcript determination, protein determination, epitope determination, or any combination thereof. In some embodiments, the assay comprises reverse transcription polymerase chain reaction (RT-PCR), real-time PCR, quantitative PCR, northern hybridization, autoradiography, chemiluminescent detection, autofluorography, flow cytometry, gene chip expression profiling, immunohistochemistry, western hybridization, radioimmunoassay, or in situ hybridization, individually or in any combination thereof. In some embodiments, at least two assaying steps are carried out at different time points during the course of disease and comparative information is obtained from the assaying steps. The obtained information can be used to implement, modify or withdraw a therapy.

In one embodiment, the tumor is melanoma and the preselected panel of antigens comprises at least two, or three, or four or more TuAAs selected from the group consisting of tyrosinase, melan-A/MART-1, NY-ESO-1, PRAME, an SSX protein, and a MAGE protein. The SSX protein can be SSX-2 or SSX-4. The MAGE protein can be MAGE-1 or MAGE-3.

In another embodiment, the tumor is breast cancer and the preselected panel of antigens comprises at least two, or three, or four or more TuAAs selected from the group consisting of NY-ESO-1, C35, Her2/Neu, an SSX protein, and a MAGE protein. The SSX protein can be SSX-2 or SSX-4. The MAGE protein can be MAGE-1 or MAGE-3.

In yet another embodiment, the tumor is colorectal cancer and the preselected panel of antigens comprises at least two, or three, or four or more TuAAs selected from the group consisting of CEA, an SSX protein, PRAME, NY-ESO-1, LAGE, PSCA, SCP-1, PSMA, and a MAGE protein. The SSX protein can be SSX-2 or SSX-4. The MAGE protein can be MAGE-1 or MAGE-3.

In yet a further embodiment, the tumor is ovarian cancer and the preselected panel of antigens comprises at least two, or three or four or more TuAAs selected from the group consisting of an SSX protein, PRAME, NY-ESO-1, PSMA, Her2/neu, C35, PSCA, SCP-1, CEA, LAGE, and a MAGE protein. The SSX protein can be SSX-2 or SSX-4. The MAGE protein can be MAGE-1 or MAGE-3. The ovarian cancer can be, for example, serous carcinoma, non-serous carcinoma, mucinous (cell) carcinoma, clear cell carcinoma, and the like.

In still another embodiment, the tumor is lung cancer and the preselected panel of antigens comprises at least two, or three, or four or more TuAAs selected from the group consisting of PSMA, NY-ESO-1, SSX-2, and a MAGE protein. The cancer can be, for example, non-small cell lung cancer. The MAGE protein can be MAGE-1 or MAGE-3.

In a further embodiment, the tumor is prostate cancer and the preselected panel of antigens comprises at least two, or three, or four or more TuAAs selected from the group consisting of NY-ESO-1, PSA, PSCA, PSMA, an SSX protein, and a MAGE protein The SSX protein can be SSX-2 or SSX-4. The MAGE protein can be MAGE-1 or MAGE-3.

In another embodiment, the tumor is pancreatic cancer and the panel of antigens comprises at least two, or three, or four or more TuAAs selected from the group consisting of PSMA, PRAME, NY-ESO, LAGE, PSCA, and a MAGE protein and an SSX protein. The SSX protein can be SSX-2 or SSX-4. The MAGE protein can be MAGE-1 or MAGE-3.

In still another embodiment, the tumor is renal cell carcinoma or renal cancer and the panel of antigens comprises at least two, or three, or four or more TuAAs selected from the group consisting of PSMA, PRAME, NY-ESO, LAGE, PSCA, SCP-1, a MAGE protein, and an SSX protein. The SSX protein can be SSX-2 or SSX-4. The MAGE protein can be MAGE-1 or MAGE-3.

The disclosure also relates to a method of marketing cancer immunotherapeutics comprising establishing a relationship with a cancer diagnostics laboratory, wherein the laboratory includes TuAA expression in it standard panel of tests, and wherein the TuAAs assayed for correspond to the immunogens of the immunotherapeutics to be marketed, and sending a report with each patient's test results identifying immunotherapeutics comprising immunogens that correspond to the TuAAs expressed by the patient's tumor. In some embodiments, the relationship comprises contract services. In some embodiments, the relationship is a partnership.

### Brief Description of the Drawings

Figure 1 is a timeline depicting the schedule of immunization with two plasmid (pCBP expressing SSX-2 41-49 and pSEM expressing Melan A).

Figure 2 is a bar graph that shows CTL activity obtained using the protocol in Figure 1.

Figure 3 is a timeline depicting the schedule of immunization of an entrain-and-amplify immunization protocol using plasmids and peptides representing two epitopes.

Figure 4 is a table showing *in vivo* clearance of epitope-pulsed cells in mice immunized according to the protocol of Figure 3.

Figures 5A and 5B are timelines depicting immunization protocols for inducing strong multivalent responses. Figure 5A shows the use of peptides for boosting restores multivalent immune responses even if plasmids and peptides are used as mixtures. Figure 5B shows segregation of plasmid and peptide components allows induction of multivalent immune responses.

### Detailed Description of the Preferred Embodiment

The frequency of expression of many tumor-associated antigens (TuAAs) in various types of cancers is known. However, the frequency of appearance of some antigens, and especially certain combinations of TuAAs in various types of cancers has not been reported. Accurate measurement of the presence of TuAAs in tumor tissues aids in determining which TuAAs will be useful for the treatment of a particular type of cancer.

Many attempts to develop immunotherapies for cancer have targeted a single antigen. This can be problematic for two distinct reasons. Firstly, the expression of any particular TuAA in cancer can be mosaic with the antigen expression ranging from high in some cells within a tumor mass to completely absent in others. Moreover, the TuAA may be expressed in some lesions but not others. By directing an immune response against more than a single antigen, if properly selected, the number of tumor cells that can be recognized is maximized. Secondly, some tumors lose expression of a TuAA following immunization, giving rise to a resistant population. If the immune response is directed against more than one TuAA it becomes much more difficult for a resistant tumor to arise because it must then simultaneously lose expression of each of the antigens in order to escape. Thus, in treating cancer with immunotherapy, it can be advantageous to use a combination of TuAAs both due to more complete coverage of the population of tumor cells, and because there will be less chance of tumor escape through loss of expression of the TuAAs. In preferred embodiments, this multivalent attack technique is employed when a tumor is positive for two, three, four or more TuAAs of the combination used.

Multivalent attack can offer another advantage in increasing the sensitivity of the tumor to attack. If more than a single antigen on a tumor cell is targeted, the effective concentration of antitumor agent is increased. In addition, attack on stroma associated with the tumor, such as vasculature, can increase the accessibility of the tumor cells to the agent(s) targeting them. Thus, even an antigen that is also expressed on some normal tissue can receive greater consideration as a target antigen, if the other antigens to be targeted in a multivalent attack are not also expressed by that tissue.

### Definitions

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell costimulatory molecules and is able to induce a T cell response. Well characterized pAPCs include dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNOPROTEASOME - a proteasome normally active in pAPCs; the immunoproteasome is also active in some peripheral cells in infected tissues or following exposure to interferon.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptides presented on the surface of cells, the peptides being non-covalently bound to the binding cleft of class I MHC, such that they can interact with T cell receptors (TCR). Epitopes presented by class I MHC may be in immature or mature form. "Mature" refers to an MHC epitope in distinction to any precursor ("immature") that may include or consist essentially of a housekeeping epitope, but also includes other sequences in a primary translation product that are removed by processing, including without limitation, alone or in any combination, proteasomal digestion, N-terminal trimming, or the action of exogenous enzymatic activities. Thus, a mature epitope may be provided embedded in a somewhat longer polypeptide, the immunological potential of which is due, at least in part, to the embedded epitope; likewise, the mature epitope can be provided in its ultimate form that can bind in the MHC binding cleft to be recognized by TCR.

MHC EPITOPE - a polypeptide having a known or predicted binding affinity for a mammalian class I or class II major histocompatibility complex (MHC) molecule.

HOUSEKEEPING EPITOPE - In a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to the foregoing definitions. Exemplary housekeeping epitopes are provided in U.S. Application Pub. No. 20030220239 A1, and in PCT Application Pub. No. WO04022709A2, filed 9/5/2003; and US application Pub. No. 20040180354 A1. Each of the listed applications is entitled "EPITOPE SEQUENCES."

IMMUNE EPITOPE - In a preferred embodiment, an immune epitope is defined as a polypeptide fragment that is an MHC epitope, and that is displayed on a cell in which immunoproteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - In a preferred embodiment, a target cell is a cell associated with a pathogenic condition that can be acted upon by the components of the immune system, for example, a cell infected with a virus or other intracellular parasite, or a neoplastic cell. In another embodiment, a target cell is a cell to be targeted by the disclosed vaccines and methods. Examples of target cells according to this definition include but are not necessarily limited to: a neoplastic cell and a cell harboring an intracellular parasite, such as, for example, a virus, a bacterium, or a protozoan. Target cells can also include cells that are targeted by CTL as a part of an assay to determine or confirm proper epitope liberation and processing by a cell expressing immunoproteasome, to determine T cell specificity or immunogenicity for a desired epitope. Such cells can be transformed to express the liberation sequence, or the cells can simply be pulsed with peptide/epitope.

TARGET-ASSOCIATED ANTIGEN (TAA) - a protein or polypeptide present in a target cell.

TUMOR-ASSOCIATED ANTIGEN (TuAA) - a TAA, wherein the target cell is a neoplastic cell. In alternate embodiments, a TuAA is an antigen associated with non-cancerous cells of the tumor such as tumor neovasculature or other stromal cells within the tumor microenvironment.

HLA EPITOPE - a polypeptide having a known or predicted binding affinity for a human class I or class II HLA complex molecule.

ANTIBODY - a natural immunoglobulin (Ig), poly- or monoclonal, or any molecule composed in whole or in part of an Ig binding domain, whether derived biochemically, or by use of recombinant DNA, or by any other means. Examples include *inter alia,* F(ab), single chain Fv, and Ig variable region-phage coat protein fusions.

SUBSTANTIAL SIMILARITY - this term is used to refer to sequences that differ from a reference sequence in an inconsequential way as judged by examination of the sequence. Nucleic acid sequences encoding the same amino acid sequence are substantially similar despite differences in degenerate positions or minor differences in length or composition of any non-coding regions. Amino acid sequences differing only by conservative substitution or minor length variations are substantially similar. Additionally, amino acid sequences comprising housekeeping epitopes that differ in the number of N-terminal flanking residues, or immune epitopes and epitope clusters that differ in the number of flanking residues at either terminus, are substantially similar. Nucleic acids that encode substantially similar amino acid sequences are themselves also substantially similar.

FUNCTIONAL SIMILARITY - this term is used to refer to sequences that differ from a reference sequence in an inconsequential way as judged by examination of a biological or biochemical property, although the sequences may not be substantially similar. For example, two nucleic acids can be useful as hybridization probes for the same sequence but encode differing amino acid sequences. Two peptides that induce cross-reactive CTL responses are functionally similar even if they differ by non-conservative amino acid substitutions (and thus may not be within the substantial similarity definition). Pairs of antibodies, or TCRs, that recognize the same epitope can be functionally similar to each other despite whatever structural differences exist. Testing for functional similarity of immunogenicity can be conducted by immunizing with the "altered" antigen and testing the ability of an elicited response, including but not limited to an antibody response, a CTL response, cytokine production, and the like, to recognize the target antigen. Accordingly, two sequences may be designed to differ in certain respects while retaining the same function.

EXPRESSION CASSETTE - a polynucleotide sequence encoding a polypeptide, operably linked to a promoter and other transcription and translation control elements, including but not limited to enhancers, termination codons, internal ribosome entry sites, and polyadenylation sites. The cassette can also include sequences that facilitate moving it from one host molecule to another.

EMBEDDED EPITOPE - in some embodiments, an embedded epitope is an epitope that is wholly contained within a longer polypeptide; in other embodiments, the term also can include an epitope in which only the N-terminus or the C-terminus is embedded such that the epitope is not wholly in an interior position with respect to the longer polypeptide.

MATURE EPITOPE - a peptide with no additional sequence beyond that present when the epitope is bound in the MHC peptide-binding cleft.

EPITOPE CLUSTER - a polypeptide, or a nucleic acid sequence encoding it, that is a segment of a protein sequence, including a native protein sequence, comprising two or more known or predicted epitopes with binding affinity for a shared MHC restriction element. In preferred embodiments, the density of epitopes within the cluster is greater than the density of all known or predicted epitopes with binding affinity for the shared MHC restriction element within the complete protein sequence.

LIBERATION SEQUENCE - a designed or engineered sequence comprising or encoding a housekeeping epitope embedded in a larger sequence that provides a context allowing the housekeeping epitope to be liberated by processing activities including, for example, immunoproteasome activity, N terminal trimming, and/or other processes or activities, alone or in any combination.

CTLp - CTL precursors are T cells that can be induced to exhibit cytolytic activity. Secondary *in vitro* lytic activity, by which CTLp are generally observed, can arise from any combination of naïve, effector, and memory CTL *in vivo.*

MEMORY T CELL - A T cell, regardless of its location in the body, that has been previously activated by antigen, but is in a quiescent physiologic state requiring re-exposure to antigen in order to gain effector function. Phenotypically they are generally CD62L⁻ CD44^{hi} CD107α⁻ IGN-γ⁻ LTβ⁻ TNF-α⁻ and is in G0 of the cell cycle.

EFFECTOR T CELL - A T cell that, upon encountering antigen, readily exhibits effector function. Effector T cells are generally capable of exiting the lymphatic system and entering the immunological periphery. Phenotypically they are generally CD62L⁻ CD44^{hi} CD107α⁺ IGN-γ⁺ LTβ⁺ TNF-α⁺ and actively cycling.

EFFECTOR FUNCTION - Generally, T cell activation generally, including acquisition of cytolytic activity and/or cytokine secretion.

INDUCING a T cell response - Includes, in many embodiments, the process of generating a T cell response from naïve, or in some contexts, quiescent cells; activating T cells.

AMPLIFYING a T cell response - Includes, in many embodiments, the process or increasing the number of cells, the number of activated cells, the level of activity, rate of proliferation, or similar parameter of T cells involved in a specific response.

ENTRAINMENT - Includes in many embodiments an induction that confers particular stability on the immune profile of the induced lineage of T cells.

TOLL-LIKE RECEPTOR (TLR) - Toll-like receptors (TLRs) are a family of pattern recognition receptors that are activated by specific components of microbes and certain host molecules. As part of the innate immune system, they contribute to the first line of defense against many pathogens, but also play a role in adaptive immunity.

TOLL-LIKE RECEPTOR (TLR) LIGAND - Any molecule capable of binding and activating a toll-like recepetor. Examples include, without limitation: poly IC A synthetic, double-stranded RNA know for inducing interferon. The polymer is made of one strand each of polyinosinic acid and polycytidylic acid, double-stranded RNA, unmethylated CpG oligodeoxyribonucleotide or other immunostimulatory sequences (ISSs), lipopolysacharide (LPS), β-glucans, and imidazoquinolines, as well as derivatives and analogues thereof

IMMUNOPOTENTIATING ADJUVANTS - Adjuvants that activate pAPC or T cells including, for example: TLR ligands, endocytic-Pattem Recognition Receptor (PRR) ligands, quillaja saponins, tucaresol, cytokines, and the like. Some preferred adjuvants are disclosed in Marciani, D.J. Drug Discovery Today 8:934-943, 2003.

IMMUNOSTIMULATORY SEQUENCE (ISS) - Generally an oligodeoxyribonucleotide containing an unmethlylated CpG sequence. The CpG may also be embedded in bacterially produced DNA, particularly plasmids. Further embodiments include various analogues; among preferred embodiments are molecules with one or more phosphorothioate bonds or non-physiologic bases.

VACCINE - In preferred embodiments a vaccine can be an immunogenic composition providing or aiding in prevention of disease. In other embodiments, a vaccine is a composition that can provide or aid in a cure of a disease. In others, a vaccine composition can provide or aid in amelioration of a disease. Further embodiments of a vaccine immunogenic composition can be used as therapeutic and/or prophylactic agents.

IMMUNIZATION - a process to induce partial or complete protection against a disease. Alternatively, a process to induce or amplify an immune system response to an antigen. In the second definition it can connote a protective immune response, particularly proinflammatory or active immunity, but can also include a regulatory response. Thus in some embodiments immunization is distinguished from tolerization (a process by which the immune system avoids producing proinflammatory or active immunity) while in other embodiments this term includes tolerization.

ENCODE - an open-ended term such that a nucleic acid encoding a particular amino acid sequence can consist of codons specifying that (poly)peptide, but can also comprise additional sequences either translatable, or for the control of transcription, translation, or replication, or to facilitate manipulation of some host nucleic acid construct.

COVERAGE - the fraction or proportion of tumor cells expressing a particular TuAA or at least one TuAA from a set of selected TuAAs.

REDUNDANCY - the degree to which a population of tumor cells, or some subset of them, express more than one of a selected set of TuAAs.

CO-TARGETING - in preferred embodiments, co-targeting involves inducing and/or amplifying an immune response against a target cell, while also inducing an immune response against at least one other agent in the vicinity and/or *milieu* of a tumor. In some embodiments, agents within the vicinity and/or *milieu* of the tumor include, but are not limited to, cancer cells, stromal cells, including those associated with neovasculature, endothelial cells, fibroblasts, inflammatory cells, epithelial cells, autocrine factors, and paracrine factors. In some embodiments, neoplastic cells and stromal cells are specifically targeted. In other embodiments, an immune response is induced and/or amplified against neovasculature and other non-transformed, non-lymphoid cells within the tumor microenvironment. In still other embodiments, an immune response is induced against cancer cells and autocrine and/or paracrine factors produced by cells in the tumor microenvironment.

### Cancer Immunotherapy and Diagnosis

Cancer immunotherapy has been strongly influenced by work on melanoma and prostate cancer, as they have been among the earliest and most widely approached targets in the field. Many of the antigens used in the various attempts to develop therapeutic vaccines for these cancers have been differentiation markers, that is antigens specific to that cell type. As a result, the existing paradigm is to develop immunotherapuetics for a particular type of cancer. Patients are then treated with the agent simply because they have been diagnosed with a particular type of cancer. In some instances, a patient's tumor is first evaluated for expression of a particular target antigen. However, many of the TuAAs now known, even some initially classified as differentiation markers, are expressed in many types of cancer. As such it can be useful to classify tumors by the TuAAs that they express rather than, or in addition to, their tissue of origin. Thus, in some embodiments, a single immunotherapeutic, preferably multivalent, can be used to treat a wide variety of tumor types. This is not to say that any particular antigen or combination of antigens will be uniformly useful across all, or even many, tumor types. To the contrary, expression frequency can vary considerably from type to type. Moreover, among the widely expressed TuAAs it is common that they are expressed in only a fraction of any particular tumor type. Indeed, this is part of the impetus to apply immunotherapeutics based on these antigens to various tumor types. Nonetheless, both individual antigens, and combinations of them, will have definable frequencies associated with particular tumor types. Thus, immunotherapeutics designed according to the more favorable frequencies observed can be more effective and/or efficient when applied to those particular tumor types.

Despite the prevalence of certain TuAAs, or combinations of them, in particular tumor types, it is not always sufficient to simply treat patients having a particular type of tumor with an immunotherapeutic targeting a prevalent antigen or antigens expressed by that tumor type. Preferably, patients' tumor tissue should be screened for the expression of TuAAs for which there is a corresponding immunotherapeutic available, whether marketed or in clinical trials. As the number and variety of cancer immunotherapies grow it will be increasingly advantageous to screen any particular patient's tumor tissue for expression of a variety of TuAAs that can be expressed by that tumor type so as to afford the clinician the widest choice in matching a cancer condition with available immunotherapies.

Although much of this disclosure focuses on agents that actively induce immunity mediated by class I MHC restricted T cells, the matching procedures described are equally applicable with immunotherapies of all kinds, active or passive, cellular or humoral, or any combination thereof. The methods claimed herein are adapted to this developing environment where there is a substantial number of immunotherapies targeting various antigens. The methods embodied herein optimize the matching between a particular patient's cancer type or condition to available immunotherapeutics. This is in contrast to existing practice that is designed to simply qualify a patient as eligible (or ineligible) for treatment with one particular agent.

Preferably, a panel of TuAAs that are expressed with relatively high frequency in a particular tumor type is assembled and assays established. Accordingly, one embodiment of the invention described herein includes assembly of the panel and establishment of appropriate assays. It can be advantageous to include a TuAA that is widely expressed in a variety of tumor types in the panel.

The methods disclosed herein can begin with an assay of a tumor tissue of the corresponding presumptive type for expression of a preselected panel of antigens. In some embodiments, a panel of TuAAs assembled for one tumor type can be used to screen other tumor types that can express at least some of the same antigens. In some embodiments, an expression profile is developed using the assay results. Selection of an appropriate immunotherapeutic can be based on how well the composition of the immunotherapeutic, such as immunogens (or effector agents in the case of passive immunotherapy), corresponds to the detected antigens of the panel. The panel can include more antigens than are likely to be targeted by any immunotherapeutic of well-defined composition, or detected in any one tissue sample.

Thus, it is not necessary that an immunotherapeutic agent comprise immunogens corresponding to every antigen in the panel. Nor is it required that the panel antigens all be the target of some set of immunotherapeutic agents that could reasonably be combined in a regimen of immunotherapy. Also, although advantageous, it is not required that there is a perfect match between the composition of the immunotherapeutic(s) and the expression profile of the tumor. Heterogeneity of antigen expression by a patient's tumor is common. Thus, there is a significant possibility of an antigen, undetectable in a tissue sample, nonetheless being expressed at another site. This is especially true for the antigens most commonly expressed by the particular tumor type. Thus, a multivalent immunotherapeutic in which one, some, or all of the constituent immunogens correspond to TuAAs expressed by an assayed portion of a patient's tumor can be used to treat that patient according to the judgment of the clinician. Similarly the patient can be treated with a combination of multi- and monovalent agents to optimize the match between the expression profile and the antigens targeted. Passive immunotherapeutics in particular are often monovalent, but the skilled clinician will nonetheless understand how they can be combined with additional passive or active immunotherapeutics into a useful immunotherapeutic regimen. Passive immunotherapies currently known in the art include: trastuzumab (HERCEPTIN®) which targets the TuAA HER2/Neu; bevacizumab (AVASTIN®) which targets VEGF (vascular endothelilal growth factor) to inhibit vascularization of tumors; cetuximab (ERBITUX™) which targets the antigen epidermal growth factor receptor (EGFR, HER1, c-erbB-1); and panitumumab which also targets the antigen epidermal growth factor receptor. Additionally there are several mono- and multivalent active immunotherapeutic in development. These include APC8015 (PROVENCE®) which targets prostatic acid phosphatase; APC8024 which targets HER2/Neu; MKC1106 which targets PRAME, PSMA, SSX-2, and NY-ESO-1; pSEM (SYNCHROVAX™ SEM) which targets Melan-A; MKC1207 which targets Melan-A and tyrosinase; pTA2M (SYNCHROTOPE® TA2M) which targets tyrosinase; DCVAX®-prostate which targets PSMA; ALVAC(2)-gp100M which targets gp100; ALVAC MAGE 1,3 which targets MAGE-1 and MAGE-3; ALVAC CEA which target CEA; the NY-ESO-1/ISCOMATRATM vaccine which targets NY-ESO-1; PANVAC™-VF which targets CEA; and MUC-1; and PROSTVAC®-VF which targets PSA.

Diagnosis of cancer type can be challenging, leading to uncertainty. Similar screening assays can be used to establish or confirm the diagnosis of tumor type by including a lineage specific marker in the panel. The marker can itself be a TuAA, as in tyrosinase for melanoma and PSA for prostate, for example. Alternatively, the marker can be any antigen that is reasonably specific to the cell type in question and the expression of which is maintained in neoplastic cells, for example, mammaglobin for breast tissue.

### Assay Technology

Many technologies to carry out the assay steps of the invention are known in the art. Generally, any reliable method of detecting specific proteins or mRNAs can be adapted. Preference is given to techniques based on characteristics such as the ability to assay large numbers of samples and/or provide results quickly or that the assay is inexpensive to practice, or some optimum of these parameters. Tumor tissue to assay can be obtained as bulk tissue through surgery or in cellular form from blood, bone marrow, cell aspirates, peritoneal lavage, plural aspirates, or bronchial washes, and the like.

The assaying step can include a determination of at least one of presence, absence, abundance or condition of a TuAA in the panel. In some embodiments, the determination includes analysis of at least one of: mRNA, peptide, polypeptide, mature protein, and MHC-peptide complex. In some embodiments, the determination includes analysis of at least one of: processing state of a polypeptide, differential splicing of a nucleic acid, mutation of a nucleic acid in comparison with a germline sequence, variation of a nucleic acid or polypeptide sequence from a consensus sequence in a population, cellular localization, subcellular localization, and co-expression with a marker.

Commonly, detection of specific proteins involves the use of antibodies. Immunohistochemistry (IHC) is broadly applicable, but western hybridization, radioimmunoassay (RIA), and flow cytometry can also be used; collectively protein determinations. TRC-tetramers and antibodies recognizing specific peptide-MHC complexes can also be used. Tumor tissue can be used as target or stimulator in a wide variety of immunological assays (Elispot, T cell hybridoma reactivity, microcytotoxicity, and the like). Such assays are specific for a target epitope, not just the parent antigen, and thus can be referred to as epitope determinations. Detection of specific mRNA can be accomplished using any of several modalities of RT-PCR (reverse transcription-polymerase chain reaction) and similar nucleic acid amplification techniques (*e.g*., 3SR), northern hybridization, querrying of gene arrays with mRNA or cDNA, and in situ hybridization; collectively transcript determinations. Reagents that detect presentation of particular T cell epitopes from target antigens can also be used. These include, for example, T cell lines and hybridomas, and more preferably, antibodies specific for the peptide-MHC complex and TCR tetramers (see for example Li et al. Nature Biotech. 23:349-354, 2005.

PCR techniques are sensitive and generally easy to implement, however they cannot detect the mosaicism of antigen expression within a sample. IHC (and other in situ techniques), though potentially more labor intensive, allow spatial variation of expression within a sample to be observed. Thus, distinctions between co-expression of antigens within the same cells versus co-expression within different cells within the same sample can be made. Both situations can be desirable, the former providing for greater redundancy of targeting and reduced likelihood of antigen-loss escape mutants arising, the latter revealing how a greater proportion of the total tumor tissue can be directly targeted Such information is also relevant to the use of antigens with more complex expression patterns. For example, PSMA, which can be expressed by prostate cells and tumor neovasculature, can be used as a prostate lineage marker if its expression can be associated specifically with the neoplastic cells, either through use of an in situ detection methodology or microdissection before assaying expression.

In preferred embodiments of the invention the immunotherapeutic agent induces a T cell response, especially including a class I MHC-restricted T cell response. Thus, it can be advantageous to confirm MHC expression by the tumor tissue. Reagents for detection of MHC, including for PCR and antibody based methods, are widely known in the art. Class-, locus- and type-specific reagents are in common usage. Class I expression can also be assessed by detection of β2-microglobulin. Class- and locus-specific reagents offer the advantage of a broadly applicable uniform procedure. Type-specific reagents allow for simultaneous confirmation of expression and MHC type. Antibody-based techniques can offer the advantage of directly detecting protein expression at the cell surface, which is of clinical relevance, in contrast to RT-PCR and the like, from which surface expression can only be inferred. TCR tetramer-based assays allow simultaneous confirmation of both MHC and target antigen (indeed, even target epitope) expression and are inherently type specific.

### Panel Design

Several modalities of the disclosed methods are envisioned. The first is concerned primarily with identifying antigens that are available to be targeted in a particular patient's tumor tissue. Thus, in some embodiments, the panel of antigens assayed for in practicing the method disclosed herein is assembled from more commonly expressed TuAAs for which targeting immunotherapeutics are available (marketed or in development). Antigens can be included in a panel on a prospective basis, for example, due to common or highly specific expression in one or another subset of tumors, or in anticipation of the development of a corresponding therapeutic product. Thus, the same research observations that indicate an antigen would be a good target for immunotherapy (e.g., specificity, prevalence, and level of expression; presentation for T cell based products or surface expression for antibody based products; and that by inclusion in a multivalent immunotherapeutic redundancy or breadth of targeting can be increased) also can justify inclusion of that antigen in the diagnostic panels of the invention:

An antigen whose expression is specific to a particular tumor type, such as tyrosinase in melanoma, is suitable for panels used in sceening that tumor type. An antigen that is expressed in a variety of tumor types, even if not highly prevalent in any particular one, can be suitable for inclusion in panels used to screen that variety of tumor types or in panels used as a general screen, e.g., not tied to an individual tumor type. In some embodiments, the panel of antigens specifically excludes markers from complex expression profiles associated with cancer, and the like, that are not appropriate targets of immunotherapy.

The histologic origin of a tumor is generally of clinical interest, for example, in designing a treatment strategy or confirming that an apparent recurrence is related to the presumptive original cancer. To this end lineage markers can be included in the panels of antigens.

### Marketing of Cancer Immunotherapeutics

Embodiments of the invention disclosed herein relate to methods for identifying patients that can benefit from particular cancer immunotherapies which can also be useful in the identification of candidates for participation in clinical trails of such products and in marketing the vaccines. Much diagnostic work for cancer is carried out in centralized labs. Whether for recruitment or marketing, an arrangement is made with one or more of these laboratories. The arrangement can entail a fee-for-service contract or a partnership or joint venture. The laboratory includes TuAA expression profiling assays, as described herein, in their standard panel of tests carried out on submitted tumor samples and the results are reported along with those of the other tests. When a tumor sample is identified as positive for one of more antigens corresponding to constituent immunogens of a vaccine a notice is included in the same communication as the test report alerting the doctor (or patient if the results of the test are reported directly to the patient) to the availability of a clinical trial the patient may be eligible for or a product that the patient may benefit from.

### Tumor Associated Antigens

Examples of TuAAs useful in embodiments disclosed herein include tyrosinase (SEQ. ID NO. 1), melan-A, (SEQ. ID NO. 2), SSX-2, (SEQ. ID NO.3), PSMA (prostate-specific membrane antigen) (SEQ. ID NO. 4), MAGE-1 (SEQ. ID NO. 5), MAGE-3 (SEQ. ID NO. 6), NY-ESO-1 (SEQ. ID NO. 7), PRAME (SEQ ID NO.8), Her2/Neu (SEQ ID NO. 9), PSA (SEQ ID NO. 10), C35 (SEQ ID NO. 11), SSX-4 (SEQ ID NO. 12), gp100 (SEQ ID NO. 13), thyroid transcription factor 1 (TTF1) (SEQ ID NO. 14), mammaglobin (SEQ ID NO. 15), prolactin-inducible protein (Brst2) (SEQ ID NO. 16), mesothelin, isoform 1 (SEQ ID NO. 17), mesothelin, isoform 2 (SEQ ID NO. 18), PSCA (SEQ ID NO. 19) and SCP-1 (SEQ ID NO. 20). The natural coding sequences for these 20 proteins, or any segments within them, can be determined from their cDNA or complete coding (cds) sequences, SEQ. ID NOS. 21-40, respectively. The sequences described in Table 1 are provided in the Sequence Listing filed herewith.

**Table 1. SEQ. ID NOS.**

| **SEQ. ID NO.** | **IDENTITY** | **ACCESSION NUMBER**** |
|---|---|---|
| 1 | Tyrosinase protein | P14679 |
| 2 | Melan-A protein | Q16655 |
| 3 | SSX-2 protein | NP_003138 |
| 4 | PSMA protein | NP_004467 |
| 5 | MAGE-1 protein | P43355 |
| 6 | MAGE-3 protein | P43357 |
| 7 | NY-ESO-1 protein | P78358 |
| 8 | PRAME protein | NP_006106 |
| 9 | Her2/Neu protein | P04626 |
| 10 | PSA protein | NP_001639 |
| 11 | C35 protein | NP_115715 |
| 12 | SSX-4 protein | NP_783856 |
| 13 | gp 100 protein | NP_008859 |
| 14 | TTF1 protein | NP_003308 |
| 15 | mammaglobin protein | NP_002402 |
| 16 | Brst2 protein | NP_002643 |
| 17 | Mesothelin, isoform 1, protein | NP_005814 |
| 18 | Mesothelin, isoform 2, protein | NP_037536 |
| 19 | PSCA protein | NP_005663 |
| 20 | SCP-1 protein | Q15431 |
| 21 | Tyrosinase cDNA | NM_000372 |
| 22 | Melan-A cDNA | U06452 |
| 23 | SSX-2 cDNA | NM_003147 |
| 24 | PSMA cDNA | NM_004476 |
| 25 | MAGE-1 cds | M77481 |
| 26 | MAGE-3 cds | U03735 |
| 27 | NY-ESO-1 cDNA | U87459 |
| 28 | PRAME cDNA | NM_006115 |
| 29 | Her2/Neu cDNA | M11730 |
| 30 | PSA cDNA | NM_001648 |
| 31 | C35 cDNA | NM_032339 |
| 32 | SSX-4 cDNA | NM_175729 |
| 33 | gp100 cDNA | NM_006928 |
| 34 | TTF1 cDNA | NM_003317 |
| 35 | mammaglobin cDNA | NM_002411 |
| 36 | Brst2 cDNA | NM_002652 |
| 37 | Mesothelin, isoform 1, cDNA | NM_005823 |
| 38 | Mesothelin, isoform 2, cDNA | NM_013404 |
| 39 | PSCA cDNA | NM_005672 |
| 40 | SCP-1 cDNA | NM_003176 |

| | | |
|---|---|---|
| **All accession numbers used here and throughout can be accessed through the NCBI databases, for example, through the Entrez seek and retrieval system on the world wide web. | | |

Tyrosinase is a melanin biosynthetic enzyme that is considered one of the most specific markers of melanocytic differentiation. Tyrosinase is expressed in few cell types, primarily in melanocytes, and high levels are often found in melanomas. The usefulness of tyrosinase as a TuAA is taught in U.S. Patent 5,747,271 entitled "METHOD FOR IDENTIFYING INDIVIDUALS SUFFERING FROM A CELLULAR ABNORMALITY SOME OF WHOSE ABNORMAL CELLS PRESENT COMPLEXES OF HLA-A2/TYROSINASE DERIVED PEPTIDES, AND METHODS FOR TREATING SAID INDIVIDUALS".

GP100, also known as PMe117, is another melanin biosynthetic protein expressed at high levels in melanomas. GP100 as a TuAA is disclosed in U.S. Patent 5,844,075 entitled "MELANOMA ANTIGENS AND THEIR USE IN DIAGNOSTIC AND THERAPEUTIC methods,".

Melan-A, also known as MART-1 (Melanoma Antigen Recognized by T cells), is another melanin biosynthetic protein expressed at high levels in melanomas. The usefulness of Melan-A/MART-1 as a TuAA is taught in U.S. Patent Nos. 5,874,560 and 5,994,523 both entitiled "MELANOMA ANTIGENS AND THEIR USE IN DIAGNOSTIC AND THERAPEUTIC METHODS," as well as U.S. Patent No. 5,620,886, entitled "ISOLATED NUCLEIC ACID SEQUENCE CODING FOR A TUMOR REJECTION ANTIGEN PRECURSOR PROCESSED TO AT LEAST ONE TUMOR REJECTION ANTIGEN PRESENTED BY HLA-A2,".

SSX-2, also know as Hom-Mel-40, is a member of a family of highly conserved cancer-testis (CT) antigens (Gure, A.O. et al. Int. J. Cancer 72:965-971, 1997). Its identification as a TuAA is taught in U.S. Patent 6,025,191 entitled "ISOLATED NUCLEIC ACID MOLECULES WHICH ENCODE A MELANOMA SPECIFIC ANTIGEN AND USES THEREOF,". Cancer-testis antigens are found in a variety of tumors, but are generally absent from normal adult tissues except testis. Expression of different members of the SSX family has been found in various tumor cell lines. Due to the high degree of sequence identity among SSX family members, similar epitopes from more than one member of the family will be generated and able to bind to an MHC molecule, so that some vaccines directed against one member of this family can cross-react and be effective against other members of this family.

MAGE-1 (melanoma-associated antigen-1), MAGE-2 (melanoma-associated antigen-2), and MAGE-3 (melanoma-associated antigen-3) are members of another family of cancer-testis antigens originally discovered in melanoma but found in a variety of tumors. The identification of MAGE proteins as TuAAs is taught in U.S. Patent 5,342,774, entitled "NUCLEOTIDE SEQUENCE ENCODING THE TUMOR REJECTION ANTIGEN PRECURSOR, MAGE-1," and in numerous subsequent patents. Currently there are 17 entries for (human) MAGE in the SWISS Protein database. There is extensive similarity among these proteins, such that in many cases, an epitope from one can induce a cross-reactive response to other members of the family. A few members of the MAGE family have not been observed in tumors, most notably MAGE-H1 and MAGE-D1, which are expressed in testes and brain, and bone marrow stromal cells, respectively. The possibility of cross-reactivity on normal tissue is ameliorated by the fact that they are among the least similar to the other MAGE proteins.

GAGE-1 is a member of the GAGE family of cancer testis antigens (Van den Eynde, B., et al., J. Exp. Med. 182: 689-698, 1995; U.S Patent Nos. 5,610,013; 5648226; 5,858,689; 6,013,481; and 6,069,001). The PubGene database currently lists 12 distinct accessible members, some of which are synonymously known as PAGE or XAGE. GAGE-1 through GAGE-8 have a very high degree of sequence identity, so most epitopes can be shared among multiple members of the family.

BAGE is a cancer-testis antigen commonly expressed in melanoma, particularly metastatic melanoma, as well as in carcinomas of the lung, breast, bladder, and squamous cells of the head and neck. Its usefulness as a TuAA is taught in U.S. Patent Nos. 5,683,88, entitled "TUMOR REJECTION ANTIGENS WHICH CORRESPOND TO AMINO ACID SEQUENCES IN TUMOR REJECTION ANTIGEN PRECURSOR BAGE, AND USES THEREOF" and 5,571,711, entitled "ISOLATED NUCLEIC ACID MOLECULES CODING FOR BAGE TUMOR REJECTION ANTIGEN PRECURSORS".

NY-ESO-1, also known as CTAG-1 (Cancer-Testis Antigen-1) and CAG-3 (Cancer Antigen-3), is a cancer-testis antigen found in a wide variety of tumors. NY-ESO-1 as a TuAA is disclosed in U.S. Patent 5,804,381, entitled "ISOLATED NUCLEIC ACID MOLECULE ENCODING AN ESOPHAGEAL CANCER ASSOCIATED ANTIGEN, THE ANTIGEN ITSELF, AND USES THEREOF,". A paralogous locus encoding antigens with extensive sequence identity, LAGE-1a/s and LAGE-1b/L, has been disclosed in publicly available assemblies of the human genome, and has been concluded to arise through alternate splicing. Additionally, CT-2 (or CTAG-2, Cancer-Testis Antigen-2) appears to be either an allele, a mutant, or a sequencing discrepancy of LAGE-1b/L. Due to the extensive sequence identity, many epitopes from NY-ESO-1 can also induce immunity to tumors expressing these other antigens. NY-ESO-1 and LAGE are virtually identical through amino acid 70. From amino acid 71 through 134 the longest run of identity between the two proteins is 6 residues, but potentially cross-reactive sequences are present. From amino acid 135 through 180, NY-ESO and LAGE-1a/s are identical except for a single residue, but LAGE-1b/L is unrelated due to the alternate splice. The CAMEL and LAGE-2 antigens appear to derive from the LAGE-1 mRNA, but from alternate reading frames, thus giving rise to unrelated protein sequences. More recently, GenBank Accession AF277315.5, Homo sapiens chromosome X clone RP5-865E18, RP5-1087L19, complete sequence, reports three independent loci in this region which are labeled as LAGE1 (corresponding to CTAG-2 in the genome assemblies), LAGE2-A and LAGE2-B (both corresponding to CTAG-1 in the genome assemblies).

PRAME, also know as MAPE, DAGE, and OIP4, was originally observed as a melanoma antigen. Subsequently, it has been recognized as a cancer-testis (CT) antigen, but unlike many CT antigens, such as, MAGE, GAGE and BAGE, PRAME is expressed in acute myeloid leukemias. PRAME is a member of the MAPE family, which consists largely of hypothetical proteins with which it shares limited sequence similarity. The usefulness of PRAME as a TuAA is taught in U.S. Patent 5,830,753, entitled "ISOLATED NUCLEIC ACID MOLECULES CODING FOR TUMOR REJECTION ANTIGEN PRECURSOR DAGE AND USES THEREOF,".

PSMA (prostate-specific membranes antigen), a TuAA described in U.S. Patent 5,538,866 entitled "PROSTATE-SPECIFIC MEMBRANES ANTIGEN" which is hereby incorporated by reference in its entirety, is expressed by normal prostate epithelium and, at a higher level, in prostatic cancer. Additionally expression has also been observed in ovarian carcinoma. It has also been found in the neovasculature of non-prostatic tumors. PSMA can thus form the basis for vaccines directed to both prostate and ovarian cancer and to the neovasculature of other tumors. This later concept is more fully described in a U.S. Application Pub. No. 20030046714 A1, filed on March 7, 2002, entitled "ANTI-NEOVASCULAR PREPARATIONS FOR CANCER,". Briefly, as tumors grow they recruit ingrowth of new blood vessels. This is understood to be necessary to sustain growth as the centers of unvascularized tumors are generally necrotic and angiogenesis inhibitors have been reported to cause tumor regression. Such new blood vessels, or neovasculature, express antigens not found in established vessels, and thus can be specifically targeted. By inducing CTL against neovascular antigens the vessels can be disrupted, interrupting the flow of nutrients to, and removal of wastes from, tumors, leading to regression.

Alternate splicing of the PSMA mRNA leads to a protein with an apparent start at Met₅₈, thereby deleting the putative membrane anchor region of PSMA as described in U.S. Patent 5,935,818, entitled "ISOLATED NUCLEIC ACID MOLECULE ENCODING ALTERNATIVELY SPLICED PROSTATE-SPECIFIC MEMBRANES ANTIGEN AND USES THEREOF,". A protein termed PSMA-like protein, Genbank accession number AF261715, is nearly identical to amino acids 309-750 of PSMA, but has a different expression profile. Thus, the most preferred epitopes are those with an N-terminus located from amino acid 58 to 308.

PSA (prostate specific antigen) is a peptidase of the kallikrein family and a differentiation antigen of the prostate. Expression in breast tissue has also been reported. Alternate names include gamma-seminoprotein, kallikrein 3, seminogelase, seminin, and P-30 antigen. PSA has a high degree of sequence identity with the various alternate splicing products prostatic/glandular kallikrein-1 and -2, as well as kalikrein 4, which is also expressed in prostate and breast tissue. Other kallikreins generally share less sequence identity and have different expression profiles. Nonetheless, cross-reactivity that might be provoked by any particular epitope, along with the likelihood that that epitope would be liberated by processing in non-target tissues (most generally by the housekeeping proteasome), should be considered in designing a vaccine.

PSCA (prostate stem cell antigen) and also known as SCAH-2, is a differentiation antigen preferentially expressed in prostate epithelial cells, and overexpresssed in prostate cancers. Lower level expression is seen in some normal tissues including neuroendocrine cells of the digestive tract and collecting ducts of the kidney. PSCA is described in U.S. Patent 5,856,136, entitled "HUMAN STEM CELL ANTIGENS,".

Synaptonemal complex protein 1 (SCP-1), also known as HOM-TES-14, is a meiosis-associated protein and also a cancer-testis antigen (Tureci, O., et al. Proc. Natl. Acad. Sci. USA 95:5211-5216, 1998). As a cancer antigen its expression is not cell-cycle regulated and it is found frequently in gliomas, breast, renal cell, and ovarian carcinomas. It has some similarity to myosins, but with few enough identities that cross-reactive epitopes are not an immediate prospect.

The ED-B domain of fibronectin is also a potential target. Fibronectin is subject to developmentally regulated alternative splicing, with the ED-B domain being encoded by a single exon that is used primarily in oncofetal tissues (Matsuura, H. and S. Hakomori Proc. Natl. Acad. Sci. USA 82:6517-6521, 1985; Carnemolla, B. et al. J. Cell Biol. 108:1139-1148, 1989; Loridon-Rosa, B. et al. Cancer Res.50:1608-1612, 1990; Nicolo, G. et al. Cell Differ. Dev. 32:401-408, 1990; Borsi, L. et al. Exp. Cell Res. 199:98-105, 1992; Oyama, F. et al. Cancer Res. 53:2005-2011, 1993; Mandel, U. et al. APMIS 102:695-702, 1994; Famoud, M.R. et al. Int. J. Cancer 61:27-34, 1995; Pujuguet, P. et al. Am. J. Pathol. 148:579-592, 1996; Gabler, U. et al. Heart 75:358-362, 1996;Chevalier, X Br. J. Rheumatol. 35:407-415, 1996; Midulla, M. Cancer Res. 60:164-169, 2000).

The ED-B domain is also expressed in fibronectin of the neovasculature (Kaczmarek, J. et al. Int. J. Cancer 59:11-16, 1994; Castellani, P. et al. Int. J. Cancer 59:612-618, 1994; Neri, D. et al. Nat. Biotech. 15:1271-1275, 1997; Karelina, T.V. and A.Z. Eisen Cancer Detect. Prev. 22:438-444, 1998; Tarli, L. et al. Blood 94:192-198, 1999; Castellani, P. et al. Acta Neurochir. (Wien) 142:277-282, 2000, each of which is hereby incorporated by reference in its entirety). As an oncofetal domain, the ED-B domain is commonly found in the fibronectin expressed by neoplastic cells in addition to being expressed by the neovasculature. Thus, CTL-inducing vaccines targeting the ED-B domain can exhibit two mechanisms of action: direct lysis of tumor cells, and disruption of the tumor's blood supply through destruction of the tumor-associated neovasculature. As CTL activity can decay rapidly after withdrawal of vaccine, interference with normal angiogenesis can be minimal. The design and testing of vaccines targeted to neovasculature is described in U.S. Patent Application Pub. No. 20030046714 A1; entitled "ANTI-NEOVASCULATURE PREPARATIONS FOR CANCER," filed on March 7, 2002.

Carcinoembryonic antigen (CEA) is a paradigmatic oncofetal protein first described in 1965 (Gold and Freedman, J. Exp. Med. 121: 439-462, 1965). Fuller references can be found in the Online Mendelian Inheritance in Man; record *114890. It has officially been renamed carcinoembryonic antigen-related cell adhesion molecule 5 (CEACAM5). Its expression is most strongly associated with adenocarcinomas of the epithelial lining of the digestive tract and in fetal colon. CEA is a member of the immunoglobulin supergene family and the defining member of the CEA subfamily.

Survivin, also known as Baculoviral IAP Repeat-Containing Protein 5 (BIRC5), is another protein with an oncofetal pattern of expression. It is a member of the inhibitor of apoptosis protein (IAP) gene family. It is widely over-expressed in cancers (Ambrosini, G. et al., Nat. Med. 3:917-921, 1997; Velculiscu V.E. et al., Nat. Genet. 23:387-388, 1999) and its function as an inhibitor of apoptosis is believed to contribute to the malignant phenotype.

HER2/NEU is an oncogene related to the epidermal growth factor receptor (van de Vijver, et al., New Eng. J. Med. 319:1239-1245, 1988), and apparently identical to the c-ERBB2 oncogene (Di Fiore, et al., Science 237: 178-182, 1987). ). The over-expression of ERBB2 has been implicated in the neoplastic transformation of prostate cancer. As with HER2, it is amplified and over-expressed in 25-30% of breast cancers among other tumors where expression level is correlated with the aggressiveness of the tumor (Slamon, et al., New Eng. J. Med 344:783-792, 2001). A more detailed description is available in the Online Mendelian Inheritance in Man; record *164870.

MESOTHELIN is an antigen originally found in mesotheliomas but also known to be upregulated in many pancreatic and ovarian cancers. Its use as a vaccine target, as well as useful epitopes, is described in Thomas, A.M. et al., J. Exp. Med. 200:297-306, 2004.

Further examples of tumor-associated antigens include MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGE), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein, β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like.

Additional tumor-associated antigens are described in Chen, YT, "Identification of human tumor antigens by serological expression cloning: an online review on SEREX" Cancer Immun. 2004 [updated 2004 Mar 10; cited 2004 Apr 1] *at* world wide web cancerimmunotherapy.org/SEREX/; and Renkvist, N. et al., "A listing of tumor antigens recognized by T cells," Cancer Immunology Immunotherapy, 50:3-15 (2001).

Table 2, adapted from Scanlan et al., "The cancer/testis genes: Review, standardization, and commentary," Cancer Immunity 4:1 (January 23, 2004) provides a listing of CT Antigens. Table 3 provides the frequency of mRNA expression in various tumor types for the CT antigens in Table 2. Scanlan et al., "The cancer/testis genes: Review, standardization, and commentary," Cancer Immunity 4:1 (January 23, 2004).

**Table 2**

| Listing of CT genes | | |
|---|---|---|
| **CT Identifier** | **Transcript/Transcript family** | **Family Members/CT Identifier (Synonyms)** |
| CT1 | MAGEA | MAGEA1/CT1.1, MAGEA2/CT1.2, MAGEA3/CT1.3, MAGEA4/CT1.4, MAGEA5/CT1.5, MAGEA6/CT1.6, MAGEA7/CT1.7, MAGEA8/CT1.8, MAGEA9/CT.9, MAGEA10/CT1.10, MAGEA11/CT1.11, MAGEA12/CT1.12 |
| CT2 | BAGE | BAGE/CT2.1, BAGE2/CT2.2, BAGE3/CT2.3, BAGE4/CT2.4, BAGE5/CT2.5 |
| CT3 | MAGEB | MAGEB1/CT3.1, MAGEB2/CT3.2, MAGEB5/CT3.3, MAGEB6/CT3.4 |
| CT4 | GAGE1 | GAGE1/CT4.1, GAGE2/CT4.2, GAGE3/CT4.3, GAGE4/CT4.4, GAGE5/CT4.5, GAGE6/CT4.6, GAGE7/CT4.7, GAGE8/CT4.8 |
| CT5 | SSX | SSX1/CT5.1, SSX2/CT5.2a, SSX2/CT5.2b, SSX3/CT5.3, SSX4/CT5.4 |
| CT6 | NY-ESO-1 | NY-ESO-1/CT6.1, LAGE-1a/CT6.2a, LAGE-1b/CT6.2b |
| CT7 | MAGEC1 | MAGEC1/CT7.1, MAGEC3/CT7.2 |
| CT8 | SYCP1 | SYCP1/CT8 |
| CT9 | BRDT | BRDT/CT9 |
| CT10 | MAGEE1 | MAGEE1/CT10 |
| CT11 | CTp11/SPANX | SPANXA1/CT11.1, SPANXB1/CT11.2, SPANXC/CT11.3, SPANXD/CT11.4 |
| CT12 | XAGE-1/GAGED | XAGE-1a/CT12.1a, XAGE-1b/CT12.1b, XAGE-1c/CT12.1c, XAGE-1d/CT12.1d, XAGE-2/CT12.2, XAGE-3a/CT12.3a, XAGE-3b/CT12.3b, XAGE-4/CT12.4 |
| CT13 | HAGE | HAGE/CT13 |
| CT14 | SAGE | SAGE/CT14 |
| CT15 | ADAM2 | ADAM2/CT15 |
| CT16 | PAGE-5 | PAGE-5/CT16.1, CT16.2 |
| CT17 | LIP1 | LIP1/CT17 |
| CT18 | NA88 | NA88/CT12 |
| CT19 | IL13RA1 | IL13RA1/CT19 |
| CT20 | TSP50 | TSP50/CT20 |
| CT21 | CTAGE-1 | CTAGE-1/CT21.1, CTAGE-2/CT21.2 |
| CT22 | SPA17 | SPA17/CT22 |
| CT23 | OY-TES-1 | OY-TES-1/CT23 |
| CT24 | CSAGE | CSAGE/CT24.1, TRAG3/CT24.2 |
| CT25 | MMA1/DSCR8 | MMA-1a/CT25.1a, MMA-1b/CT25.1b |
| CT26 | CAGE | CAGE/CT26 |
| CT27 | BORIS | BORIS/CT27 |
| CT28 | HOM-TES-85 | HOM-TES-85/CT28 |
| CT29 | AF15q14/D40 | D40/CT29 |
| CT30 | E2F-like/HCA661 | HCA661/CT30 |
| CT31 | PLU-1 | PLU-1/CT31 |
| CT32 | LDHC | LDHC/CT32 |
| CT33 | MORC | MORC/CT33 |
| CT34 | SGY-1 | SGY-1/CT34 |
| CT35 | SPO11 | SPO11/CT35 |
| CT36 | TPX1 | TPX-1/CT36 |
| CT37 | NY-SAR-35 | NY-SAR-35/CT37 |
| CT38 | FTHL17 | FTHL17/CT38 |
| CT39 | NXF2 | NXF2/CT39 |
| CT40 | TAF7L | TAF7L/CT40 |
| CT41 | TDRD1 | TDRD1/CT41.1, NY-CO-45/CT41.2 |
| CT42 | TEX15 | TEX15/CT42 |
| CT43 | FATE | FATE/CT43 |
| CT44 | TPTE | TPTE/CT44 |
| --- | PRAME | (MAPE, DAGE) |

**Table 3.**

| **CT Family (Member)** | **Frequency (%) of Expression in Tumor Type** | | | | | | | | | | | | | | | | **Ref** |
|---|---|---|---|---|---|---|---|---|---|---|---|---|---|---|---|---|---|
| | **Blad** | **Brn** | **Brst** | **Col** | **Eso** | **Gas** | **H/N** | **Liver** | **Leuk/ Lymph** | **Lung (NSCLC)** | **Mel** | **Ov** | **Pancr** | **Pros** | **Renal** | **Sarc** | |
| MAGEA1/CT1.1 | 22 | - | 18 | 2 | 53 | 29 | 28 | 80 | 0 | 49 | 48 | 28 | - | 15 | 0 | 14 | 44 |
| BAGE1/CT2.1 | 5 | - | 10 | 0 | - | - | 8 | - | 0 | 4 | 26 | 15 | - | 0 | 0 | 6 | 44 |
| MAGEB1/CT3.1 | - | 0 | 17 | 0 | - | 0 | 0 | - | 0 | 14 | 22 | - | - | 0 | 0 | 9 | 45 |
| GAGE/CT4.1 | 12 | - | 9 | 0 | - | - | 19 | 38^{b} | 1 | 19 | 28 | 31 | - | 10 | 0 | 25 | 44 |
| SSX2/CT5.2 | 44 | 6 | 7 | 12 | - | - | 35 | 9^{b} | 36 | 16 | 35 | - | - | 40 | 5 | 50 | 46 |
| NY-ESO-1/CT6.1 | 80 | 0 | 30 | 0 | - | 0 | - | 29 | 0 | 17 | 34 | 25 | 0 | 25 | 9 | 0 | 8 |
| MAGEC1/CT7.1 | 44 | - | 30 | 10 | - | - | 36 | - | - | 33 | 70 | - | - | - | - | 60 | 20 |
| SYCP1/CT8 | - | 47 | 20 | 0 | - | 7 | - | 28^{b} | 0 | 7 | 14 | 0 | - | 0 | 8 | 0 | 9 |
| BRDT/CT9 | 0 | - | 0 | 0 | 8 | - | 8 | - | - | 25 | 0 | - | - | - | 0 | - | 16 |
| MAGEE1/CT10 | 44 | - | 38 | 0 | - | - | 36 | - | - | 24 | 50 | - | - | - | - | 0 | 12 |
| SPANXC/CT11.3 | 9 | - | 25 | 22 | 0 | - | - | - | - | 33 | 70 | - | 0 | - | - | - | 14 |
| XAGE-1a/CT12.1a | - | - | - | - | - | - | - | - | - | - | 8 | - | - | - | - | 22 | 47 |
| HAGE/CT13 | 24 | 37 | 5 | 31 | 27 | - | - | 20 | 9 | 32 | 17 | - | - | 22 | 6 | 20 | 13 |
| SAGE/CT14 | 12 | 0 | 5 | 0 | 20 | - | 17 | - | 4 | 22 | 4 | - | - | 0 | 5 | 5 | 13 |
| ADAM2/CT15 | - | - | 0 | 0 | - | - | - | - | - | 0 | 0 | 0 | - | - | 12 | - | 17 |
| PAGE-5/CT16 | - | - | 5 | 11 | - | - | - | - | - | 39 | 22 | 0 | - | - | 44 | - | 17 |
| LIP1/CT17 | - | - | 5 | 0 | - | - | - | - | - | 0 | 0 | 0 | - | - | 25 | - | 17 |
| NA88/CT18 | - | - | - | - | - | - | - | - | - | - | 11 | - | - | - | - | - | 48 |
| TSP50/CT20 | - | - | 28 | - | - | - | - | - | - | - | - | - | - | - | - | - | 49 |
| CTAGE-1/CT21.1 | - | - | - | - | - | - | - | - | 35 | - | - | - | - | - | - | - | 50 |
| SPA17/CT22 | - | - | - | - | - | - | - | - | 26 | - | - | - | - | - | - | - | 51 |
| OYTES1/CT23 | 28 | - | 40 | 15 | - | 0 | - | 40 | - | 20 | - | - | - | - | 0 | - | 52 |
| MMA1a/CT25.1a | - | - | 0 | 0 | 0 | - | - | - | - | 40 | 26 | - | 0 | - | - | 18 | 15 |
| CAGE/CT26 | - | - | - | - | - | 89 | - | - | - | 100 | - | - | - | - | - | - | 53 |
| HOMTES85/CT28 | - | 35 | 0 | 10 | - | - | - | 19 | - | 28 | 36 | 32 | - | 0 | - | - | 54 |
| D40/CT29 | - | 20 | - | 13 | - | 0 | - | - | - | 41 | - | 36 | 27 | - | - | - | 55 |
| HCA661/CT30 | 0 | - | - | - | - | 0 | 0 | 29 | - | - | 20 | - | - | - | - | - | 56 |
| PLU-1/CT31 | - | - | 86 | - | - | - | - | - | - | - | - | - | - | - | - | - | 27 |
| LDHC/CT32 | - | - | 35 | 15 | - | - | - | - | - | 47 | 44 | 42 | - | 37 | 57 | - | 18 |
| MORC/CT33 | - | - | 0 | 0 | - | - | - | - | - | 18 | 18 | 14 | - | 0 | 0 | - | 18 |
| SGY-1/CT34 | - | - | 20 | 0 | - | - | - | - | - | 12 | 25 | 57 | - | 12 | 0 | - | 18 |
| SPO11/CT35 | - | - | 0 | 0 | - | - | - | - | - | 0 | 6 | 0 | - | 0 | 0 | - | 18 |
| TPX1/CT36 | - | - | 15 | 0 | - | - | - | - | - | - | 6 | 14 | - | 37 | 14 | - | 18 |
| NYSAR35/CT37 | 42 | - | 23 | 0 | 8 | - | - | - | - | 17 | 6 | 8 | - | - | 0 | 8 | 57 |
| FTHL17/CT38 | 22 | - | 14 | 0 | 0 | - | 10 | - | 0 | 25 | 0 | - | - | 0 | 0 | 0 | 58 |
| NXF2/CT39 | 19 | - | 0 | 11 | 12 | - | 5 | - | 0 | 15 | 55 | - | - | 14 | 0 | 27 | 58 |
| TAF7L/CT40 | 10 | - | 0 | 0 | 0 | - | 10 | - | 0 | 9 | 21 | - | - | 0 | 0 | 12 | 58 |
| TDRD1/CT41.1 | 28 | - | 37 | 0 | 10 | - | 22 | - | 5 | 5 | 0 | - | - | 38 | 0 | 0 | 58 |
| TEX15/CT42 | 21 | - | 0 | 0 | 20 | - | 11 | - | 0 | 21 | 27 | - | - | 12 | 33 | 28 | 58 |
| FATE/CT43 | - | - | - | 21 | - | 7 | - | 66 | - | 0 | - | - | - | - | - | - | 19 |
| TPTE/CT44 | - | - | - | 0 | - | 0 | - | 39 | - | 36 | - | - | - | - | - | - | 19 |

| | | | | | | | | | | | | | | | | | |
|---|---|---|---|---|---|---|---|---|---|---|---|---|---|---|---|---|---|
| ^{a}Abbreviations: Blad, bladder; Brn, brain; Brst, breast; Col, colon; Gas, gastric; H/N, head and neck; Leuk, leukemia; Lymph, Lymphoma, NSCLC, non-small cell lung carcinoma; Mel, melanoma; Ov, ovarian; Pancr, pancreatic; Pros, prostate; Sarc, sarcoma; Ref, reference. ^{b}Reference 59. | | | | | | | | | | | | | | | | | |

Additional antigens associated with tumor neovasculature include VEGFR2 (vascular endothelial growth factor receptor 2) described in U.S. Patent No. 6,342,221 and Tie-2, an endothelium specific receptor tyrosine kinase which is described in WO9943801.

In addition to the disruption of blood flow to tumors that can be achieved using anti-neovasculature agents such as those recited above, co-targeting molecules expressed on cancer cells as well as molecules expressed on underlying non-transformed stromal cells (including neovasculature as well as interstitial tissue, for example) can also improve the effectiveness of multivalent immunotherapeutics in limiting tumor growth and promoting cancer regression by other mechanisms. Stroma encompasses neovasculature as well as fibroblasts, and in general, all non-transformed, non-lymphoid cells within a tumor microenvironment. For example, immune mediated attack of the endothelial cells (via cytotoxic T lymphocytes (CTLs) or antibody dependent cytoxic cells (ADCC)) can result in neovasculature permeabilization and initiation of inflammatory events that result in recruitment and translocation of immune effectors, such as CTLs, targeting the neoplastic cells within primary tumor and metastatic lesions. Compared to strategies targeting only cancer cells, methods to co-target associated stromal tissue improve the efficacy of the former. Similarly, compared to strategies targeting neovasculature only, methods to co-target cancer cells improve the overall therapeutic effect by attacking lesions, including those of limited size and vascularization, especially those adversely located within vital organs. With regard to neovasculature, co-targeting VEGFRs (such as II), CD55 and PSMA as well as other molecules expressed by neovasculature, can be accomplished by generating CTL or antibodies with capability to initiate ADCC or complement activated cell injury. Alternatively, initial endothelial injury can be brought about though passive immunotherapy using available anti-angiogenic antibodies.

In addition or alternatively, co-targeting target-associated antigens, together with growth, metastasis, or survival promoting factors produced by cancer cells or non-transformed cells that are found in the extracellular compartment (diffusing or associated with the extracellular matrix), can also result in a more substantial therapeutic effect. By co-targeting antigens expressed within or on cancer cells as well as factors that exert autocrine or paracrine effects (growth, survival, and/or invasiveness), the pathogenic process can be slowed or disrupted to significant degree. Co-targeting autocrine or paracrine factors (such as, but not limited to, NF-kB activating molecules - CXCL1, CXCL8, CCL2; or growth factors such as, but not limited to, chorionic-gonadotropic hormone and gastrin) can be carried out by co-induction of neutralizing antibodies or secondarily, by CTLs recognizing cells that produce such factors.

Overall, co-targeting multiple elements of biological importance for tumor growth and metastasis can limit progression of the malignant process by impacting the processes of clonal selection, immune evasion and escape. Thus, co-targeting stroma-associated antigens provides an additional mode of attack in that such activities are inhibited and/or disrupted.

One of skill in the art will appreciate that any other antigen or protein associated with vascular or other tumor-associated stromal cells can be a target for the immunogenic compositions, including those that are presently known and those yet to be identified.

### Compositions

Immunogenic compositions, including, for example, vaccines, can be prepared using whole antigen or an epitopic peptide. Peptide immunogens can be readily prepared using standard peptide synthesis means known in the art, for example. Immunogens can be prepared commercially by one of numerous companies that do chemical synthesis. An example such a company is American Peptides, Inc., where the distributor is CLINALFA AG (Laufelfingen, Switzerland). The antigens or immunogens can be prepared in accordance with GMP standards and purity can be assessed by analytical HPLC. The product can be characterized by amino-acid analysis and tested for sterility and the absence of pyrogens.

The immunogenic compositions can also include adjuvants or other biological response modifiers (BRMs). Particularly advantageous methods of using adjuvants and BRMs are disclosed in US 20060153844 A1 entitled, "METHODS TO TRIGGER, MAINTAIN AND MANIPULATE IMMUNE RESPONSES BY TARGETED ADMINISTRATION OF BIOLOGICAL RESPONSE MODIFIERS INTO LYMPHOID ORGANS," filed 12/29/2004.

An antigen can be delivered to an animal's system either directly or indirectly. For example, a polypeptide can be delivered directly as the polypeptide, or it can be delivered indirectly, for example, using a DNA construct or vector, or a recombinant virus that codes for the desired antigen. Any vector driving expression in a professional antigen presenting cell can be suitable for this purpose. In indirect delivery, the antigen is expressed in the cell, then presented by the MHC Class I on the surface of the cell to stimulate a CTL response. Expression of a secreted form of the antigen can be useful to induce an antibody response recognizing antigens that are membrane proteins.

In a preferred embodiment, an encoded antigen can be delivered in the form of a naked plasmid expression vector. Particularly useful constructs are disclosed in U.S. Patent Application filed November 17, 2002 (Pub. No. 20030228634 A1), entitled "EXPRESSION VECTORS ENCODING EPITOPES OF TARGET-ASSOCIATED ANTIGENS AND METHODS FOR THEIR DESIGN;" U.S. Patent Application filed August 20, 2002 (Pub No. 2003-0138808); PCT Application fled August 19, 2003 (Pub. No. WO 2004/018666); U.S. Patent No. 6,709,844, entitled "AVOIDANCE OF UNDESIRABLE REPLICATION INTERMEDIATES IN PLASMIND PROPAGATION," and in U.S. Patent Application filed December 7, 2001 (Pub. No. 20030215425 A1), entitled "EPITOPE SYNCHRONIZATION IN ANTIGEN PRESENTING CELLS." Additional methodology, compositions, peptides, and peptide analogues are disclosed in US 20060063913 A1, filed June 17, 2004, entitled "SSX-2 PEPTIDE ANALOGS;" US 20060094661 A1 entitled "NY-ESO PEPTIDE ANALOGS;" COMMERCIALIZING AN ANTIGEN"; US 20060165711 A1, filed on December 29, 2004, entitled, "METHODS TO ELICIT, ENHANCE AND SUSTAIN IMMUNE RESPONSES AGAINST MHC CLASS I- RESTRICTED EPITOPES, FOR PROPHYLACTIC OR THERAPEUTIC PURPOSES"; and US 20080124352 A1, filed on December 29, 2004, entitled "METHODS TO BYPASS CD4+ CELLS IN THE INDUCTION OF AN IMMUNE RESPONSE,". The feasibility of and general procedures related to the use of naked DNA for immunization are described in U.S. Patent No. 5,589,466, entitled "INDUCTION OF A PROTECTIVE IMMUNE RESPONSE IN A MAMMAL BY INJECTING A DNA SEQUENCE" and in U.S. Patent No. 5,679,647, entitled "METHODS AND DEVICES FOR IMMUNIZING A HOST AGAINST TUMOR-ASSOCIATED ANTIGENS THROUGH ADMINISTRATIONS OF NAKED POLYNUCLEOTIDES WHICH ENCODE TUMOR-ASSOCIATED ANTIGENIC PEPTIDES,". The former teaches only intramuscular or intradermal injection while the latter teaches only administration to skin or mucosa.

In a preferred embodiment, the antigen can be administered directly to the lymphatic system, Intranodal administration for the generation of CTL is taught in U.S. Patent No. 6,994,851, filed September 1, 1999 and US patent application Pub. No.20020007173 A1, and in PCT Application WO9902183A2 each entitled "A METHOD OF INDUCING A CTL RESPONSE". Single bolus injection intra lymph node (i.ln.) required only 0.1% of the dose required in order to obtain a similar level of CTL response by intramuscular (i.m.) injection. Therefore a protective response can be established against systemic viral infection with a single bolus delivered i.ln., but not with a dose nearing the practical limit delivered i.m. Repeated bolus injections i.m. failed to establish a protective response against a peripheral virus infection or transplanted tumor, whereas lower doses administered i.ln. were completely effective. Particularly useful intranodal immunization protocols are taught in US 20050079152 A1 and U.S. Patent Application Pub. No. 20050118186 A1 filed June 17, 2004, both entitled "METHODS TO CONTROL MAGNITUDE AND QUALITY THE MHC CLASS I-RESTRICTED IMMUNE RESPONSE," and in U.S. Patent Application Pub No. 20050079152 A1 and US 20060165711 A1 filed on December 29, 2004, both entitled "METHODS TO ELICIT, ENHANCE AND SUSTAIN IMMUNE RESPONSES AGAINST MHC CLASS I-RESTRICTED EPITOPES, FOR PROPHYLACTIC OR THERAPEUTIC PURPOSE", entirety.

A class of epitopes that can be advantageous in anti-cancer immunogenic compositions are housekeeping epitopes. These are produced through the action of the housekeeping (or standard) proteasome. Housekeeping epitopes can be liberated from the translation product of expression vectors through proteolytic processing by the immunoproteasome of professional antigen presenting cells (pAPC). In one embodiment of the invention, sequences flanking the housekeeping epitope(s) can be altered to promote cleavage by the immunoproteasome at the desired location(s). Housekeeping epitopes, their uses, and identification are described in US 20070269464 A1 and U.S. Patent Application Pub. No. 20030215425 A1, filed on December 7, 2001, entitled "EPITOPE SYNCHRONIZATION IN ANTIGEN PRESENTING CELLS," and U.S. Patent No. 6,861,234, entitled "METHOD OF EPITOPE DISCOVERY".

Examples of housekeeping epitopes are disclosed in US 20030220239 A1; US 20040180354 A1; U.S. Patent Application Publication No. 20030220239A1 and PCT Application Pub. No. WO04022709A2 both entitled "EPITOPE SEQUENCES,".

In other embodiments of the invention, the housekeeping epitope(s) can be flanked by arbitrary sequences or by sequences incorporating residues known to be favored in immunoproteasome cleavage sites. As used herein the term "arbitrary sequences" refers to sequences chosen without reference to the native sequence context of the epitope, their ability to promote processing, or immunological function. In further embodiments of the invention multiple epitopes can be arrayed head-to-tail. These arrays can be made up entirely of housekeeping epitopes. Likewise, the arrays can include alternating housekeeping and immune epitopes. Alternatively, the arrays can include housekeeping epitopes flanked by immune epitopes, whether complete or distally truncated. Further, the arrays can be of any other similar arrangement. There is no restriction on placing a housekeeping epitope at the terminal positions of the array. The vectors can additionally contain authentic protein coding sequences or segments thereof containing epitope clusters as a source of immune epitopes. The term "authentic" refers to natural protein sequences.

Epitope clusters and their uses are described in US 20030215425 A1 entitled "EPITOPE SYNCHRONIZATION IN ANTIGEN PRESENTING CELLS," and US 20030046714 A1 entitled ANTI-NEOVASCULATURE PREPARATIONS FOR CANCER.

In another embodiment of the invention an encoded antigen can be delivered in the form of a viral vector. A wide array of viruses with modified genomes adapted to express interposed reading frames but often no, or at least a reduced number of, viral proteins are known in the art, including without limitation, retroviruses including lentiviruses, adenoviruses, parvoviruses including adeno-associated virus, herpesviruses, and poxviruses including vaccinia virus. Such viral vectors facilitate delivery of the nucleic acid component into the cell allowing for expression. A subset of these vectors, such as retroviruses and parvoviruses, promote integration of their nucleic acid component into the host genome, whereas others do not.

Bacteria can also serve as vectors, that is they can be used to deliver a nucleic acid molecule capable of causing expression of an antigen. For example, a strain of *Listeria monocytogenes* has been devised that effects its own lysis upon entering the cytosol of macrophages (its normal target), thereby releasing plasmid from which antigen is subsequently expressed (Dietrich, G. et al., Biotechnology 16:181-185, 1998, which is hereby incorporated by reference in its entirety). *Shigela flexneri* and *Escherichia coli* have been similarly used (Sizemore, D.R. et al., Science 270:299-302, 1995, and Courvalin, P. et al., Life Sci. 318:1207-1212, 1995, respectively.

The use of microbial vectors for nucleic acid delivery can be complicated by the immune reactions the vectors themselves provoke. When prolonged or repeated administration is required, antibody elicited by the earlier treatment can prevent useful quantities of the vector from ever reaching its intended host. However, by direct administration intra lymph node, for example, the combination of proximity to host cells and the much reduced effective dose makes it possible to administer a dose capable of evading or overwhelming an existing antibody titer.

The word vector has been used, here and elsewhere, in reference to several modalities and variously modified (e.g., expression vector, viral vector, delivery vector, etc.). The underlying principle is that a nucleic acid capable of causing expression of an antigen, rather than the antigen itself, ultimately arrives in an APC. Unless modified, explicitly or by local context, the term vector as used herein is intended to encompass all such possibilities.

The techniques discussed above are distinct from the approach of modifying the microbial genome, including extra-chromosomal DNA, such that the antigen is produced as a component of the microbe, which is then itself administered as the immunogen. Examples of microbes used in the genomic modification approach include viruses, bacteria, fungi, and protazoa. In embodiments described herein, the compositions, including the vaccines, can include the already synthesized antigen or a nucleic acid capable of causing an APC to express the antigen *in vivo.* In alternative embodiments, combinations of these two techniques are used. For example, one embodiment contemplates the use of a virus vector as discussed above that also incorporates a target epitope into a capsid or envelope protein.

Antigens may be used alone or may be delivered in combination with other antigens or with other compounds such as cytokines. Cytokines that are known to enhance immune stimulation of CTL responses, include, for example, GM-CSF, IL- 12, IL-2, TNF, IFN, IL-18, IL-3, IL-4, IL-8, IL-9, IL-13, IL-10, IL-14, IL-15, G-SCF, TFN alpha, IFN beta, IFN gamma, TGF alpha, TGF beta, and the like. Cytokines are known in the art and are readily available in the literature or commercially. Many animal and human tumors have been shown to produce cytokines, such as IL-4, IL-10, TGF-B, that are potent modulators of the immune response and that protect tumors from immune-mediated destruction. The production of IL-4, IL-10 or TGF-B by tumors may achieve this protective effect by suppressing the induction of cellular immunity, including the elaboration of CTL responses. Alternatively, cytokines that support CTL responses can be exogenously added to help in the balance between induction of anti-tumor cell mediated and non-tumor-destructive humoral responses. Several such exogenous cytokines show utility in experimental mouse vaccination models which are known to enhance CTL responses, including GM-CSF, IFN and IL-2. An example of an effective exogenous cytokine that can be used is GM-CSF. GM-CSF is reported to enhance the expression of the so called "co-stimulatory" molecules, such as B7-1 or B7-2 on antigen presenting cells (APC). These co-stimulatory molecules are important players in the variety of interactions that occur during stimulation of CTL by APC. Moreover, GM-CSF is known to induce activation of APCs and to facilitate growth and differentiation of APCs, thereby making these APCs important CTL stimulating cells available both in greater numbers and potency.

Immunogenic compositions can additionally contain non-target antigens in order to improve the response to the target antigen. Thus co-induction of a helper response, such as Th and/or B cell immunity against non-self or foreign antigens not expressed within the tumoral process or in the body, can result in a substantial improvement in the magnitude and quality of the immune response to the "self" or "self-modified" target antigens expressed within the tumor or underlying stroma. For example, co-initiating a Th immune response against a non-target antigen such as tetanus toxoid can result in the generation of helper cells with bystander effect relative to generation of CTL or B cell responses against the target tumor or self antigens. Any defined sequence expressing or encompassing peptide motifs that bind to at least one class II MHC protein expressed by recipient, where such sequences are non-homologous or contain non-homologous segments relative to self antigens, can be used. Preferably, such sequences are of microbial origin and shown to be immunogenic in HLA-defined or broader populations. In addition to tetanus toxoid (whole or portions, including, but not limited to, portions that are 90%, 80%, 75%, 70%, 60%, 50%, 40%, 30%, 25%, 20%, 15%, 10%, or 5% of a whole toxoid), further examples include, but are not limited to, sequences derived from HBVcore, influenza hemagglutinin, Plasmodium circumsporozoite antigen, and HTLV-1 envelope protein, and fragments of these sequences that are 90%, 80%, 75%, 70%, 60%, 50%, 40%, 30%, 25%, 20%, 15%, 10%, or 5% of the respective full-length sequences. In some embodiments, the tetanus toxoid portion is 5% to 90% of a whole toxoid, in other embodiments, the portion is 15% to 80% of a whole toxoid, in still other embodiments, the toxoid portion is 25% to 70% of a whole toxoid, in yet other embodiments, the toxoid portion is 35% to 60% of a whole toxoid, in still other embodiments, the toxoid portion is 45% to 55% of a whole toxoid. Similarly, co-administration of a strongly immunogenic B cell epitope (a non-self antigen) with or without a Th epitope (a non-self antigen) with target epitopes (self, tumoral) in a cognate fashion (that is, within the same molecule), can result in improved immune response, or even break of tolerance (T cell) against the therapeutic target, via immune antibody antigen complexes and bystander T cell help.

### Delivery of the Antigen

While not wanting to be bound by any particular theory, it is thought that T cells do not have a functional memory that is long-lived. Antibody-mediated B-cell memory, on the other hand, appears to have a long-lived effector memory. Thus, delivering an antigen that induces a CTL response is most preferably done over time to keep the patient's immune system appropriately stimulated to attack the target cells. In one approach the presence of antigen is maintained virtually continuously within the lymphatic system to maintain effector CTL function as disclosed in U.S. Patent Application Pub. No.20020007173 A1, entitled "A METHOD OF INDUCING A CTL RESPONSE." In another approach T cell memory is repeatedly induced, and re-amplified and reactivated as described in in U.S. Patent Application No. 10/871,707, entitled "METHODS TO ELICIT, ENHANCE AND SUSTAIN IMMUNE RESPONSES AGAINST MHC CLASS I-RESTRICTED EPITOPES, FOR PROPHYLACTIC OR THERAPEUTIC PURPOSE" (Pub. No. 2005-0079152 A1), filed June 17, 2004. While it has been suggested that antigens and adjuvants can be prepared as biodegradable microspheres or liposomes, none of these preparations have thus far provided a CTL response that is useful for attacking cancer cells or pathogens on a long term basis. Preferably, delivery of the antigen is sustained over the desired period of time at a level sufficient to maintain the antigen level to obtain the desired response. In one embodiment, a reservoir having fluid antigen composition can be used to deliver the antigen such that it reaches the animal's lymphatic system. While much of the following discussion focuses on the use of infusion to deliver the antigen it is also possible to use bolus injections directly into the lymphatic system, the number and frequency of which will depend on the persistence of antigen conferred by the particular form and formulation of antigen used.

Ultimately antigen finds its way into the lymphatic system in order to most efficiently stimulate CTL. Delivery of antigen can involve infusion into various compartments of the body, including but not limited to subcutaneous, intravenous, intraperitoneal and intralymphatic, the latter being preferred. While each of these points of infusion results in antigen uptake into the lymphatic system, the relative amounts of antigen needed to induce a beneficial CTL response varies according to the site of infusion. In general, direct infusion of antigen into the lymph system is deemed to be the most efficient means of inducing a CTL response, however, any delivery route can be used. Pump systems are capable of delivering material quantities of antigen in a range that is suitable for inducing a CTL response through delivery to all compartments of the body. CTL stimulation following delivery of antigen via the various routes will vary depending on the properties of different antigens, including factors that influence antigen behavior in the body and its rate of equilibration to (or longevity in) the lymph, such as antigen stability in the body fluid, solubility of antigen in body fluid, binding affinity for HLA and potency as a stimulator of CTL.

In a preferred embodiment, introduction of the antigen is done as directly as possible to the lymphatic system to avoid the destruction of the antigen by metabolism in the body. When introduction of a fluid antigen composition occurs subcutaneously, larger quantities of antigen are needed to assure enough antigen reaches the lymphatic system. Such subcutaneous injection is disclosed herein, depending on factors such as cost, stability of the antigen, how quickly the antigen gets to the lymph system, how well it equilibrates with the lymph, and other factors that the attending doctor or specialist will recognize. Subcutaneous delivery generally can require 100 to 1000 times more antigen than direct delivery to the lymph system. It is preferable, therefore, that the antigen composition is introduced through a device for local administration to the lymphatic system, *e*.*g*., the spleen, a lymph node, or a lymph vessel. The device for local administration can be positioned outside the patient or implanted into the patient. In either case, the device can have a reservoir to hold the fluid antigen-containing composition, a pump to transfer the composition, and a transmission channel leading from the reservoir to be directed to the preferred region of administration in the patient's body. In either case it is preferably portable.

For the device positioned outside the patient's body (the external device), there are numerous devices used for delivering insulin to diabetic patients that are useful in delivering antigen according to the embodiments described herein. Generally these devices can be comprised of a reservoir for holding the antigen composition (instead of insulin), a programmable pump to pump the composition out of the reservoir, a transmission channel or line for transmitting the composition, and a means to introduce the composition into the animal's body to ultimately reach the lymphatic system.

Preferably, the reservoir for the antigen composition should be large enough for delivery of the desired amount of antigen over time and easily refillable or replaceable without requiring the user to reinsert the means for introducing the antigen composition to the lymph system.

In preparing the antigen compositions disclosed herein, a composition (preferably aqueous) can be prepared to be compatible with the lymph system and physiologically acceptable to the animal being treated. Relevant considerations include, for example, the physicochemical properties of the antigen, such as the isoelectric point, molecular weight, glycosylation or other post-translational modification, and overall amino acid composition. These properties along with any known behavior of the drug in different solutions (*e.g*., different buffers, cofactors, etc.) as well as its *in vivo* behavior can help guide the choice of formulation components. One parameter that impacts all the major degradation pathways is the solution pH. Thus, the initial formulations also assess the pH dependence of the degradation reactions and the mechanism for degradation, which can often be determined from the pH dependence to determine the stability of the protein in each solution. Rapid screening methods usually involve the use of accelerated stability at elevated temperatures (e.g., 40° C) using techniques known in the art.

In general the antigen compositions useful in embodiments described herein can be suitable for parenteral injection, in very small quantities. As such a composition should be free of contamination and have a pH compatible with the lymphatic system. However, because very small quantities of the antigenic composition will be delivered it need not be the same pH as blood or lymph, and it need not be aqueous-based. The preferable pH range that is compatible is from about 6.7 - 7.3 and can be prepared using water for injection to meet USP specifications (see Remington: The Science and Practice of Pharmacy, Nineteenth Edition; Chapters 86-88).
For antigens that are less soluble, a suitable cosolvent or surfactant can be used, such as dimethyl sulfoxide (DMSO) or PLURONIC brand surfactants. Generally, a standard saline solution that is buffered with a physiologically acceptable weak acid and its base conjugate, *e*.*g*., a phosphate or citrate buffering system, will be the basis of the antigen composition. In some cases, a small amount of an antioxidant may be useful to stabilize the composition and prevent oxidation. Factors to consider in preparing the antigen compositions can be found in the 1994 American Chemical Society book entitled "Formulation and Delivery of Proteins and Peptides" (Acs Symposium Series, No. 567) by Jeffery L. Cleland and Robert Langer (Editor).

For nucleic acid encoded antigens similar considerations can apply, although the variety of physico-chemical properties encountered with polypeptides is absent, so that acceptable formulations will have nearly universal applicability. As seen in Examples 6-10, plasmid DNA in standard phosphate buffered saline (PBS) is an acceptable and effective formulation. In some disclosed embodiments, DNA is administered continuously or intermittently at short intervals, from a reservoir worn on, or implanted in, the patient's body. It is preferable that the DNA be maintained in a soluble, stable form at or near body temperature over a period of time measured minimally in days. In such applications where the formulated nucleic acid will be delivered from a reservoir over a period of several days or longer, the stability of the nucleic acid at room or body temperature for that period of time, as well as its continued sterility, take on increased importance. The addition of bacteriostatic agents (*e.g*., benzyl or ethyl alcohol) and chelating agents (e.g. EDTA) is useful toward these ends. Formulations containing about 0.5-2 % ethyl alcohol, 0.25-0.5mM EDTA generally perform well. Such formulations are also appropriate for bolus injections.

Generally the amount of the antigen in the antigen composition will vary from patient to patient and from antigen to antigen, depending on such factors as the activity of the antigen in inducing a response and the flow rate of the lymph through the patient's system. In general the antigen composition may be delivered at a rate of from about 1 to about 500 microliters/hour or about 24 to about 12000 microliters/day. The concentration of the antigen is such that about 0.1 micrograms to about 10,000 micrograms of the antigen will be delivered during 24 hours. The flow rate is based on the knowledge that each minute approximately about 100 to about 1000 microliters of lymph fluid flows through an adult inguinal lymph node. The objective is to maximize local concentration of vaccine formulation in the lymph system. A certain amount of empirical investigation on patients will be necessary to determine the most efficacious level of infusion for a given vaccine preparation in humans.

To introduce the antigen composition into the lymphatic system of the patient the composition is preferably directed to a lymph vessel, lymph node, the spleen, or other appropriate portion of the lymphatic system. Preferably, the composition is directed to a lymph node such as an inguinal or axillary node by inserting a catheter or needle to the node and maintaining the catheter or needle throughout the delivery. Suitable needles or catheters are available made of metal or plastic (e.g., polyurethane, polyvinyl chloride [PVC], TEFLON, polyethylene, and the like). In inserting the catheter or needle into the inguinal node for example, the inguinal node is punctured under ultrasonographic control using a Vialon™ Insyte-W™ cannula and catheter of 24G3/4 (Becton Dickinson, USA) which is fixed using Tegaderm™ transparent dressing (Tegaderm™ 1624, 3M, St. Paul, MN 55144, USA). This procedure is generally done by an experienced radiologist. The location of the catheter tip inside the inguinal lymph node is confirmed by injection of a minimal volume of saline, which immediately and visibly increases the size of the lymph node. The latter procedure allows confirmation that the tip is inside the node. This procedure can be performed to ensure that the tip does not slip out of the lymph node and can be repeated on various days after implantation of the catheter. In the event that the tip does slip out of location inside the lymph node, a new catheter can be implanted.

### Formulation and Treatment protocol

There are several approaches to utilizing the combination of TuAAs with DNA vaccines. A first approach is to include all the antigens or epitopes from all the antigens in a given combination into a single DNA expression vector. This approach has the advantages of simplicity for manufacturing and administration to patients. However, in some instances, epitope competition can limit the usefulness of this approach. That is, it is possible that only the most immunogenic epitope will elicit an immune response when a vaccine with several epitopes representing all TuAAs in the combination is given to patients. It is also more difficult to design and construct a DNA vaccine in which all epitopes are expressed at high efficiencies. Nevertheless, because the procedure for treating patients is simple and uniform within each type of cancer, the cost is likely to be lower than for the other approaches described below.

An alternate approach is to include only one antigen or epitopes of one antigen in a DNA expression vector. This approach has the advantages of simplicity in designing and constructing the DNA vector, flexibility, and customized administration to patients. If a large number of individual TuAA vaccines are available, then one can customize treatment for each individual patient based on the TuAA expression profile of his or her tumor. For example, if the standard combination for treating a given type of cancer is TuAAs A, B, and C (where A, B, and C designate different tumor associated antigens), but a patient's tumor expresses TuAAs A, C, and Z (but not B), then the patient can be treated with separate vaccines for each of A, C, and Z. This flexibility and customizability improves the success rate of immunotherapy because antigen redundancy can be achieved for each patient. However, the procedure of treating the patient can be more complex. For example, delivery using this approach can include a sequential administration scheme (one antigen at a time), or injection into multiple, anatomically separate sites of the patient at about the same time.

Still another approach is to combine epitopes from multiple TuAAs that have similar immunogenicity into a DNA expression vector (more than one vector may be used for some combinations).

A profile of the antigen expression of a particular tumor can be used to determine which antigen or combination of antigens to use. Exemplary methodology and specific antigenic combinations of particular benefit in directing an immune response against particular cancers are disclosed in US 20050118186 A1, U.S. Patent Application Publication No. 20050079152 A1, filed on June 17, 2004, US 20060159694 A1, all entitled "COMBINATIONS OF TUMOR-ASSOCIATED ANTIGENS IN VACCINES FOR VARIOUS TYPES OF CANCER".

Patients that can benefit from such methods of immunization can be recruited using methods to define their MHC protein expression profile and general level of immune responsiveness. In addition, their level of immunity can be monitored using standard techniques in conjunction with access to peripheral blood. Finally, treatment protocols can be adjusted based on the responsiveness to induction or amplification phases and variation in antigen expression. For example, rather than amplifying after some set number of entrainment doses, repeated entrainment doses can be administered until a detectable response is obtained, and then amplifying peptide dose(s) can be administered. Similarly, scheduled amplifying or maintenance doses of peptide can be discontinued if their effectiveness wanes, antigen-specific regulatory T cell numbers rise, or some other evidence of tolerization is observed, and further entrainment can be administered before resuming amplification with the peptide. The integration of diagnostic techniques to assess and monitor immune responsiveness with methods of immunization is discussed more fully in US 20050287068 A1 entitled "IMPROVED EFFICACY OF ACTIVE IMMUNOTHERAPY BY INTEGRATING DIAGNOSTIC WITH THERAPEUTIC METHODS,".

Combination of active immunotherapies, as disclosed herein, with other treatment modalities can increase the susceptibility of tumoral processes to the elicited immune response and thereby result in increased therapeutic benefit. Tumor debulking prior to or during active immunotherapy increases the potential for any particular level of immune response to slow or halt disease progression or to bring about tumor regression or elimination. Additionally, tissue damage, necrosis, or apoptosis initiated with antibody therapy, radiotherapy, biotherapy, chemotherapy, passive immunotherapy or surgery, can facilitate the active immunotherapeutic approach via general inflammation resulting in recruitment of immune effector cells including antigen-specific effectors. In general, any method to induce a transient or more permanent general inflammation within one or multiple tumors / metastatic lesions can facilitate the active immunotherapy. Alternatively or in addition to enabling recruitment of effectors, general inflammation can also increase the susceptibility of target cells to immune mediated attack (e.g., as interferons increase expression of target molecules on cancer cells and underlying stroma). Still other strategies to increase susceptibility of tumor cells to immune mediated attack - by providing factors that interfere with the "stress response" or increase target molecules on cancer cells or stromal cells - can synergize with active immunotherapy.

The following examples are for illustrative purposes only and are not intended to limit the scope of the embodiments in any way.

### Examples

### Example 1

### TuAA analysis and selection of combinations

The presence of TuAAs was measured by Real-Time PCR (RT-PCR). Briefly, total RNA was isolated from tumor specimens by standard methods and cDNA was made with standard reverse transcription procedures. Complementary DNA (cDNA) was amplified with specially designed, gene specific, primers that anneal only to cDNA but not genomic DNA. TuAA expression patterns of 12 ovarian and 7 colorectal tumor specimens were analyzed by RT-PCR. The results are summarized in the Table 4 below.

**Table 4**

| | Total # | PRAME | NY-ESO-1 | SSX-2 | PSMA | MAGE1 | MAGE3 |
|---|---|---|---|---|---|---|---|
| Ovarian | 12 | 12 | 5 | 6 | 6 | 4 | 3 |
| Colorectal | 7 | 5 | 1 | 2 | 5 | 0 | 1 |

### Example 2

### Ovarian Cancer

In the case of ovarian cancer, all samples analyzed were positive for FRAME. Thus the inclusion of PRAME in the combination improves coverage of the cases with ovarian cancer.

In order to achieve antigen redundancy and improve, coverage in a large population, combinations of other antigens in addition to PRAME were considered. SSX-2 as well as PSMA were present in 6 of the 12 cases individually, but the combination of SSX-2 and PSMA provided coverage in 9 of 12 cases. Although NY-ESO-1 and SSX-2 were only present in 5 and 6 of the 12 cases, respectively, either NYESO-1 or SSX-2 was detected in 7 of the 12 cases.

Thus, for assembling panels, the combination of PRAME, SSX-2, and PSMA or PRAME, NY-ESO-1, and SSX-2 provided preferable coverage and redundancy compared to the combination of PRAME and PSMA or the combination of FRAME and SSX-2. The combination of PRAME, SSX-2, and PSMA provided excellent coverage of cases and good antigen redundancy because the majority of ovarian tumor samples analyzed had at least two of the four TuAA in the combination present. The combination of PRAME, SSX-2, PSMA, and NY-ESO-1 provided more preferred antigen redundancy, and thus, lower possibility of tumor escape.

### Example 3

### Colorectal cancer

In the case of colorectal cancer, PRAME and PSMA were each detected in 5 of the 7 samples analyzed. In 6 of the 7 cases, either PRAME or PSMA was detected. Although SSX-2 was only detected in 2 of 7 cases, both SSX-2-PRAME and SSX2-PSMA combinations increased coverage to 6 of 7. Similarly, although NYESO-1 was detected in only 1 of 7 cases, the combination of NY-ESO-1-PRAME as well as the NYESO-1-PSMA combination increased coverage to 6 of 7. The addition of SSX-2 or NYESO-1 to the PRAME and PSMA combination improved coverage to 7 of 7. Thus, for assembling panels, the combination of PRAME, PSMA, and NYESO-1, or the combination of PRAME, PSMA, and SSX-2 provided good coverage of cases and redundancy of antigens for a majority of patients. The combination of PRAME, PSMA, NY-ESO-1, and SSX-2 provided further redundancy.

### Example 4

### Pancreatic Cancer

Real-Time PCR (RT-PCR) was utilized to determine the presence of PRAME, SSX2, NY-ESO-1, and PSMA. Briefly, total RNA was isolated from 5 pancreatic tumor specimens by standard methods and cDNA was made with standard reverse transcription procedures. Complementary DNA (cDNA) was amplified with specially designed, gene specific, primers that anneal only to cDNA but not genomic DNA.

In the pancreatic cancer specimens, the presence of PRAME, NYESO-1, SSX-2, and PSMA was detected in 100%, 40%, 20%, and 100% of the specimens, respectively (see Table 5). Elsewhere, PSMA and over-expression of HER2-/neu were reported to be present in 100% and 21% of pancreatic tumors, respectively (Chang SS et al, Cancer Res 1999, 59:3192; Safran H et al, Am J Clin Oncol. 2001, 24:496). Although over-expression of HER2/neu may render the cancer tissue a preferred target, thus providing some specificity for immunotherapy, low level expression of HBR2/neu in normal tissues remains a concern. Thus, for assembling panels of antigens, the combination of NYESO-1, SSX-2, plus PRAME or PSMA provides excellent coverage and some redundancy for pancreatic cancer. Inclusion of both PRAME and PSMA significantly improves redundancy.

**Table 5**

| TAA | PRAME | SSX2 | NY-ESO-1 | PSMA |
|---|---|---|---|---|
| Detection Freq. | 5/5 | 1/5 | 2/5 | 5/5 |
| %positive | 100 | 20 | 40 | 100 |

### Example 5

### Renal cell carcinoma

For renal cell carcinoma, SSX-2, PSMA and FRAME were detected with frequencies of 5, 100 and 40%, respectively (Sahin, U et al, Clin Cancer Res. 2000, 6:3916; Chang SS et al, Urology 2001, 57:801; Neumann E et al, Cancer Res. 1998, 58:4090). Thus, the combination of PSMA and PRAME provides excellent coverage and redundancy for renal cell carcinoma. Adding SSX-2 to the combination of PSMA and FRAME improves redundancy.

### Example 6

### Non-small cell lung cancer

For non-small cell lung cancer, the reported presence of NYESO-1, SSX-2, MAGE-3, BAGE, over-expression of Her2/neu, and PSMA was 21, 15, 60, 6, 16, and 100%, respectively (Scanlan MJ et al, Cancer lett 2000, 150:155; Chang SS et al, Cancer Res 1999, 59:3192; Selvaggi G et al, Cancer 2002, 94:2669). Thus, the combination of NYESO-1, SSX-2, MAGB-3, and PSMA provides coverage and antigen redundancy for the immunotherapy of non-small cell lung cancer.

### Example 7

### Melanoma

For melanoma, Melan A, Tyrosinase, NYESO-1, and SSX-2 were reported to be present in 92, 92, 41, and 35% of tumor specimens, respectively (Fetsch PA, et al, Cancer 1999, 87:37; Fetsch PA, et al, Cancer 2000, 90:252; Schultz-Thater E et al, Br J Cancer 2000, 83:204; Sahin, U et al, Clin Cancer Res. 2000, 6:3916). Therefore, the combination of Melan A, Tyrosinase, NYESO-1, and SSX-2 provides excellent coverage and antigen redundancy for the immunotherapy of melanoma. Significant redundancy is achieved using tyrosinase and melan-A together, or by combining NY-ESO-1 and SSX-2 with either of tyrosinase or melan-A.

### Example 8

Further studies involving the foregoing tumor types established more robust support for the observed expression patterns and preferred panels of TuAA. A total of 34 ovarian, 44 colon, 18 renal, and 13 pancreatic tissue samples obtained from various vendors were analyzed for tumor-associated antigen expression using qRTPCR. The results of these assays showed that PRAME and PSMA were expressed frequently (ranging from 68% to 100%) in all four types of tumors studied. NY-ESO-1 and SSX2 were expressed in 20% to 40% of ovarian and pancreatic tumors, although the expression of NY-ESO-1 and SSX-2 in colorectal and renal tumors was substantially lower (6% to 12%).

**Table 6:**

| Overall Expression Profiles for Tumor Associated Antigens | | | | |
|---|---|---|---|---|
| From RTPCR analysis of Primary Tumors and Metastases | | | | |
| **Tumor-Associated Antigen** | **% Samples Expressing a Given Antigen** | | | |
| | **Ovarian^{a}** | **Renal^{b}** | **Pancreatic^{c}** | **Colorectal^{d}** |
| SSX2 | 36 | 6 | 20 | 8 |
| NY-ESO-1 | 30 | 6 | 40 | 12 |
| PRAME | 97 | 83 | 80 | 76 |
| PSMA | 91 | 100 | 100 | 68 |
| MAGE-1 | 27 | 6 | 33 | 8 |
| MAGE-3 | 30 | 22 | 42 | 20 |
| SCP-1 | 30 | 11 | 0 | 0 |
| CEA | 30 | 0 | 58 | 92 |

| | | | | |
|---|---|---|---|---|
| ^{a} 33 samples (27 primary tumors and 6 metastases) ^{b} 18 samples (18 primary tumors) ^{c} 15 samples (14 primary tumors and 1 metastasis; PSMA on 10 samples) ^{d} 25 samples (13 primary tumors and 12 metastases) | | | | |

### Example 9

### Schedule of immunization with plasmids expressing epitopes from two antigens

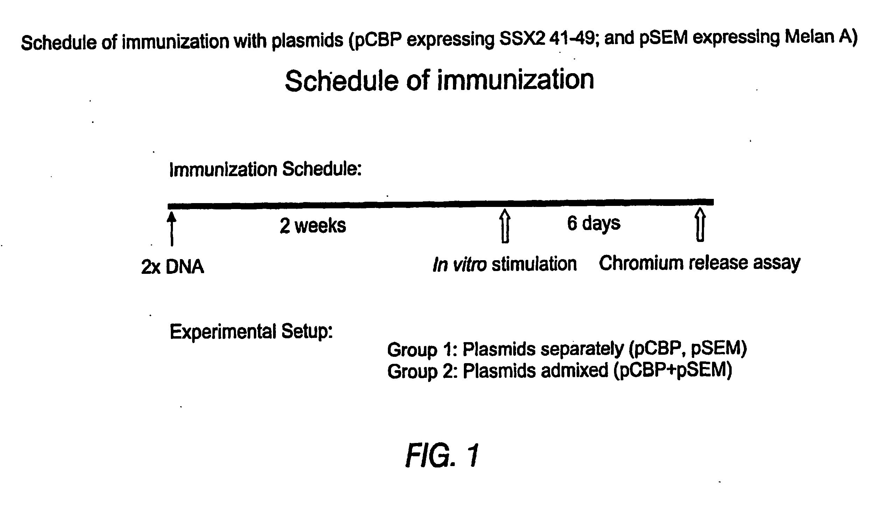

Two groups of HHD mice (n=4) were immunized via intra lymph node injection with either pSEM expressing Melan-A ₂₆₋₃₅A27L (ELA) and pCBP expressing SSX-2₄₁₋₄₉ as a mixture; or with pSEM in the left inguinal lymph node and pCBP in the right inguinal lymph node, twice, at day 0 and 4 as shown in Figure 1. The amount of the plasmid was 25µg/plasmid/dose. Two weeks later, the animals were sacrificed, and cytotoxicity was measured against T2 cells pulsed or not with peptide.

### Example 10

### Co-administration of different vectors carrying distinct antigens

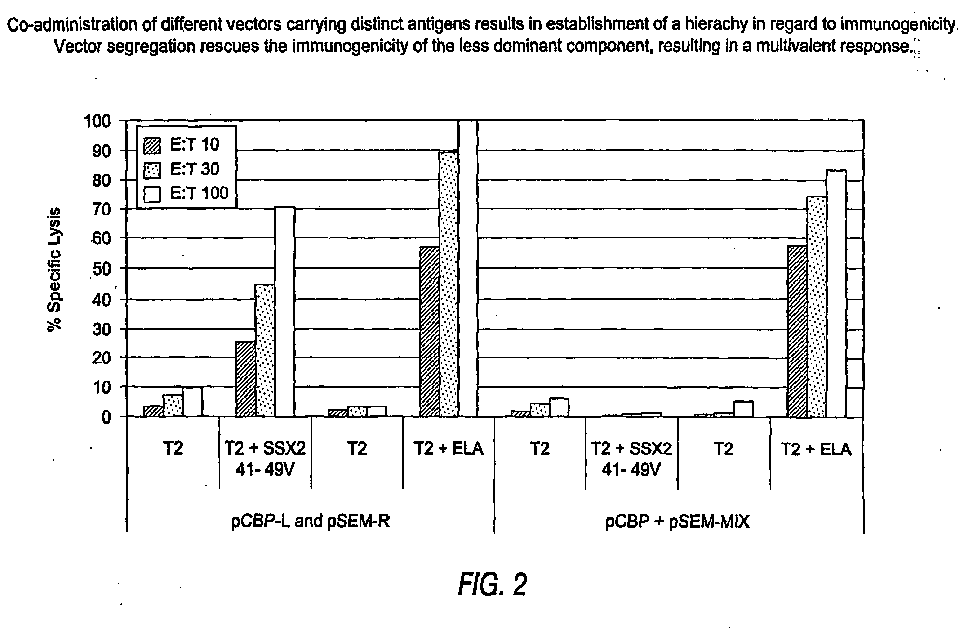

The animals immunized as described in Example 9, were sacrificed and splenocytes from each group pooled and stimulated with the two peptides (ELA or SSX-2₄₁₋₄₉) in parallel. The cytotoxicity was measured by incubation with Cr⁵¹-tagged, peptide loaded T2 target cells. Data in Figure 2 show mean of specific cytotoxicity (n=4/group) against various target cells.

The results show that use of plasmid mixture interferes with the response elicited by pCBP plasmid; however, segregating the two plasmids relative to site of administration rescues the activity of pCBP. Thus, the co-administration of different vectors carrying distinct antigens can result in establishment of a hierarchy with regard to immunogenicity. Vector segregation can rescue the immunogenicity of the less dominant component, resulting in a multivalent response.

### Example 11

### Rescue of Multivalent Response by Addition of Peptide Boost Steps

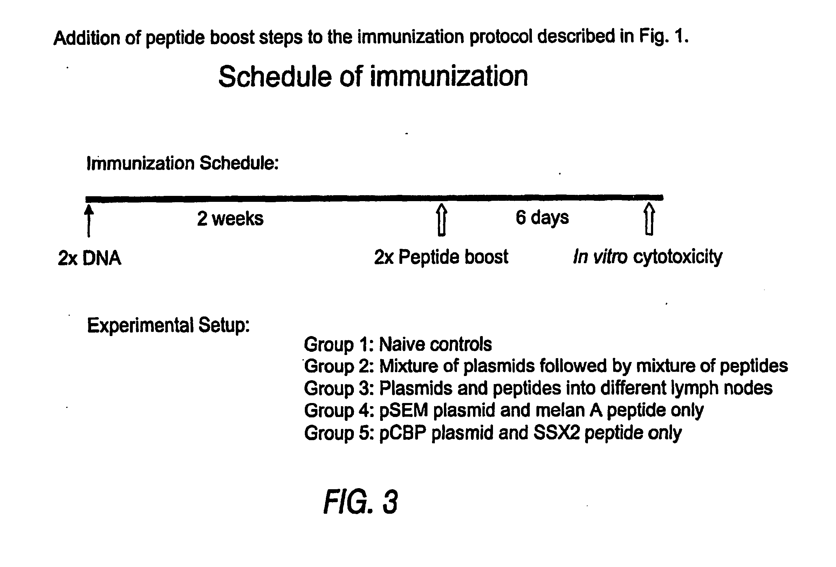

Four groups of HHD mice (n=6) were immunized via intra lymph node injection with either pSEM and pCBP as a mixture; or with pSEM in the left inguinal lymph node and pCBP in the right inguinal lymph node, twice, at day 0 and 4 as shown in Figure 3. As a control, mice were immunized with either pSEM or pCBP plasmid. The amount of the plasmid was 25µg/plasmid/dose. Two weeks later, the animals were boosted with melan A and/or SSX-2 peptides, mirroring the plasmid immunization dose and combination. Two weeks later, the animals were challenged with splenocytes stained with CFSE and loaded or not with Melan A or SSX-2 peptide, for evaluation of *in vivo* cytotoxicity.

### Example 12

### Peptide amplification rescues the immunogenicity of the less dominant epitope

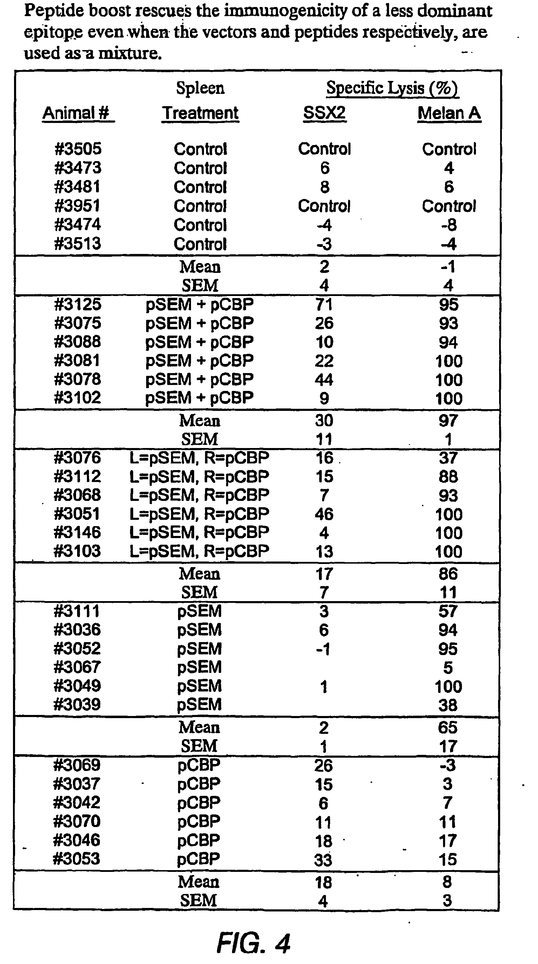

Mice were immunized as described in Example 11 and challenged with HHD littermate splenocytes coated with ELA or SSX-2 peptide, employing a triple peak CFSE *in vivo* cytotoxicity assay that allows the assessment of the specific lysis of two antigen targets simultaneously. Equal numbers of control-CFSE^{lo}, SSX-2₄₁₋₄₉-CFSE^{med}, and ELA-CFSE^{hi} cells were intravenously infused into immunized mice and 18 hours later the mice were sacrificed and target cell elimination was measured in the spleen (Figure 4) by CFSE fluorescence using a flow cytometry. Figure 4 shows the percent specific lysis of the SSX-2 and Melan-A antigen targets from individual mice, as well as the mean and SEM for each group.

The results show that immunizing the animals with a mixture of the two vaccines comprising plasmids followed by peptides generated immunity to both antigens and resulted in the highest immune response, representing an average SSX-2 percent specific lysis in the spleen of 30+/-11, and an average Melan-A percent specific lysis of 97+/-1.

### Example 13

### Clinical practice for entrain-and-amplify immunization

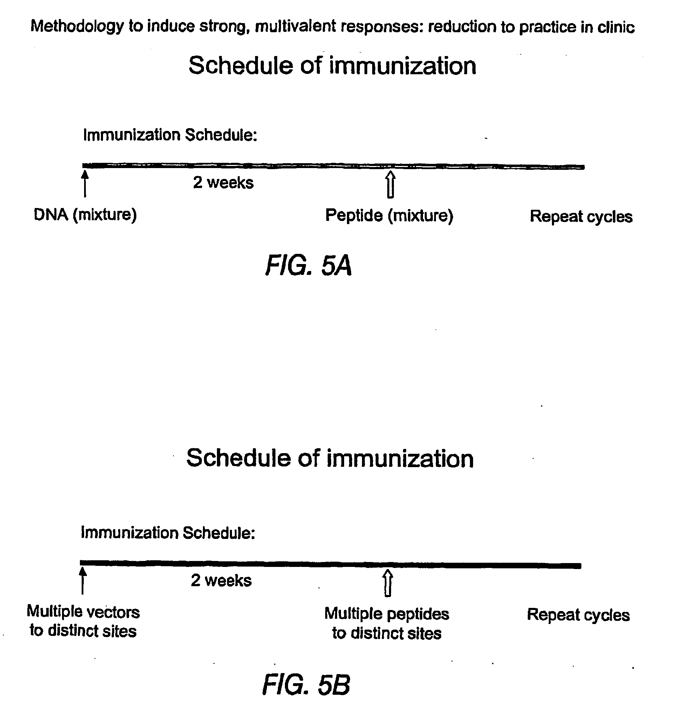

The data in figures 2 and 4 suggest two scenarios for achieving a strong multivalent response in the clinic, shown in Figure 5. In the first scenario (A), use of peptides for boosting restores multivalent immune responses even if plasmids and peptides are used as mixtures. In the second scenario (B), segregation of plasmid and peptide components respectively, allows induction of multivalent immune responses.

### Example 14

### MKC1207: an Entrain-and-Amplify Therapeutic for Melanoma

MKC1207 comprises the plasmid pSEM (US 20030228634 A1 filed November 7, 2002, in which it is referred to as pMA2M) and peptides corresponding to Melan-A 26-35 and tyrosinase 369-377. The plasmid encodes the A27L analogue of the Melan-A epitope and the native tyrosinase epitope sequence. The plasmids encode both of these epitopes in such a manner that they can be expressed and presented by pAPC. In alternate embodiments of the therapeutic the peptides can comprise the native sequence or be analogues such as those disclosed in US 20060057673 A1 entitled EPITOPE ANALOGUES, filed on date even with this disclosure.

Briefly, the plasmid is administered intranodally to the inguinal lymph nodes as an entraining immunogen. Subsequently the peptides are administered intranodally, one to the left node, the other to the right as amplifying immunogens. The entrain-and-amplify protocol is described in greater detail in US 20050079152 A1 filed on June 17, 2004 and US 20060165711 A1.

Melanoma patients can be screened according to the methods disclosed herein and MKC1207 administered to patients whose tumor antigen profile includes Melan-A and/or tyrosinase. In a preferred embodiment the pateint's tumor tissue also expresses HLA-A2, particularly HLA-A*0201.

### Example 15

### MKC1106: a Tetravalent Entrain-and-Amplify Therapeutic for Carcinoma

MKC1106 comprises the plasmids pCBP (described in US 20030228634 A1, filed November 7, 2002), and pRP12 (described in US 20070004662 A1) entitled METHODS AND COMPOSITIONS TO ELICIT MULTIVALENT IMMUNE RESPONSES AGAINST DOMINANT AND SUBDOMINANT EPITOPES, EXPRESSED ON CANCER CELLS AND TUMOR STROMA, filed on date even with this disclosure, and peptides corresponding to NY-ESO-1 157-165, SSX-2 41-49, FRAME 425-433 and PSMA 288-297. The plasmids encode both of these epitopes in such a manner that they can be expressed and presented by pAPC. In alternate embodiments of the therapeutic the peptides can comprise the native sequence or be analogues such as those disclosed in US 20060063913 A1, entitled SSX-2 PEPITDE ANALOGS, and US 20060094661 A1, entitled NY-ESO-1 PEPTIDE ANALOGS, and US 20060057673 A1 entitled EPITOPE ANALOGS, and US 20070060524 A1, entitled EPITOPE ANALOGS, each of which is filed on date even with the instant application.

Briefly, the plasmids are administered intranodally to the inguinal lymph nodes, one to the left side and one to the right, as an entraining immunogen. Subsequently the peptides are sequentially administered intranodally, two on separate days to the left node, the other two on separate days to the right as amplifying immunogens. It is preferred, but not absolutely required that the peptides be administered to the same lymph node that received the plasmid encoding the corresponding epitopes. The entrain-and-amplify protocol is described in greater detail in US 20050079152 A1, filed on June 17, 2004 and US 20060165711 A1.

Carcinoma patients, especially those with ovarian, colorectal, pancreatic, or renal cell carcinoma, can be screened according to the methods disclosed herein and MKC1106 administered to patients whose tumor profile includes PRAME, PSMA, NY-ESO-1, and/or SSX-2. The NY-ESO-1 epitope targeted by MKC1106 is also found in LAGE 1a/s, so the presence of this antigen in a profile would also be considered a match. As tumor antigen expression tends to be heterogeneous, any particular tissue sample is likely not to give a complete indication of all the antigens expressed. Thus, it is not necessary that a patient's profile contain all four of the antigens for that patient to be a candidate for treatment with MKC1106. However, preferably the profile contains 2, 3, or 4 of the antigens.

## Claims

1. A method of matching a cancer condition in a patient with one or more immunotherapeutic agents for use in an immunotherapeutic regimen, comprising the steps of:
classifying the patient's tumor tissue on the basis of assaying the tumor tissue for two or more expressed tumor-associated antigens (TuAAs) in a preselected panel, wherein the preselected panel comprises two or more antigens selected from the group consisting of an SSX protein, SSX-2, SSX-4, a MAGE protein, MAGE-1, MAGE-3, PRAME, NY-ESO-1, LAGE, PSMA, PSCA, SCP-1, melan-A/MART-1 and tyrosinase, to develop an antigen profile for the tumor; and
selecting one or more immunotherapeutic agents for use in an immunotherapeutic regimen based on the profile, the regimen comprising administration of one or more immunotherapeutic agents targeting two or more antigens in the profile, wherein the tumor tissue is classified by the TuAAs that it expresses and not by the tissue of origin.

2. The method of Claim 1, wherein the preselected panel further comprises at least one TuAA selected from the group consisting of a cancer testis antigen, a tissue-specific antigen, an oncofetal antigen, a differentiation antigen, a growth factor, a growth factor receptor, an adhesion factor, a signal transduction protein, a transcription factor, an oncogene product, a tumor suppressor gene product, and a microbial antigen.

3. The method of Claim 1, wherein the cancer condition is a carcinoma.

4. The method of Claim 3, wherein the carcinoma is selected from the group consisting of breast, colorectal, prostate, pancreatic, lung, ovarian, renal cell, and melanocyte.

5. The method of Claim 1 wherein the immunotherapeutic agent is an active immunotherapeutic.

6. The method of Claim 1, wherein the immunotherapeutic agent comprises or encodes at least a segment of at least one of the expressed TuAAs.

7. The method of Claim 1 wherein the immunotherapeutic agent is a passive immunotherapeutic.

8. The method of Claim 7, wherein the immunotherapeutic agent is a monoclonal antibody.

9. The method of Claim 1, comprising at least two assaying steps carried out at different time points during the course of disease, wherein comparative information is obtained from the assaying steps.

10. The method of Claim 1, wherein the tumor is melanoma and the panel of TuAAs comprises at least two TuAAs selected from the group consisting of tyrosinase, melan-A/MART-1, NY-ESO-1, PRAME, an SSX protein, and a MAGE protein.

11. The method of Claim 10, wherein the SSX protein is SSX-2 or SSX-4.

12. The method of Claim 10, wherein the MAGE protein is MAGE-1 or MAGE-3.

13. The method of Claim 1, wherein the tumor is breast cancer and the panel of TuAAs comprises at least two TuAAs selected from the group consisting of NY-ESO-1, Her2/neu, an SSX protein, and a MAGE protein.

14. The method of Claim 1, wherein the tumor is colorectal cancer and the panel of TuAAs comprises at least two TuAAs selected from the group consisting of CEA, an SSX protein, PRAME, NY-ESO, LAGE, PSCA, SCP-1, PSMA and a MAGE protein.

15. The method of Claim 1, wherein the tumor is lung cancer and the panel of TuAAs comprises at least two TuAAs selected from the group consisting of PSMA, NY-ESO-1, SSX-2, and a MAGE protein.

16. The method of Claim 1, wherein the tumor is prostate cancer and the panel of TuAAs comprises at least two TuAAs selected from the group consisting of NY-ESO-1, PSA, PSCA, PSMA, an SSX protein, and a MAGE protein.

17. The method of Claim 1, wherein the tumor is ovarian cancer and the panel of TuAAs comprises at least two TuAAs selected from the group consisting of PRAME, PSMA, PSCA, a MAGE protein, SCP-1, an SSX protein, CEA, Her-2/Neu, NY-ESO-1, and LAGE.

18. The method of Claim 17, wherein the ovarian cancer is selected from the group consisting of serous carcinoma, non-serous carcinoma, mucinous (cell) carcinoma, and clear cell carcinoma.

19. The method of Claim 1, wherein the tumor is renal cancer and the panel of TuAAs comprises at least two TuAAs selected from the group consisting of an SSX protein, PRAME, NY-ESO, LAGE, PSCA, SCP-1, PSMA and a MAGE protein.

20. The method of Claim 1, wherein the tumor is pancreatic cancer and the panel of TuAAs comprises at least two TuAAs selected from the group consisting of an SSX protein, PRAME, NY-ESO, LAGE, PSCA, PSMA and a MAGE protein.

21. The method of Claim 1, wherein antigen expression is detected by a technique comprising at least one of RT-PCR, transcript determination, protein determination, epitope determination or any combination thereof.

22. The method of Claim 1, wherein at least one expressed tumor associated antigen is a neoplastic cell antigen, or a tumor-associated stromal antigen, or both.

23. The method of Claim 22, wherein the tumor-associated stromal antigen is a neovasculature-associated antigen.

24. The method of Claim 23, wherein the neovasculature-associated antigen is PSMA and the neoplastic cell antigen is selected form the group consisting of NY-ESO-1, SSX2, LAGE, and PRAME.

25. The method of Claim 1, wherein the tumor tissue comprises primary tumor tissue.

26. The method of Claim 1, wherein the tumor tissue comprises metastatic tumor tissue.

27. The method of Claim 1, wherein the regimen comprises administering both an active immunotherapeutic agent and a passive immunotherapeutic agent.

28. The method of Claim 1, wherein the two or more antigens expressed by the tumor include an antigen expressed by a neoplastic cell and an antigen expressed by a tumor-associated stromal cell.

29. The method of Claim 1, wherein the immunotherapeutic agent is suitable for intranodal administration.

30. The method of Claim 1, wherein the immunotherapeutic agent is suitable for administration directly to the lymphatic system of the patient.

31. The method of Claim 30, wherein the immunotherapeutic agent comprises an immunopotentiating adjuvant.

## Patentansprüche

1. Verfahren zum Abgleich einer Krebserkrankung in einem Patienten mit einem oder mehreren immuntherapeutischen Mitteln für die Verwendung in einem immuntherapeutischen Behandlungsplan umfassend die Schritte:
Klassifizieren des Tumorgewebes des Patienten basierend auf dem Untersuchen des Tumorgewebes auf zwei oder mehr exprimierte tumorassoziierte Antigene (TuAAs) in einer vorausgewählten Gruppe, wobei die vorausgewählte Gruppe zwei oder mehr Antigene ausgewählt aus der Gruppe bestehend aus einem SSX-Protein, SSX-2, SSX-4, einem MAGE-Protein, MAGE-1, MAGE-3, PRAME, NY-ESO-1, LAGE, PSMA, PSCA, SCP-1, Melan-A/MART-1 und Tyrosinase umfasst, um ein Antigenprofil für den Tumor zu entwickeln; und
Auswählen eines oder mehrerer immuntherapeutischer Mittel für die Verwendung in einem immuntherapeutischen Behandlungsplan basierend auf dem Profil, wobei der Behandlungsplan, welcher die Verabreichung von einem oder mehreren immuntherapeutischen Mitteln, welche gegen zwei oder mehr Antigene in dem Profil gerichtet sind, umfasst, wobei das Tumorgewebe anhand der TuAAs, die es exprimiert, und nicht anhand des Ursprungsgewebes klassifiziert wird.

2. Verfahren nach Anspruch 1, wobei die vorausgewählte Gruppe weiterhin mindestens ein TuAA umfasst, welches ausgewählt ist aus der Gruppe bestehend aus einem Cancer/Testis-Antigen, einem gewebespezifischen Antigen, einem onkofötalem Antigen, einem Differenzierungsantigen, einem Wachstumsfaktor, einem Wachstumsfaktorrezeptor, einem Adhäsionsfaktor, einem Signaltransduktionsprotein, einem Transkriptionsfaktor, einem Onkogenprodukt, einem Tumorsuppressorgenprodukt und einem mikrobiellen Antigen.

3. Verfahren nach Anspruch 1, wobei die Krebserkrankung ein Karzinom ist.

4. Verfahren nach Anspruch 3, wobei das Karzinom ausgewählt ist aus der Gruppe bestehend aus Brust-, Kolorektal-, Prostata-, Bauchspeicheldrüsen-, Lungen-, Ovarial-, Nierenzell- und Melanozytenkarzinom.

5. Verfahren nach Anspruch 1, wobei das immuntherapeutische Mittel ein aktives Immuntherapeutikum ist.

6. Verfahren nach Anspruch 1, wobei das immuntherapeutische Mittel mindestens ein Segment von mindestens einem der exprimierten TuAAs umfasst oder kodiert.

7. Verfahren nach Anspruch 1, wobei das immuntherapeutische Mittel ein passives Immuntherapeutikum ist.

8. Verfahren nach Anspruch 7, wobei das immuntherapeutische Mittel ein monoklonaler Antikörper ist.

9. Verfahren nach Anspruch 1, umfassend mindestens zwei Untersuchungsschritte, die zu verschiedenen Zeitpunkten während des Krankheitsverlaufs durchgeführt werden, wobei vergleichende Informationen durch die Untersuchungsschritte erhalten werden.

10. Verfahren nach Anspruch 1, wobei der Tumor ein Melanom ist und die Gruppe der TuAAs mindestens zwei TuAAs umfasst, ausgewählt aus der Gruppe bestehend aus Tyrosinase, Melan-A/MART-1, NY-ESO-1, PRAME, einem SSX-Protein und einem MAGE-Protein.

11. Verfahren nach Anspruch 10, wobei das SSX-Protein SSX-2 oder SSX-4 ist.

12. Verfahren nach Anspruch 10, wobei das MAGE-Protein MAGE-1 oder MAGE-3 ist.

13. Verfahren nach Anspruch 1, wobei der Tumor Brustkrebs ist und die Gruppe von TuAAs mindestens zwei TuAAs umfasst, ausgewählt aus der Gruppe bestehend aus NY-ESO-1, Her2/Neu, einem SSX-Protein und einem MAGE-Protein.

14. Verfahren nach Anspruch 1, wobei der Tumor Kolorektalkrebs ist und die Gruppe von TuAAs mindestens zwei TuAAs umfasst, ausgewählt aus der Gruppe bestehend aus CEA, einem SSX-Protein, PRAME, NY-ESO, LAGE, PSCA, SCP-1, PSMA und einem MAGE-Protein.

15. Verfahren nach Anspruch 1, wobei der Tumor Lungenkrebs ist und die Gruppe der TuAAs mindestens zwei TuAAs umfasst, ausgewählt aus der Gruppe bestehend aus PSMA, NY-ESO-1, SSX-2 und einem MAGE-Protein.

16. Verfahren nach Anspruch 1, wobei der Tumor Prostatakrebs ist und die Gruppe von TuAAs mindestens zwei TuAAs umfasst, ausgewählt aus der Gruppe bestehend aus NY-ESO-1, PSA, PSCA, PSMA, einem SSX-Protein und einem MAGE-Protein.

17. Verfahren nach Anspruch 1, wobei der Tumor Ovarialkrebs ist und die Gruppe von TuAAs mindestens zwei TuAAs umfasst, ausgewählt aus der Gruppe bestehend aus PRAME, PSMA, PSCA, einem MAGE-Protein, SCP-1, einem SSX-Protein, CEA, Her-2/Neu, NY-ESO-1 und LAGE.

18. Verfahren nach Anspruch 17, wobei der Ovarialkrebs ausgewählt ist aus der Gruppe bestehend aus serösem Karzinom, nicht-serösem Karzinom, muzinösem (Zell-) Karzinom und klarzelligem Karzinom.

19. Verfahren nach Anspruch 1, wobei der Tumor Nierenkrebs ist und die Gruppe von TuAAs mindestens zwei TuAAs umfasst, ausgewählt aus der Gruppe bestehend aus einem SSX-Protein, PRAME, NY-ESO, LAGE, PSCA; SCP-1, PSMA und einem MAGE-Protein.

20. Verfahren nach Anspruch 1, wobei der Tumor Bauchspeicheldrüsenkrebs ist und die Gruppe von TuAAs mindestens zwei TuAAs umfasst, ausgewählt aus der Gruppe bestehend aus einem SSX-Protein, PRAME, NY-ESO, LAGE, PSCA, PSMA und einem MAGE-Protein.

21. Verfahren nach Anspruch 1, wobei die Antigenexpression mithilfe einer Technik detektiert wird, die mindestens eine Methode von RT-PCR, Transkriptbestimmung, Proteinbestimmung, Epitopbestimmung oder beliebigen Kombinationen davon umfasst.

22. Verfahren nach Anspruch 1, wobei mindestens ein exprimiertes tumorassoziiertes Antigen ein neoplastisches Zellantigen oder ein tumorassoziiertes stromales Antigen oder beides ist.

23. Verfahren nach Anspruch 22, wobei das tumorassoziierte stromale Antigen ein Neovaskulatur-assoziiertes Antigen ist.

24. Verfahren nach Anspruch 23, wobei das Neovaskulatur-assoziierte Antigen PSMA ist und das neoplastische Zellantigen ausgewählt ist aus der Gruppe bestehend aus NY-ESO-1, SSX-2, LAGE und PRAME.

25. Verfahren nach Anspruch 1, wobei das Tumorgewebe ein primäres Tumorgewebe umfasst.

26. Verfahren nach Anspruch 1, wobei das Tumorgewebe ein metastatisches Tumorgewebe umfasst.

27. Verfahren nach Anspruch 1, wobei der Behandlungsplan das Verabreichen von sowohl einem aktiven immuntherapeutischen Mittel als auch einem passiven immuntherapeutischen Mittel umfasst.

28. Verfahren nach Anspruch 1, wobei die zwei oder mehr Antigene, die von dem Tumor exprimiert werden, ein Antigen, welches von einer neoplastischen Zelle exprimiert wird, und ein Antigen, welches von einer tumorassoziierten stromalen Zelle exprimiert wird, einschließen.

29. Verfahren nach Anspruch 1, wobei das immuntherapeutische Mittel für die intranodale Verabreichung geeignet ist.

30. Verfahren nach Anspruch 1, wobei das immuntherapeutische Mittel für die direkte Verabreichung an das lymphatische System eines Patienten geeignet ist.

31. Verfahren nach Anspruch 30, wobei das immuntherapeutische Mittel ein immunverstärkendes Adjuvans umfasst.

## Revendications

1. Procédé de mise en concordance d'un état de cancer chez un patient avec un ou plusieurs agents immunothérapeutiques destinés à être utilisés dans un régime immunothérapeutique, comportant les étapes de :
classification du tissu tumoral du patient sur la base d'une analyse du tissu tumoral permettant de révéler deux ou plusieurs antigènes tumoraux (TuAA) exprimés dans une gamme présélectionnée, la gamme présélectionnée comportant deux ou plusieurs antigènes sélectionnés dans le groupe comprenant une protéine SSX, SSX-2, SSX-4, une protéine MAGE, MAGE-1, MAGE-3, PRAME, NY-ESO-1, LAGE, PSMA, PSCA, SCP-1, melan-A/MART-1 et la tyrosinase, pour mettre au point un profil d'antigène pour la tumeur ; et
sélection d'un ou plusieurs agents immunothérapeutiques destinés à être utilisés dans un régime immunothérapeutique sur la base du profil, le régime comportant l'administration d'un ou plusieurs agents immunothérapeutiques ciblant deux ou plusieurs antigènes dans le profil, le tissu tumoral étant classifié selon les TuAA qu'il exprime et non selon le tissu d'origine.

2. Le procédé de la revendication 1, où la gamme présélectionnée comprend en outre au moins un TuAA sélectionné dans le groupe comprenant un antigène testiculaire du cancer, un antigène tissulaire spécifique, un antigène oncofoetal, un antigène de différenciation, un facteur de croissance, un récepteur de facteur de croissance, un facteur d'adhésion, une protéine de transmission de signal, un facteur de transcription, un produit oncogène, un produit de gène suppresseur de tumeur et un antigène microbien.

3. Le procédé de la revendication 1, où l'état de cancer est un carcinome.

4. Le procédé de la revendication 3, où le carcinome est sélectionné dans le groupe comprenant le carcinome du sein, le carcinome colorectal, le carcinome de la prostate, le carcinome pancréatique, le carcinome du poumon, le carcinome ovarien, le carcinome à cellules rénales et le carcinome à mélanocytes.

5. Le procédé de la revendication 1, où l'agent immunothérapeutique est un agent immunothérapeutique actif.

6. Le procédé de la revendication 1, où l'agent immunothérapeutique comprend ou code au moins un segment d'au moins un des TuAA exprimés.

7. Le procédé de la revendication 1, où l'agent immunothérapeutique est un agent immunothérapeutique passif.

8. Le procédé de la revendication 7, où l'agent immunothérapeutique est un anticorps monoclonal.

9. Le procédé de la revendication 1, comprenant au moins deux étapes d'analyse effectuées à différents moments au cours de la maladie, des informations comparatives étant obtenues à partir des étapes d'analyse.

10. Le procédé de la revendication 1, où la tumeur est un mélanome et la gamme de TuAA comporte au moins deux TuAA sélectionnés dans le groupe comprenant la tyrosinase, melan-A/MART-1, NY-ESO-1, PRAME, une protéine SSX et une protéine MAGE.

11. Le procédé de la revendication 10, où la protéine SSX est SSX-2 ou SSX-4.

12. Le procédé de la revendication 10, où la protéine MAGE est MAGE-1 ou MAGE-3.

13. Le procédé de la revendication 1, où la tumeur est un cancer du sein et la gamme de TuAA comporte au moins deux TuAA sélectionnés dans le groupe comprenant NY-ESO-1, Her2/neu, une protéine SSX et une protéine MAGE.

14. Le procédé de la revendication 1, où la tumeur est un cancer colorectal et la gamme de TuAA comporte au moins deux TuAA sélectionnés dans le groupe comprenant CEA, une protéine SSX, PRAME, NY-ESO, LAGE, PSCA, SCP-1, PSMA et une protéine MAGE.

15. Le procédé de la revendication 1, où la tumeur est un cancer du poumon et la gamme de TuAA comporte au moins deux TuAA sélectionnés dans le groupe comprenant PSMA, NY-ESO-1, SSX-2 et une protéine MAGE.

16. Le procédé de la revendication 1, où la tumeur est un cancer de la prostate et la gamme de TuAA comporte au moins deux TuAA sélectionnés dans le groupe comprenant NY-ESO-1, PSA, PSCA, PSMA, une protéine SSX et une protéine MAGE.

17. Le procédé de la revendication 1, où la tumeur est un cancer ovarien et la gamme de TuAA comporte au moins deux TuAA sélectionnés dans le groupe comprenant PRAME, PSMA, PSCA, une protéine MAGE, SCP-1, une protéine SSX, CEA, Her-2/Neu, NY-ESO-1 et LAGE.

18. Le procédé de la revendication 17, où le cancer ovarien est sélectionné dans le groupe comprenant un carcinome séreux, un carcinome non séreux, un carcinome (cellulaire) mucoïde et un carcinome à cellules claires.

19. Le procédé de la revendication 1, où la tumeur est un cancer rénal et la gamme de TuAA comporte au moins deux TuAA sélectionnés dans le groupe comprenant une protéine SSX, PRAME, NY-ESO, LAGE, PSCA, SCP-1, PSMA et une protéine MAGE.

20. Le procédé de la revendication 1, où la tumeur est un cancer pancréatique et la gamme de TuAA comporte au moins deux TuAA sélectionnés dans le groupe comprenant une protéine SSX, PRAME, NY-ESO, LAGE, PSCA, PSMA et une protéine MAGE.

21. Le procédé de la revendication 1, où l'expression d'antigènes est détectée par une technique comportant au moins l'une des techniques suivantes : RT-PCR, détermination par transcription, détermination de protéine, détermination d'épitope ou toute combinaison de celles-ci.

22. Le procédé de la revendication 1, où au moins un antigène tumoral exprimé est un antigène cellulaire néoplastique, ou un antigène tumoral stromal, ou les deux.

23. Le procédé de la revendication 22, où l'antigène tumoral stromal est un antigène néovasculaire.

24. Le procédé de la revendication 23, où l'antigène néovasculaire est PSMA et l'antigène cellulaire néoplastique est sélectionné dans le groupe comprenant NY-ESO-1, SSX2, LAGE et PRAME.

25. Le procédé de la revendication 1, où le tissu tumoral comprend du tissu tumoral primaire.

26. Le procédé de la revendication 1, où le tissu tumoral comprend du tissu tumoral métastatique.

27. Le procédé de la revendication 1, où le régime comporte l'administration à la fois d'un agent immunothérapeutique actif et d'un agent immunothérapeutique passif.

28. Le procédé de la revendication 1, où les deux ou plusieurs antigènes exprimés par la tumeur incluent un antigène exprimé par une cellule néoplastique et un antigène exprimé par une cellule tumorale stromale.

29. Le procédé de la revendication 1, où l'agent immunothérapeutique est apte à être administré par voie intranodale.

30. Le procédé de la revendication 1, où l'agent immunothérapeutique est apte à être administré directement dans le système lymphatique du patient.

31. Le procédé de la revendication 30, où l'agent immunothérapeutique comprend un adjuvant immunopotentialisant.
